# EUROPEAN PATENT SPECIFICATION

(11) **EP 2 079 747 B1**
(45) Date of publication and mention of the grant of the patent: **09.03.2011**
(21) Application number: 07825335.8
(22) Date of filing: 03.10.2007
(51) Int. Cl.: C07D 487/06, A61K 31/55, A61P 3/00, A61P 43/00

(54) **HETEROCYCLIC COMPOUNDS USEFUL AS ANABOLIC AGENTS FOR LIVESTOCK ANIMALS**
ALS ANABOLE MITTEL FÜR VIEH GEEIGNETE HETEROZYKLISCHE VERBINDUNGEN
COMPOSÉS HÉTÉROCYCLIQUES UTILES EN TANT QU'AGENTS ANABOLIQUES POUR LE BÉTAIL

(30) Priority: 13.10.2006 US 829419 P
(43) Date of publication of application: 22.07.2009
(73) Proprietor: Pfizer Limited, Sandwich, Kent CT13 9NJ (GB)
(72) Inventor: BOYLE, Jessica, Sandwich Kent CT13 9NJ (GB); FENWICK, Ashley, Edward, Sandwich Kent CT13 9NJ (GB); GETHIN, David, Morris, Sandwich Kent CT13 9NJ (GB); McCUSKER, Catherine Frances, Shawlands, Glasgow, G41 4 AN (GB)
(74) Representative: Lane, Graham Mark Hamilton
(86) International application number: PCT/IB2007/003027
(87) International publication number: WO 2008/044127

(56) References cited:
- EP-A- 0 272 976
- US-B1- 6 841 563

## Description

The present invention relates to a series of 6-amino-7-hydroxy-4,5,6,7-tetrahydroimidazo[4,5,1 jk]-[1]benzazepin-2(1*H*)-ones. More particularly it relates to a series of 6-(aryl-1-methylalkyl)amino-7-hydroxy-4,5,6,7-tetrahydroimidazo[4,5,1 jk][1]benzazepin-2(1*H*)-ones. The compounds act as agonists at the beta-2 adrenoceptor and are useful as anabolic agents for livestock animals.

### BACKGROUND

The primary focus in livestock production remains efficiency via optimising the conversion of feed into lean meat. Feed constitutes a high proportion of the total economic investment in the final stages of livestock production, and hence there is a continued demand for agents which enhance feed conversion ratio (FCR). The most effective way of improving FCR is via metabolic manipulation to enhance the animals' potential to deposit muscle protein, which also provides obvious benefits in yield grade and carcass composition.

One approach to achieving higher quality meat and improving the meat yield is to administer agents that are agonists at the beta-2 adrenoceptor. Examples of agents registered for such use in livestock animals are Zilmax™ (zilpaterol) and Optaflexx™ (ractopamine). Zilpaterol is (±)-trans-6-(isopropylamino)-7-hydroxy-4,5,6,7-tetrahydro-imidazo[4,5,1-*jk*][1]benzazepin-2(1*H*)-one. Zilpaterol and similar analogues were first disclosed in FR2534257 and subsequently their use as animal feed additives was discussed in FR2608046 and EP272976. Ractopamine is (±)-4-(3-{[2-hydroxy-2-(4-hydroxyphenyl)ethyl]amino}butyl)phenol and was first disclosed by van Dijk and Moed, (Recl. Trav. Chim. Pays Bas, 1973, 92, 1281-1279). Its use as a feed additive was described in GB2133986. Both zilpaterol and ractopamine are administered during the latter stages of a production animal's life and cause an activation of a biological cascade mechanism, starting with interaction at the beta-2 adrenoceptor, which promotes and enhances lean muscle growth. A series of aryloxypropanolamines for improving livestock production have been recently disclosed in US-6841563.

There is a continuing need for alternative beta-2 adrenoceptor agonists for use as agents to improve meat production in livestock animals, and particularly for agonists with improved properties. For reasons of economy, the agent should preferably provide the desired improvement in meat production at a low dose. It must also not produce any undesired effects in the target animal. Finally, the meat produced by the animal must be safe for human consumption, which implies that the residual levels of the agent in the meat must be minimised. The ideal agent will therefore have a high affinity for, and be a fully efficacious agonist at, the beta-2 adrenoceptor of the target animal species. It will have a high degree of selectivity for this receptor, and it will be rapidly cleared from the animal in order to minimise the presence of residues in the meat without requiring an extended withdrawal period. A zero-day withdrawal period provides the maximum economic benefit to the farmer. Thus it is an aim of this invention to provide compounds which have a high affinity, selectivity, agonist efficacy and/or potency at the beta-2 adrenoceptor of relevant livestock animals, and/or that are rapidly metabolically cleared from the animal.

### SUMMARY OF THE INVENTION

In a first aspect, the present invention provides a compound of formula (1) 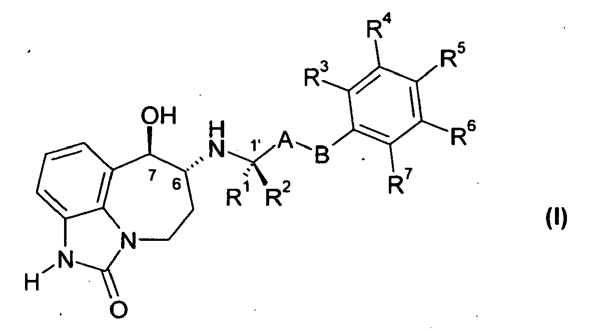 or a pharmaceutically acceptable salt thereof, wherein:
A is -CH₂-; and
B is -CH₂-, -C(CH₃)₂-, -O-, -CH₂-CH₂-,-CH₂-O-, or -O-CH₂-; or -A-B- is-CH=CH-;
one of R¹ and R² is CH₃ and the other is H;
R³, R⁴, R⁵, R⁶ and R⁷ are each independently selected from H, R⁸ and R⁹; or
R⁴ and R⁵ together are -O-CH₂-CH₂-, -CH₂CH₂-O- or -O-CH₂-O-, and R³, R⁶ and R⁷ are each independently selected from H, R⁸ and R⁹;
R⁸ is halo, -CN, C₁-C₄ alkyl, C₁-C₄ haloalkyl, -CH₂OH, -O-(C₁-C₄ alkyl), -O-CH₂-(C₃-C₅)cycloalkyl, -CO₂H, -CO₂(C₁-C₄ alkyl), -CONH₂, -CONH(C₁-C₄ alkyl), -CONH(C₁-C₄ haloalkyl), -CONH(C₃-C₆ cycloalkyl) or NH₂; and
R⁹ is -OH, -NHSO₂(C₁-C₃ alkyl), -NHCO(C₁-C₄ alkyl), -NHCO(C₁-C₄ haloalkyl), -NHSO₂(C₁-C₃ haloalkyl) or -NHSO₂(phenyl).

In a further aspect, the present invention provides a feed additive for a livestock animal comprising a compound of formula (I) or a pharmaceutically acceptable salt thereof.

Although it is not part of the invention a method of improving meat yield or meat quality in a livestock animal comprising administering to said livestock animal an effective amount of a compound of formula (I) or a pharmaceutically acceptable salt thereof. , is disclosed.

In a yet further aspect, the present invention provides the use of a compound of formula (I) or a pharmaceutically acceptable salt thereof as a medicament.

In a yet further aspect, the present invention provides a pharmaceutical composition comprising a compound of formula (I) or a pharmaceutically acceptable salt thereof.

### DETAILED DESCRPTION OF THE INVENTION

For the purposes of the present document, the following definitions apply.

"Alkyl" means a saturated monovalent hydrocarbon radical CₙH₂ₙ₊₁ which may be linear or branched. C₁-C₄, alkyl includes methyl, ethyl, *n-*propyl, isopropyl (1-methylethyl), *n-*butyl, sec-butyl (1-methylpropyl), isobutyl (2-methylpropyl) and *tert*-butyl (1,1-dimethytethyl).

"Cycloalkyl" means a saturated monovalent monocyclic or bridged or fused polycyclic hydrocarbon radical. C₃-C₅ cycloalkyl includes cyclopropyl, cyclobutyl and cyclopentyl.

"Halo" includes fluoro, chloro, bromo and iodo.

Haloalkyl means an alkyl group as defined above wherein one or more hydrogen atoms is replaced by a halogen atom selected from fluorine, chlorine, bromine and iodine. When the group contains more than one halogen atom then these atoms may be the same or different. Haloalkyl includes perhaloalkyl, i.e. an alkyl group wherein all the hydrogen atoms are replaced by halogen atoms. C₁-C₄ haloalkyl groups include fluoromethyl, difluoromethyl, trifluoromethyl, chlorodifluoromethyl, 2-bromoethyl, 2,2,2-trifluoroethyl, 3-iodopropyl, and 2,2,2-trichloro-1,1-dimethylethyl.

The compounds of formula (I) have three asymmetric carbon atoms (chiral centres), labelled 1', 6 and 7 in the structural formula. Certain embodiments of the substituents R³ to R⁷ may include additional chiral centres. Unless otherwise indicated, formula (I) depicts the relative stereochemistry at the three centres C-1', C-6 and C-7. It is not intended that the representation of formula (I) should be taken as implying the absolute stereochemistry at these centres. Accordingly, the present invention includes individual enantiomers of the compounds of formula (I) and mixtures thereof, including racemates. Where there is an additional chiral centre in a substituent then the invention includes diastereomeric mixtures as well as individual stereoisomers.

The compounds of formula (I) wherein -A-B- is -CH=CH- may exist as geometric isomers. Unless otherwise indicated, no particular geometry is implied by this notation. Accordingly, the present invention encompasses such compounds in the *cis (Z-)* or *trans* (*E*-) configuration, as well as mixtures of these geometric isomers.

Certain compounds of formula (I) may exist in more than one tautomeric form. The present invention encompasses all such tautomers, as well as mixtures thereof.

The present invention includes all pharmaceutically acceptable isotopically-labelled compounds of formula (I) wherein one or more atoms are replaced by atoms having the same atomic number, but an atomic mass or mass number different from the atomic mass or mass number which predominates in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labelled compounds of formula (I), for example, those incorporating a radioactive isotope, are useful in drug and/or substrate tissue distribution studies. The radioactive isotopes tritium, i.e. ³H, and carbon-14, i.e. ¹⁴C, are particularly useful for this purpose in view of their ease of incorporation and ready means of detection.

Substitution with heavier isotopes such as deuterium, i.e. ²H, may afford certain therapeutic advantages resulting from greater metabolic stability, for example, increased in vivo half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy.

Isotopically-labeled compounds of formula (I) can generally be prepared by conventional techniques known to those skilled in the art or by processes analogous to those described in the accompanying Examples and Preparations using an appropriate isotopically-labeled reagent in place of the non-labeled reagent previously employed.

The compounds of formula (I) are able to form addition salts with acids. Certain compounds of formula (I) which have an acidic functional group are able to form salts with suitable bases. Such salts are included within the scope of the present invention to the extent that they are acceptable for veterinary or pharmaceutical use.

Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinafoate salts.

Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts.

Hemisalts of acids and bases may also be formed, for example, hemisulphate and hemicalcium salts.

For a review on suitable salts, see Handbook of Pharmaceutical Salts: Properties, Selection, and Use by Stahl and Wermuth (Wiley-VCH, 2002).

Pharmaceutically acceptable salts of compounds of formula (I) may be prepared by one or more of three methods:
(i) by reacting the compound of formula (I) with the desired acid or base;
(ii) by removing an acid- or base-labile protecting group from a suitable precursor of the compound of formula (I) or by ring-opening a suitable cyclic precursor, for example, a lactone or lactam, using the desired acid or base; or
(iii) by converting one salt of the compound of formula (I) to another by reaction with an appropriate acid or base or by means of a suitable ion exchange column.

All three reactions are typically carried out in solution. The resulting salt may precipitate out and be collected by filtration or may be recovered by evaporation of the solvent.

The compounds of formula (I) and their salts may exist in a continuum of solid states ranging from fully amorphous to fully crystalline. The term 'amorphous' refers to a state in which the material lacks long range order at the molecular level and, depending upon temperature, may exhibit the physical properties of a solid or a liquid. Typically such materials do not give distinctive X-ray diffraction patterns and, while exhibiting the properties of a solid, are more formally described as a liquid. Upon heating, a change from solid to liquid properties occurs which is characterised by a change of state, typically second order ('glass transition'). The term 'crystalline' refers to a solid phase in which the material has a regular ordered internal structure at the molecular level and gives a distinctive X-ray diffraction pattern with defined peaks. Such materials when heated sufficiently will also exhibit the properties of a liquid, but the change from solid to liquid is characterised by a phase change, typically first order ('melting point').

The compounds of formula (I) and their salts may also exist in unsolvated and solvated forms. The term 'solvate' is used herein to describe a molecular complex comprising the compound of the invention and one or more pharmaceutically acceptable solvent molecules, for example, ethanol. The term 'hydrate' is employed when said solvent is water:

A currently accepted classification system for organic hydrates is one that defines isolated site, channel, or metal-ion coordinated hydrates - see Polymorphism in Pharmaceutical Solids by K. R. Morris (Ed. H. G. Brittain, Marcel Dekker, 1995). Isolated site hydrates are ones in which the water molecules are isolated from direct contact with each other by intervening organic molecules. In channel hydrates, the water molecules lie in lattice channels where they are next to other water molecules. In metal-ion coordinated hydrates, the water molecules are bonded to the metal ion.

When the solvent or water is tightly bound, the complex will have a well-defined stoichiometry independent of humidity. When, however, the solvent or water is weakly bound, as in channel solvates and hygroscopic compounds, the water/solvent content will be dependent on humidity and drying conditions. In such cases, non-stoichiometry will be the norm.

Pharmaceutically acceptable solvates in accordance with the invention include those wherein the solvent of crystallization may be isotopically substituted, *e.g*. D₂O, d₆-acetone, d₆-DMSO.

Also included within the scope of the invention are multi-component complexes (other than salts and solvates) wherein the drug and at least one other component are present in stoichiometric or non-stoichiometric amounts. Complexes of this type include clathrates (drug-host inclusion complexes) and co-crystals. The latter are typically defined as crystalline complexes of neutral molecular constituents which are bound together through non-covalent interactions, but could also be a complex of a neutral molecule with a salt. Co-crystals may be prepared by melt crystallisation, by recrystallisation from solvents, or by physically grinding the components together - see Chem Commun, 17, 1889-1896, by O. Almarsson and M. J. Zaworotko (2004). For a general review of multi-component complexes, see J Pharm Sci, 64 (8),1269-1288, by Haleblian (August 1975).

The compounds of formula (I) and their salts may also exist in a mesomorphic state (mesophase or liquid crystal) when subjected to suitable conditions. The mesomorphic state is intermediate between the true crystalline state and the true liquid state (either melt or solution). Mesomorphism arising as the result of a change in temperature is described as 'thermotropic' and that resulting from the addition of a second component, such as water or another solvent, is described as 'lyotropic'. Compounds that have the potential to form lyotropic mesophases are described as 'amphiphilic' and consist of molecules which possess an ionic (such as -COO⁻Na⁺, -COO⁻K^{*}, or -SO₃⁻Na⁺) or non-ionic (such as -N⁻N⁺(CH₃)₃) polar head group. For more information, see Crystals and the Polarizing Microscope by N. H. Hartshorne and A. Stuart, 4th Edition (Edward Arnold, 1970).

Hereinafter all references to compounds of formula (I) include references to salts, solvates, multi-component complexes and liquid crystals thereof and to solvates, multi-component complexes and liquid crystals of salts thereof.

Not included in the present invention there are the so-called 'prodrugs' of the compounds of formula (I). Thus certain derivatives of compounds of formula (I) which may have little or no pharmacological activity themselves can, when administered into or onto the body, be converted into compounds of formula (I) having the desired activity, for example, by hydrolytic cleavage. Such derivatives are referred to as'prodrugs'. Further information on the use of prodrugs may be found in Pro-drugs as Novel Delivery Systems, Vol.14, ACS Symposium Series (T. Higuchi and W. Stella) and Bioreversible Carriers in Drug Design, Pergamon Press, 1987 (Ed. E. B. Roche, American Pharmaceutical Association).

Prodrugs can, for example, be produced by replacing appropriate functionalities present in the compounds of formula I with certain moieties known to those skilled in the art as 'pro-moieties' as described, for example, in Design of Prodrugs by H. Bundgaard (Elsevier, 1985).

Examples of prodrugs include:
(i) derivatives of the C-7 hydroxyl function such as esters and acyloxymethyl ethers, wherein the hydrogen of the hydroxyl group is replaced by an acyl group such as (C₁-C₆ alkyl)CO- or (optionally substituted aryl)CO-, or by an acyloxymethyl group such as (C₁-C₆ alkyl)CO₂CH₂-; and
(ii) derivatives of the C-6 secondary amine function such as amides and carbamates, wherein the hydrogen of the amine group is replaced by an acyl group such as (C₁-C₆ alkyl)CO- or by an alkyloxycarbonyl group such as (C₁-C₈ alkyl)OCO-.

Certain of the options for R³ to R⁷ may also be amenable to the formation of prodrugs.

In a further aspect, the present invention provides processes for the preparation of a compound of formula (I), or a pharmaceutically, veterinarily or agriculturally acceptable salt thereof, or a pharmaceutically, veterinarily or agriculturally acceptable solvate (including hydrate) of either entity, as illustrated below.

It will be apparent to those skilled in the art that sensitive functional groups may need to be protected and deprotected during synthesis of a compound of the invention. This may be achieved by conventional methods, for example as described in "Protective Groups in Organic Synthesis" by T.W. Greene and P.G.M. Wuts, John Wiley & Sons Inc (1999), and references therein.

The following processes are illustrative of the general synthetic procedures which may be adopted in order to obtain the compounds of the invention.

When one or more of R³, R⁴, R⁵, R⁶, and R⁷ contain reactive functional groups then additional protection may be provided, according to standard procedures, during the synthesis of compounds of formula (I). In the processes described below, for all synthetic precursors used in the synthesis of compounds of formula (I), the definitions of R³, R⁴, R⁵, R⁶, and R⁷ , wherein R³, R⁴, R⁵, R⁶, and R⁷, are as defined for formula (I), are intended to optionally include suitably protected variants, P³, P⁴, P⁵, P⁶, and P⁷. Such suitable protecting groups for these functionalities are described in the references listed below and the use of these protecting groups where needed is specifically intended to fall within the scope of the processes described in the present invention for producing compounds of formula (I) and its precursors. When suitable protecting groups are used, then these will need to be removed to yield compounds of formula (I). Deprotection can be effected according to standard procedures including those described in the references listed below.

### 1. Preparation of compounds of Formula (I)

### 1.1. Reductive Amination

Compounds of formula (I) may be synthesised by the reductive amination of the methyl ketones of formula (II), wherein R³, R⁴, R⁵, R⁶, R⁷, A and B are as defined for formula (I), using the amino-alcohol of formula **(III),** as illustrated in Scheme A: 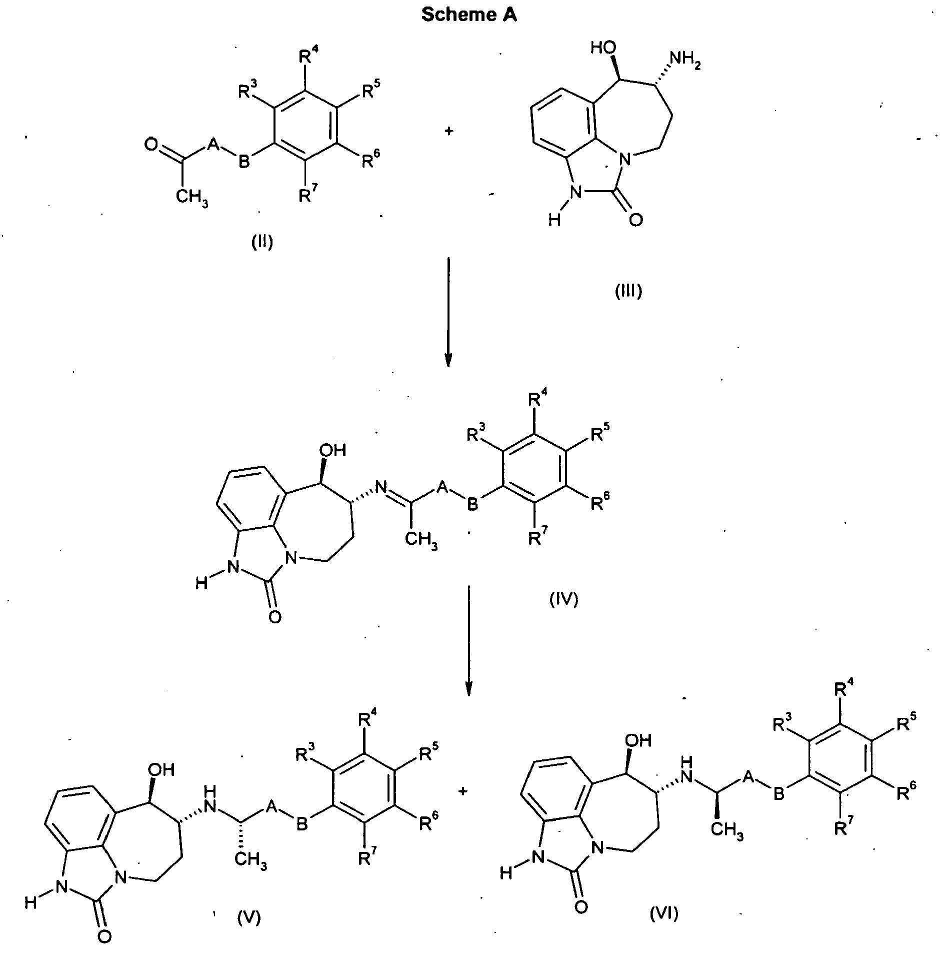 wherein the wedge and dashed bonds indicate the relative stereochemistry of the 6-amino and 7-hydroxy substituents. The skilled person will appreciate that the individual enantiomers or the racemate of formula (III) may be used for the reductive amination reaction.

A variety of reaction conditions may be used. In general, reaction of the amino-alcohol (III) with the ketones of formula (II) yields an imine, (IV), which may be reduced *in situ* to give compounds of formula (I). Imine formation is achieved by standard methods, for example, by reaction of the amino-alcohol (III) with the ketones (II) in an alcoholic solvent, preferably methanol, in the presence of a base, such as triethylamine or potassium hydroxide. Reaction conditions may vary from room temperature to 50°C for periods ranging from 10 minutes to 60 hours, optionally under nitrogen and optionally heating in a microwave. Compounds of formula (1) may then be prepared by in situ imine reduction, typically using sodium borohydride or sodium cyanoborohydride, at temperatures ranging from 0°C to 60°C for 1 - 60 hours, typically overnight.
The imine reduction proceeds with a range of diastereoselectivities, though no predictive trend has yet been observed.

Compounds of formula (I) wherein A-B is CH=CH may be prepared using similar conditions to those described above by reductive amination of the amino-alcohol (III) with the α,β-unsaturated enones of formula (VII) wherein R³, R⁴, R⁵, R⁶, and R⁷ are as defined for formula (I), as illustrated in Scheme B.

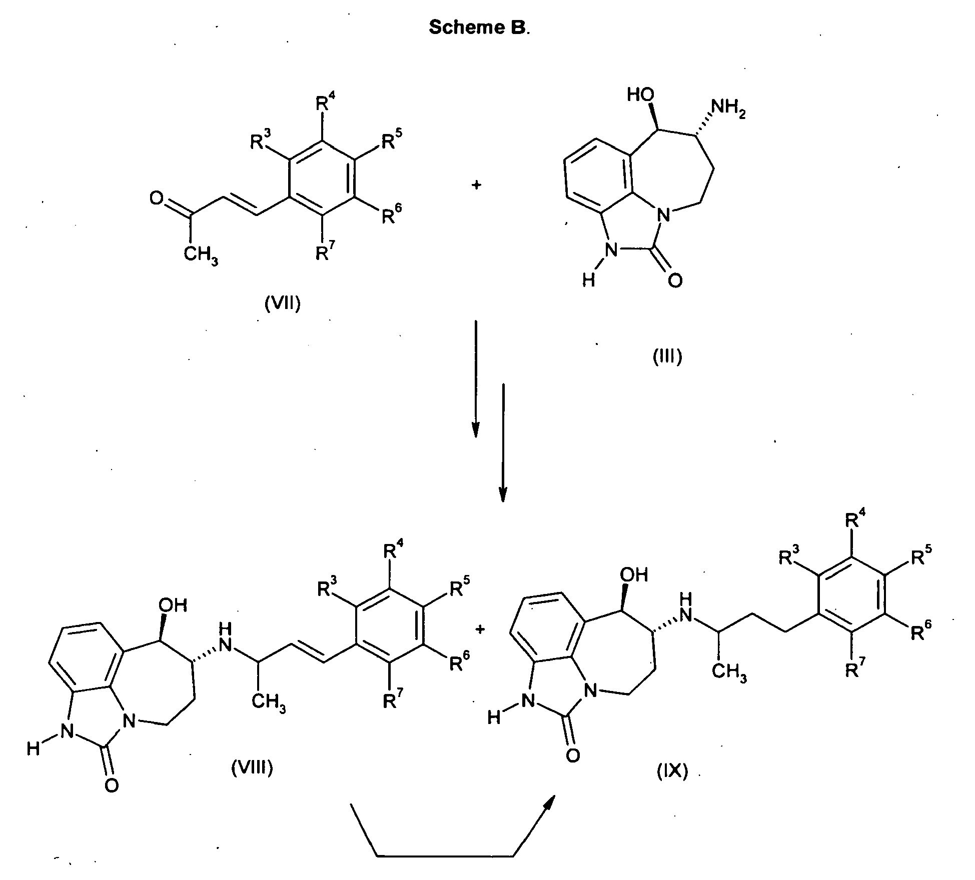

Using excess borohydride reducing agent will also reduce the double bond, so using enones of formula (VII) may yield compounds of formula (I) wherein A-B is CH₂CH₂ or A-B is CH=CH, i.e. compounds of formula (IX) or compounds of formula (VIII).

Compounds of formula (I) wherein A-B is CH₂-CH₂ may also be prepared from compounds of formula (I) wherein A-B is CH=CH using standard reducing agents, such as hydrogen in the presence of a metal catalyst such as Wilkinson's catalyst, palladium on carbon or platinum oxide in a protic solvent, for example methanol, or those described in "Handbook of Reagents for Organic Synthesis - Oxidising and Reducing Agents" edited by S.D.Burke and R.L.Danheiser.

### 2. Preparation of tricyclic intermediates

### 2.1 Aminoalcohol (III)

The amino-alcohol of formula (III) may be prepared as shown in Scheme C. 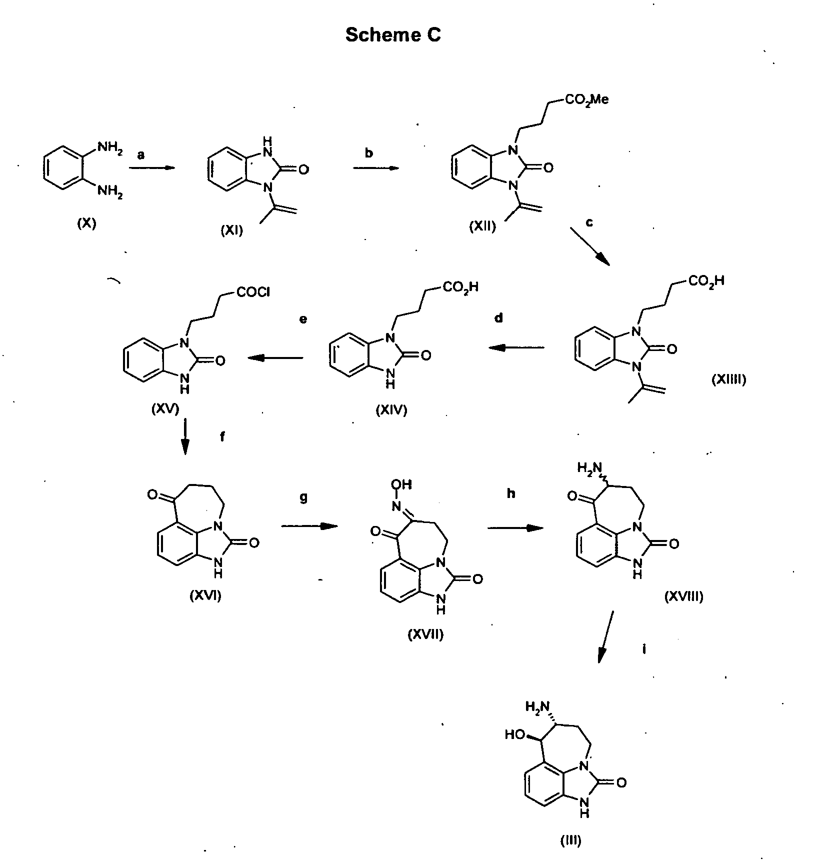 a) Ethyl acetoacetate, xylenes, 150°C; b) 4-Bromobutyric acid methyl ester, K₂CO₃, acetone, reflux; c) 15% NaOH, THF, reflux; d) Conc. HCl, THF; e) SOCl₂, DCM; f) AlCl₃, DCM, reflux; g) *t-*BuONO, HCI, AcOH, 40°C; h) Pd/C, H₂, MeOH, conc. HCl, 1.5 atm; i) NaBH₄, MeOH, 0°C;

The preparation of the compounds of formula (XI), (XII), (XIII), (XIV), (XV) and (XVI) is disclosed in Tetrahedron Letters, 1995, 36, 9, 1387. The preparation of the compounds of formula (XVII) and (III) is disclosed in US Patent, US-45857.70.

The enantiomers of the amino-alcohol (III) may be separated by chiral HPLC. N-protection facilitates the separation. Those skilled in the art will appreciate that a variety of N-protected compounds may be used, for example; the t-butyloxycarbamate prepared by reacting the amino-alcohol (III) with t-BOC-anhydride in a suitable solvent such as methanol, in the presence of a base such as triethylamine. Following chiral HPLC separation, the t-BOC protecting group may be removed by acid hydrolysis, for example, stirring in 4N HCI/dioxane at room temperature for several hours, typically 1 hour.

The desired enantiomer of the amino-alcohol (III) may also be prepared by the enantioselective reduction of the keto-oxime (XVII). Those skilled in the art will appreciate that the degree of enantioselectivity will depend on the catalyst, ligand, solvent and reaction temperature. Particularly useful conditions use hydrogen in the presence of a metal catalyst such as rhodium chloro(norbornadiene) dimer complexed with a ligand such as 1-[(*S*)-ferrocenyl-2-(*R*)-ethyl-1-dimethylamino)phenyl]-(*S*)-phosphino-1'-dicyclohexylphosphino-ferrocene (Solvias AG) in a protic solvent, typically aqueous methanol, at elevated temperatures, normally 80°C, for 10-40 hours, typically 16 hours.

### 3. Preparation of ketones (II)

Many of the methyl ketones of formula (II) used in the reductive amination procedure are commercially available. Those skilled in the art will appreciate that others may be prepared by experimental procedures as described in the literature.

### 3.1 Compounds wherein A-B is CH=CH

Enones of formula (VII) may be prepared according to the method illustrated in Scheme D from benzaldehydes of formula (XVII), wherein R³, R⁴, R⁵, R⁶ and R⁷ are as defined for formula (I), by a base catalysed condensation with acetone, typically using sodium hydroxide, as base, at 0°C.

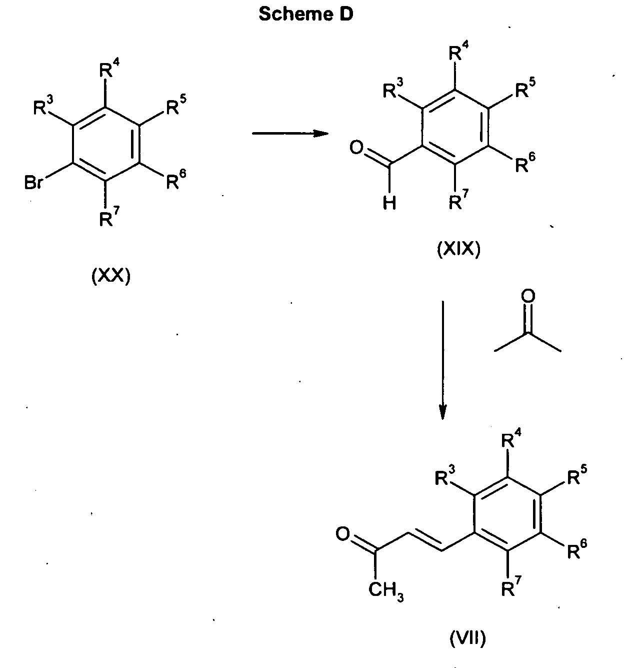

Substituted benzaldehydes of formula (XIX), can be obtained by lithiation of the aryl bromides (XX) using, for example, n-butyl lithium in tetrahydrofuran, followed by reaction of the aryl lithium reagent with *N,N*-dimethylformamide. Alternatively, enones of formula (VII) may be prepared by reaction of aldehydes of formula (XIX) with 1-triphenylphosphoranylidene-2-propanone at reflux in a suitable solvent, such as tetrahydrofuran for 5 - 20 hours, normally 12 hours.

### 3.2 Compounds wherein A-B is CH₂CH₂

Ketones of formula (II) wherein A-B is CH₂-CH₂ may be prepared from enones of formula (VII) wherein A-B is CH=CH using standard reducing agents, such as hydrogen in the presence of a metal catalyst such as palladium on alumina in a suitable solvent, for example ethyl acetate, or those described in "Handbook of Reagents for Organic Synthesis - Oxidising and Reducing Agents" edited by S.D.Burke and R.L.Danheiser, as illustrated in Scheme E.

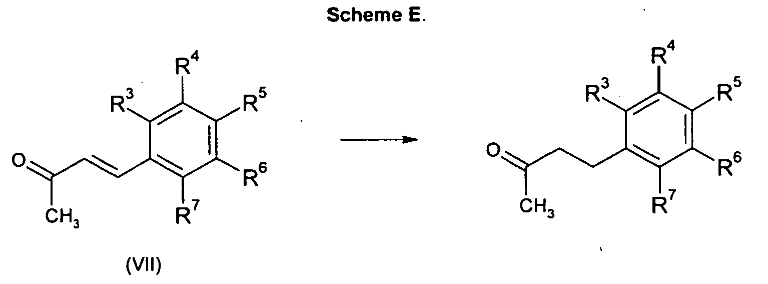

Ketones of formula (II) wherein A-B is CH₂-CH₂ may also be prepared by Heck coupling of the iodo compounds (XXI) with but-3-en-2-ol using Pd(OAc)₂ as catalyst in a suitable solvent, such as *N,N-*dimethylformamide, in the presence of a base, such as triethylamine, with optionally added inorganic salts, such as lithium chloride, as illustrated in Scheme F. The α,(β-enone, (VII), may be obtained as a byproduct of this Heck coupling reaction.

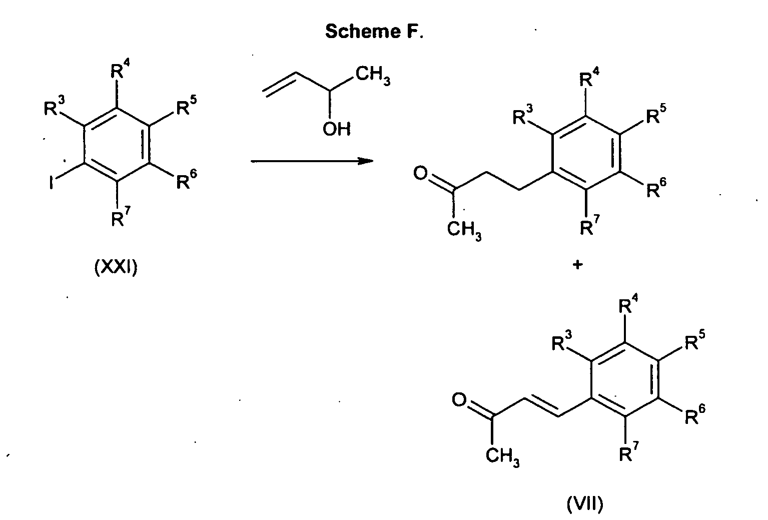

Compounds wherein R³ and R⁷ are both H, and R⁵ is OH, i.e. ketones of formula (XXII), wherein R⁴ and R⁶ are as defined for formula (I), may be obtained by reaction of the phenols of formula (XXIII) with methyl vinyl ketone in a suitable solvent, such as toluene, in the presence of an acid catalyst, typically sulphuric acid, as illustrated in Scheme G. Preferably, reagent addition occurs at 0°C followed by stirring of the reaction mixture for 2 - 24 hours, typically overnight.

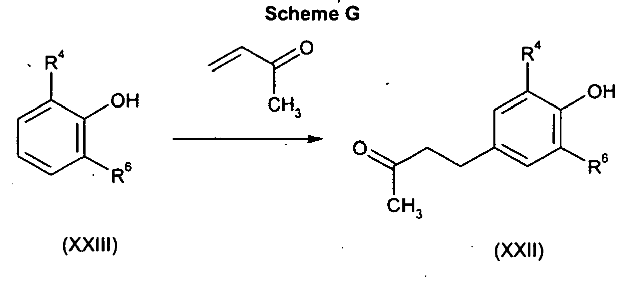

Compounds of formula (XXVIII), wherein W = -SO₂ or -CO and R¹⁰ is as defined for formula (I) may be prepared as shown in Scheme H.

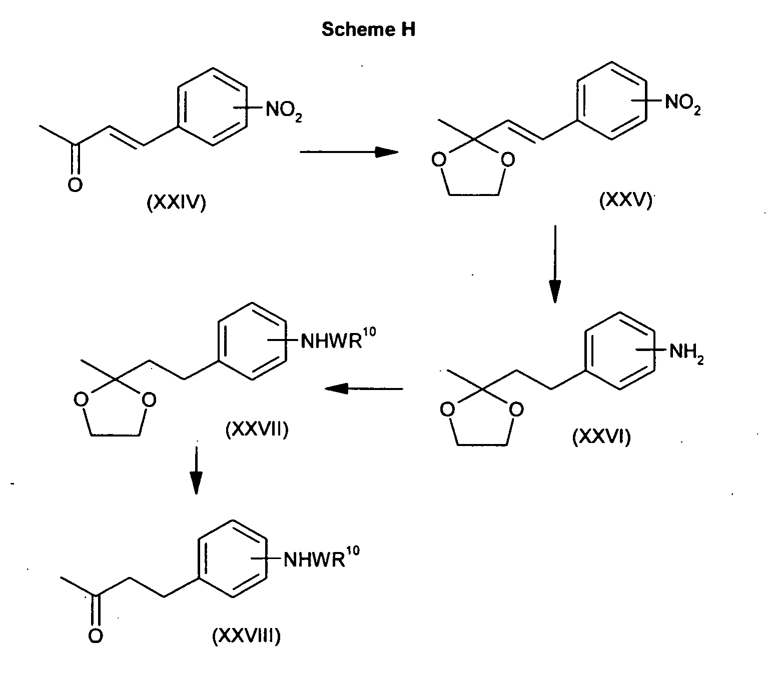

The enones of formula (XXIV) may be protected as the ethylene ketals of formula (XXV) by reaction with ethylene glycol in a suitable solvent, such as toluene, in the presence of an acid catalyst, such as p-toluene sulfonic acid by heating at reflux in a Dean-Stark apparatus for several hours, typically 5 hours. The amines of formula (XXVI) may be prepared from the compounds of formula (XXV) using standard reducing agents, such as hydrogen in the presence of a metal catalyst such as 10% palladium on carbon in a suitable solvent, for example methanol using a flow-through H-Cube hydrogenator, or those described in "Handbook of Reagents for Organic Synthesis - Oxidising and Reducing Agents" edited by S.D.Burke and R.L.Danheiser. The amines of formula (XXVI) may be acylated and sulphonylated using standard literature conditions well known to those skilled in the art. The ketals of formula (XXVII) may be deprotected by acid catalysed hydrolysis, for example, stirring in concentrated HCL/methanol at room temperature for several hours, typically 2 hours.

### 3.3 Compounds wherein A-B is CH₂-C(CH₃)₂

Ketones of formula (II), wherein A-B = -CH₂C(CH₃)₂-, may be prepared by the reductive arylation of 4-methylpent-3-en-2-one (XXIX) with benzenediazonium salts of formula (XXX) in a suitable aprotic solvent, such as *N,N*-dimethylformamide in the presence of a Lewis acid catalyst such as titanium tetrachloride, as illustrated in Scheme 1.

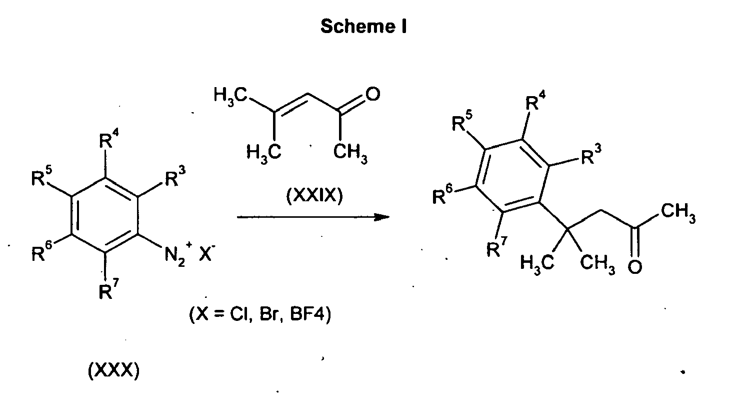

Compounds wherein R³ is OH, i.e. the methyl ketones (XXXI) may be prepared by the sequence of reactions shown in Scheme J

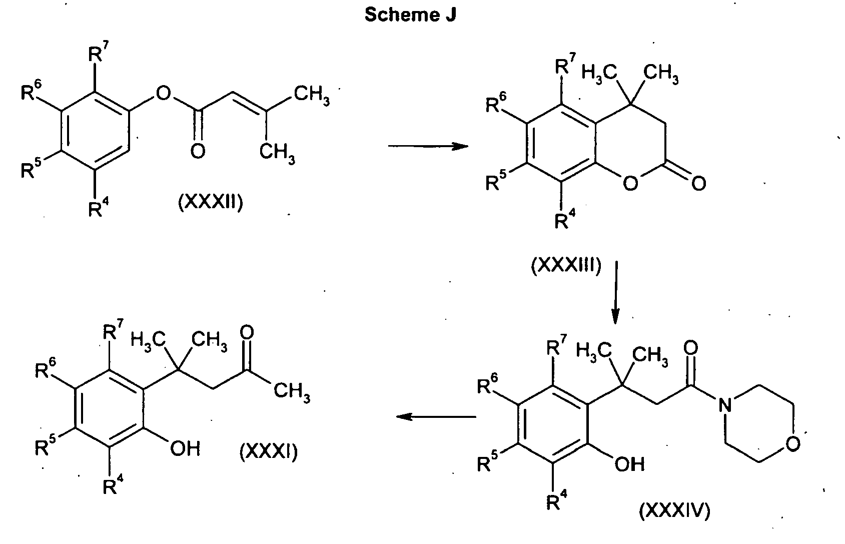

The chromanones of formula (XXXIII) may be prepared by stirring a solution of compounds (XXXII) in a suitable solvent, typically dichloroethane, in the presence of a Lewis acid, such as aluminium chloride, under nitrogen for 5 - 24 hours, typically overnight. The morpholinamides (XXXIV) are obtained by heating (XXXIII) in morpholine at elevated temperature, typically 85°C, for several hours, for example 2 hours. Reaction of the morpholinamides, (XXXIV), with methyl lithium in a suitable solvent, such as tetrahydrofuran, at reduced temperature, typically -60°C, under nitrogen yields the ketones of formula (XXXI).

### 3.4 Compounds wherein A-B is CH₂-O

Alkylation of a monohaloacetone with phenols of formula (XXXV), or the corresponding phenolate anion, in a suitable aprotic solvent, such as acetonitrile, optionally in the presence of a base, for example triethylamine gives ketones of formula (II) in which A-B = -CH₂O-, as illustrated in Scheme K.

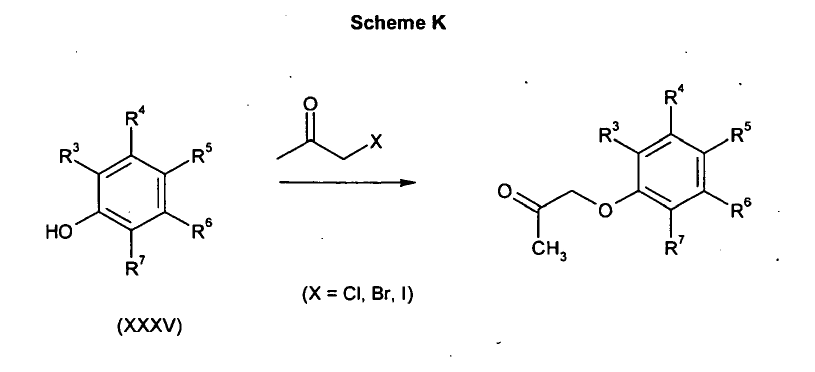

### 3.5 Compounds wherein A-B is -CH₂-CH₂-O

Ketones of formula (II), wherein A-B = -CH₂CH₂O-, may be prepared by the reaction of phenols of formula (XXXV) with methyl vinyl ketone using procedures similar to those described in J. Amer. Chem. Soc., 1971, 93, 4, 985, as illustrated in Scheme L.

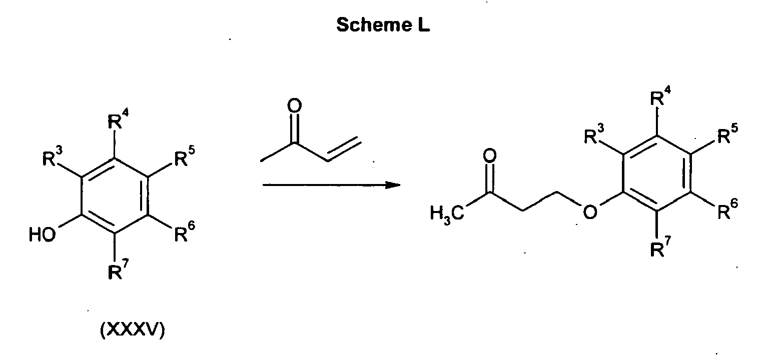

### 3.6 Compounds wherein A-B is CH₂-O-CH₂

Ketones of formula (II), wherein A-B = -CH₂OCH₂-, may be prepared by the alkylation of hydroxyacetone with the appropriate benzyl bromide, (XXXVI), by methods similar to those described in US-5360819, Example 36, as illustrated in Scheme M.

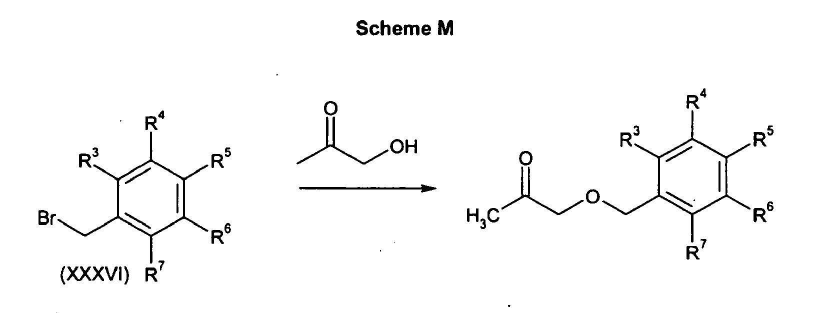

### 3.7 Compounds wherein A-B is CH₂CH₂-CH₂

Ketones of formula (II), wherein A-B = -CH₂CH₂CH₂- may be prepared by an organometallic coupling of, for example, an organozinc reagent of formula (XXXVII) with 4-chloro-2-butanone optionally in the presence of a copper catalyst and lithium salts, as illustrated in Scheme N. Those skilled in the art will recognise that other organometallic reagents may be used.

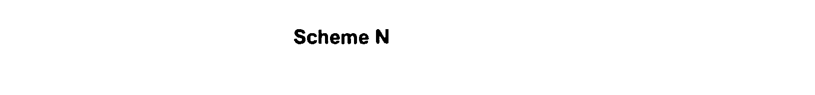

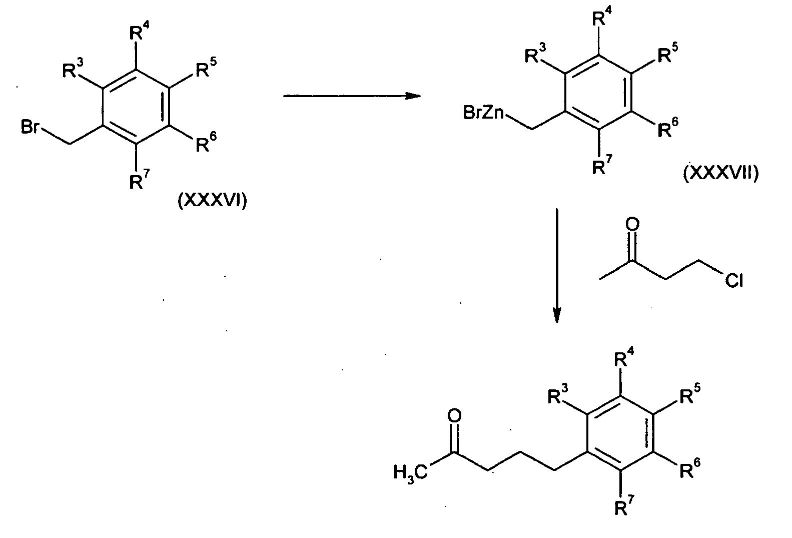

The orgaonozinc reagents of formula (XXXVII) may be prepared from the corresponding benzyl bromides using standard literature procedures.

### 4. Miscellaneous transformations

It will be appreciated that certain of the substituents on the phenyl ring of the compounds of formula (II) will be amenable to further synthetic manipulation. For example, the methods illustrated in Schemes G and I result in products that contain a phenolic hydroxyl group. Where the corresponding ethers are required, then O-alkylation of these phenols can be effected by reaction with the appropriate organic halides using a base, such as potassium carbonate, in a suitable solvent such as acetone. Reactions are stirred at elevated temperatures, typically reflux for several hours, typically overnight.

Phenols of formula (XXXVIII), wherein A and B are as defined for formula (I), readily undergo standard substitution reactions.

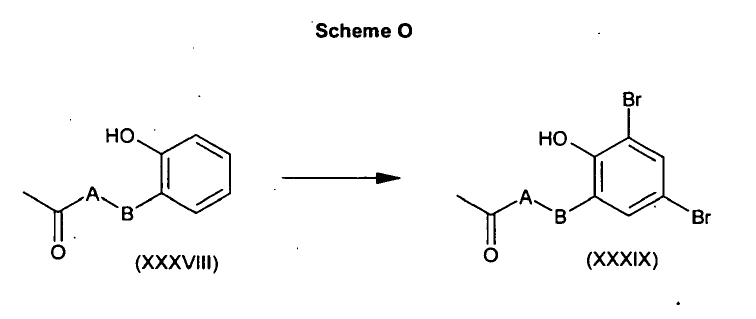

For example, compounds of formula (XXXIX) may be prepared by the reaction of compounds of formula (XXXVIII) with *N*-bromosuccinimide in a suitable solvent, such as *N,N*-dimethylformamide, at room temperature for 10 - 25 hours, typically 18 hours, as shown in Scheme O.

Amides of formula (XLI), wherein A and B are as defined for formula (I), may be prepared from the acids of formula (XL) as shown in Scheme P

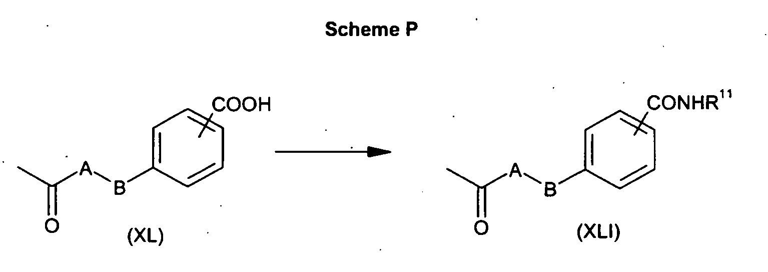

Those skilled in the art will recognise that many standard literature reaction conditions may be used to effect such amide formation; some of these are reviewed in "Amide bond formation and peptide coupling" C.A.G.N.Montalbetti and V.Falque, Tetrahedron, 2005, 61, 10827-10852. For example, the acids of formula (XL) may be converted to the corresponding acid chlorides by reaction with oxalyl chloride in a suitable solvent, such as *N, N*-dimethylformamide. These acid chlorides may be reacted with amines of formula R¹¹NH₂ in a suitable solvent such as dichloromethane.
It will also be appreciated by persons skilled in the art that, within certain of the processes described, the order of the synthetic steps employed may be varied and will depend *inter alia* on factors such as the nature of other functional groups present in a particular substrate, the availability of key intermediates, and the protecting group strategy (if any) to be adopted. Clearly, such factors will also influence the choice of reagent for use in the said synthetic steps.

The skilled person will appreciate that the compounds of the invention could be made by methods other than those herein described, by adaptation of the methods herein described and/or adaptation of methods known in the art, for example the art described herein, or using standard textbooks such as "Comprehensive Organic Transformations - A Guide to Functional Group Transformations", RC Larock, Wiley-VCH (1999 or later editions).

It is to be understood that the synthetic transformation methods mentioned herein are exemplary only and they may be carried out in various different sequences in order that the desired compounds can be efficiently assembled. The skilled chemist will exercise his judgement and skill as to the most efficient sequence of reactions for synthesis of a given target compound.

In a preferred embodiment. -A- is -CH₂- and -B- is -CH₂-, or -C(CH₃)₂-; or -A-B- is -CH=CH-. In a more preferred embodiment -A- is -CH₂- and -B- is -CH₂-.

When -A-B- is -CH=CH- then the double bond preferably has the *trans-* (or *E*-) configuration.

In another preferred embodiment of the compounds of formula (I), R⁸ is halo, -CN, C₁-C₄ alkyl, C₁-C₄ haloalkyl, -CH₂OH, -O-(C₁-C₄ alkyl), or -O-CH₂-(C₃-C₅)cycloalkyl, and R⁹ is -OH or -NHSO₂(C₁-C₃ alkyl).

In another preferred embodiment of the compounds of formula (I), R¹ is H and R² is methyl such that the compound of formula (I) has the 1'*R*, 6*R*, 7*R* relative configuration. More preferably the compound of formula (I) has the 1'*R*, 6*R*, 7*R* absolute configuration.

In another preferred embodiment of the compounds of formula (I), R³, R⁴, R⁵, R⁶ and R⁷ are each independently selected from H, R⁸ and R⁹, provided that at least two of R³, R⁴, R⁵, R⁶ and R⁷ are H; or R⁴ and R⁵ together are -O-CH₂-CH₂-, -CH₂-CH₂-O- or -O-CH₂-O-, and R³, R⁶ and R⁷ are H; R⁸ is halo, - CN, C₁-C₄ alkyl, -CF₃, -CH₂OH, -O-(C₁-C₄ alkyl), or -O-CH₂-(C₃-C₅)cycloalkyl; and R⁹ is -OH or -NHSO₂(C₁-C₃ alkyl).

In another preferred embodiment of the compounds of formula (I), one of R³, R⁴, R⁵, R⁶ and R⁷ is R⁸ or R⁹, another two of R³, R⁴, R⁵, R⁶ and R⁷ are H or R⁸, and the other two of R³, R⁴, R⁵, R⁶ and R⁷ are H; R⁸ is halo, -CN, C₁-C₄ alkyl, -CF₃, -CH₂OH, -O-(C₁-C₄ alkyl), or -O-CH₂-(C₃-C₅)cycloalkyl; and R⁹ is -OH or -NHSO₂(C₁-C₃ alkyl). More preferably R⁹ is -OH.

Another preferred embodiment is a compound of formula (I^{A}) 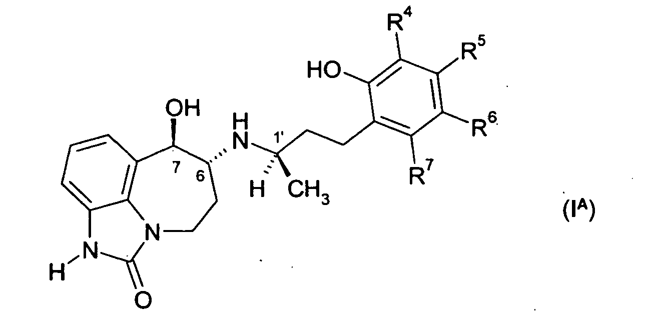 or a pharmaceutically acceptable salt thereof, wherein two of R⁴, R⁵, R⁸ and R⁷ are H or R⁸, and the other two of R⁴, R⁵, R⁶ and R⁷ are H; and R⁸ is halo, -CN, C₁-C₄ alkyl, -CF₃, -CH₂OH, -O-(C₁₋C₄ alkyl), or -O-CH₂-(C₃-C₅)cydoalkyl.

Another preferred embodiment is a compound of formula (I^{A}) or a pharmaceutically acceptable salt thereof that has the 1'R, 6R, 7R absolute configuration.

Another preferred embodiment is a compound of formula (I^{B}) 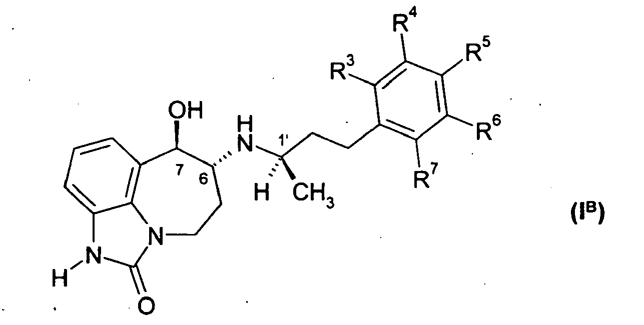 or a pharmaceutically acceptable salt thereof, wherein one of R³, R⁴, R⁵, R⁶ and R⁷ is R⁸ or R⁹, and the other four of R³, R⁴, R⁵, R⁶ and R⁷ are H; R⁸ is halo, -CN, (C₁-C₄)alkyl, -CF₃, -O-(C₁-C₄ alkyl), or -O-CH₂-(C₃-C₅)cycloalkyl; and R⁹ is -OH.

Another preferred embodiment is a compound of formula (I^{B}) or a pharmaceutically acceptable salt thereof that has the 1'*R*, 6*R*, 7*R* absolute configuration.

In embodiments of the compounds of formula (I), (I^{A}) and (1^{B}) wherein R⁸ is halo then preferably it is fluoro or chloro. In embodiments of the compounds of formula (I), (I^{A}) and (I^{B}) wherein R⁸ is (C₁-C₄)alkyl then preferably it is methyl, ethyl, propyl or isopropyl, and more preferably it is methyl. In embodiments of the compounds of formula (I), (I^{A}) and (I^{B}) wherein R⁸ is (C₁-C₄) haloalkyl then preferably it is trifluoromethyl. In embodiments of the compounds of formula (I), (I^{A}) and (I^{B}) wherein R⁸ is -O-(C₁-C-₄)alkyl then preferably it is methoxy, ethoxy, propoxy or isopropoxy, and more preferably it is methoxy. In embodiments of the compounds of formula (I), (I^{A}) and (I^{B}) wherein R⁸ is -O-CH₂-(C₃-C₅)cycloalkyl then preferably it is cyclopropylmethoxy.

Preferred individual compounds of formula (I) are:
(6*R**,7*R**)-7-hydroxy-6-{[(1*R**)-1-methyl-3-phenylpropyl]amino}-4,5,6,7-tetrahydroimidazo[4,5,1-*jk*][1]benzazepin-2(1*H*)-one;
(6*R**,7*R**)-7-hydroxy-6-{[(1*S**)1-methyl-3-phenylpropyl]amino}-4,5,6,7-tetrahydroimidazo[4,5,1-*jk*][1]benzazepin-2(1*H*)-one;
(6*R*,7*R*)-7-hydroxy-6-{[(1*RS*)-1-methyl-3-phen ylpropyl]amino}-4,5,6,7-tetrahydroimidazo[4,5,1-*jk*][1]benzazepin-2(1*H*)-one;
(6*R*,7*R*)-7-hydroxy-6-{[(1*R*)-1-methyl-3-phenylpropyl]amino)-4,5,6,7-tetrahydroimidazo[4,5,1-*jk*][1]benzazepin-2(1*H*)-one;
(6*R*,7*R*)-7-hydroxy-6-{[(1*S*)-1-methyl-3-phenylpropyl]amino}-4,5,6,7-tetrahydroimidazo[4,5,1-*jk*][1]benzazepin-2(1*H*)-one;
(6*R**,7*R**)-7-hydroxy-6-{[(1*R**)-3-(2-hydroxyphenyl)-1-methylpropyl]amino}-4,5,6,7-tetrahydroimidazo[4,5,1-*jk*][1]benzazepin-2(1*H*)-one;
(6*R**,7*R**)-7-hydroxy-6-{[(1*S**)-3-(2-hydroxyphenyl)-1-methylpropyl]amino}-4,5,6,7-tetrahydroimidazo[4,5,1-*jk*][1]benzazepin-2(1*H*)-one;
(6*R*,7*R*)-7-hydroxy-6-{[(1*RS*)-3-(2-hydroxyphenyl)-1-methylpropyl]amino}-4,5,6,7-tetrahydroimidazo[4,5,1-*jk*][1]benzazepin-2(1*H*)-one;
(6*R*,7*R*)-7-hydroxy-6-{[(1*R*)-3-(2-hydroxyphenyl)-1-methylpropyl]amino}-4,5,6,7-tetrahydroimidazo[4,5,1-*jk*][1]benzazepin-2(1*H*)-one;
(6*R*,7*R*)-7-hydroxy-6-{[(1*S*)-3-(2-hydroxyphenyl)-1-methylpropyl]amino}-4,5,6,7-tetrahydroimidazo[4,5,1-*jk*][1]benzazepin-2(1*H*)-one;
(6*R**,7*R**)-7-hydroxy-6-{[(1*R**)-3-(3-hydroxyphenyl)-1-methylpropyl]amino}-4.5.6.7-tetrahydroimidazo[4,5,1-*jk*][1]benzazepin-2(1*H*)-one;
(6*R**,7*R**)-7-hydroxy-6-{[(1*S**)-3-(3-hydroxyphenyl)-1-methylpropyl]amino}-4,5,6,7-tetrahydroimidazo[4,5,1-*jk*][1]benzazepin-2(1*H*)-one;
(6*R*,7*R*)-7-hydroxy-6-{[(1*RS*)-3-(3-hydroxyphenyl)-1-methylpropyl]amino}-4,5,6,7-tetrahydroimidazo[4.5,1-jk][1]benzazepin-2(1*H*)-one;
(6*R*,7*R*)-7-hydroxy-6-{[(1*R*)-3-(3-hydroxyphenyl)-1-methylpropyl]amino}-4,5,6,7-tetrahydroimidazo[4,5,1-*jk*][1]benzazepin-2(1*H*)-one;
(6*R*,7*R*)-7-hydroxy-6-{[(1*S*)-3-(3-hydroxyphenyl)-1-methylpropyl]amino}-4,5,6,7-tetrahydroimidazo[4,5,1-*jk*][1]benzazepin-2(1*H*-one;
(6*R**,7*R**)-7-hydroxy-6-{[(1*R**)-3-(4-hydroxyphenyl)-1-methylpropyl]amino}-4,5,6,7-tetrahydroimidazo[4,5,1-*jk*][1]benzazepin-2(1*H*)-one;
(6*R**,7*R**)-7-hydroxy-6-{[(1*S**)-3-(4-hydroxyphenyl)-1-methylpropyl]amirio}-4,5,6,7-tetrahydroimidazo[4,5,1-*jk*][1]benzazepin-2(1*H*)-one;
(6*R*,7*R*)-7-hydroxy-6-{[(1*RS*)-3-(4-hydroxyphenyl)-1-methylpropyl]amino}-4,5,6,7-tetrahydroimidazo[4,5,1-*jk*][1]benzazepin-2(1*H*)-one;
(6*R*,7*R*)-7-hydroxy-6-{[(1*R*)-3-(4-hydroxyphenyl)-1-methylpropyl]amino}-4,5,6,7-tetrahydroimidazo[4,5,1-*jk*][1]benzazepin-2(1*H*)-one;
(6*R*,7*R*)-7-hydroxy-6-{[(1*S*)-3-(4-hydroxyphenyl)-1-methylpropyl]amino}-4,5,6,7-tetrahydroimidazo[4,5,1-*jk*][1]benzazepin-2(1*H*)-one;
(6*R**,7*R**)-7-hydroxy-6-{[(1*R**)-3-(4-methoxyphenyl)-1-methylpropyl]amino}-4,5,6,7-tetrahydroimidazo[4,5,1-*jk*][1]benzazepin-2(1*H*)-one;
(6*R**,7*R**)-7-hydroxy-6-{[(1*S**)-3-(4-methoxyphenyl)-1-methylpropyl]amino}-4,5,6,7-tetrahydroimidazo[4,5,1-*jk*][1]benzazepin-2(1*H*)-one;
(6*R*,7*R*)-7-hydroxy-6-{[(1*RS*)-3-(4-methoxyphenyl)-1-methylpropyl]amino}-4,5,6,7-tetrahydroimidazo[4,5,1-*jk*][1]benzazepin-2(1*H*)-one;
(6*R*,7*R*)-7-hydroxy-6-{[(1*R*)-3-(4-methoxyphenyl)-1-methylpropyl]amino}-4,5,6,7-tetrahydroimidazo[4,5,1-*jk*][1]benzazepin-2(1*H*)-one;
(6*R*,7*R*)-7-hydroxy-6-{[(1*S*)-3-(4-methoxyphenyl)-1-methylpropyl]amino}-4,5,6,7-tetrahydroimidazo[4,5,1-*jk*][1]benzazepin-2(1*H*)-one:
(6*R*',7*R*')-6-{[(1*R**)-3-(4-(cyclopropylmethyloxy)phenyl)-1-methylpropyl]amino}-7-hydroxy-4,5,6,7-tetrahydroimidazo[4,5,1-*jk*][1]benzazepin-2(1*H*)-one;
(6*R**,7*R**)-6{[(1S*)-3-(4-(cyclopropylmethyloxy)phenyl)-1-methylpropyl]amino}-7-hydroxy-4,5,6,7-tetrahydroimidazo[4,5,1-*jk*][1]benzazepin-2(1*H*)-one;
(6*R*,7*R*)-6-{[(1*RS*)-3-(4-(cyclopropylmethyloxy)phenyl}-1-methylpropyl]amino}-7-hydroxy-4,5,6,7-tetrahydroimidazo[4,5,1-*jk*][1]benzazepin-2(1*H*)-one;
(6*R*,7*R*)-6-{[(1*R*)-3-(4-(cyclopropylmethyloxy)phenyl)-1-methylpropyl]amino}-7-hydroxy-4,5,6,7-tetrahydroimidazo[4,5,1-*jk*][1]benzazepin-2(1*H*)-one; and
(6*R*,7*R*)-6-{[(1*S*)-3-(4-(cyclopropylmethyloxy)phenyl)-1-methylpropyl]amino}-7-hydroxy-4,5,6,7-tetrahydroimidazo[4,5,1-*jk*][1]benzazepin-2(1*H*)-one.

Further preferred individual compounds of formula (I) are:
(6*R**,7*R**)-7-hydroxy-6-{(1*R**)-[3-(4-hydroxyphenyl)-1-methylpropyl]amino}-4,5,6,7-tetrahydro-imidazo[4,5,1-*jk*][1]benzazepin-2(1*H*)-one
(6*R**,7*R**)-7-hydroxy-6-{(1*S**)-[3-(4-hydroxyphenyl)-1-methylpropyl]amino}-4,5,6,7-tetrahydro-imidazo[4,5,1-*jk*][1]benzazepin-2(1*H*)-one
(6*R*,7*R*)-7-hydroxy-6-{(1*RS*)-[3-(4-hydroxyphenyl)-1-methylpropyl]amino}-4,5,6,7-tetrahydro-imidazo[4,5,1-*jk*][1]benzazepin-2(1*H*)-one
(6*R*,7*R*)-7-hydroxy-6-{(1*S*)-[3-(4-hydroxyphenyl)-1-methylpropyl]amino}-4,5,6,7-tetrahydroimidazo[4,5,1-*jk*][1]benzazepin-2(1*H*)-one
(6*R*,7*R*)-7-hydroxy-6-{(1*R*)-[3-(4-hydroxyphenyl)-1-methylpropylJamino}-4,5,6,7-tetrahydroimidazo[4,5,1-*jk*][1]benzazepin-2(1*H*)-one
(6*R**,7*R**)-7-hydroxy-6-{[(1*R**)-3-(2-hydroxyphenyl)-1-methylpropyl]amino}-4,5,6,7-tetrahydro-imidazo[4,5,1-*jk*][1]benzazepin-2(1*H*)-one
(6*R**,7*R**)-7-hydroxy-6-{[(1*S**)-3-(2-hydroxyphenyl)-1-methylpropyl]amino}-4,5,6,7-tetrahydro-imidazo[4,5,1-*jk*][1]benzazepin-2(1*H*)-one,
(6*R*,7*R*)-7-hydroxy-6-{[(1*RS*)-3-(2-hydroxyphenyl)-1-methylpropyl]amino}-4,5,6,7-tetrahydro-imidazo[4,5,1-*jk*][1]benzazepin-2(1*H*)-one,
(6*R*,7*R*)-7-hydroxy-6-{[(1*R*)-3-(2-hydroxyphenyl)-1-methylpropyl]amino}-4,5,6,7-tetrahydroimidazo[4,5,1-*jk*][1]benzazepin-2(1*H*)-one,
(6*R*,7*R*)-7-hydroxy-6-{[(1*S*)-3-(2-hydroxyphenyl)-1-methylpropyl]amino}-4,5,6,7-tetrahydroimidazo[4,5,1-*jk*][1]benzazepin-2(1*H*)-one,
(6*R**,7*R**)-6-{[(1*R**)-3-(5-fluoro-2-hydroxyphenyl)-1-methylpropyl]amino}-7-hydroxy-4,5,6,7-tetrahydro-imidazo[4,5,1-*jk*][1]benzazepin-2(1*H*)-one,
(6*R**,7*R**)-6-{[(1*S**)-3-(5-fluoro-2-hydroxyphenyl)-1-methylpropyl]amino]-7-hydroxy-4,5,6,7-tetrahydro-midazo[4,5,1-*jk*][1]benzazepin-2(1*H*)-one,
(6*R*,7*R*)-6-{[(1*RS*)-3-(5-fluoro-2-hydroxyphenyl)-1-methylpropyl]amino}-7-hydroxy-4,5,6,7-tetrahydro-imidazo[4,5,1-*jk*][1]benzazepin-2(1*H*)-one,
(6*R*,7*R*)-6-{[(1*R*)-3-(5-fluoro-2-hydroxyphenyl)-1-methylpropyl]amino}-7-hydroxy-4,5,6,7-tetrahydro-imidazo[4,5,1-*jk*][1]benzazepin-2(1*H*)-one,
(6*R*,7*R*)-6-{[(1*S*)-3-(5-fluoro-2-hydroxyphenyl)-1-methylpropyl]amino}-7-hydroxy-4,5,6,7-tetrahydro-imidazo[4,5,1-*jk*][1]benzazepin-2(1*H*)-one,
(6*R**,7*R**)-6-{[(1*R**)-3-(4,5-difluoro-2-hydroxyphenyl)-1-methylpropyl]amino}-7-hydroxy-4,5,6,7-tetrahydroimidazo[4,5,1-*jk*][1]benzazepin-2(1*H*)-one,
(6*R**,7*R**)-6-{[(1*S**)-3-(4,5-difluoro-2-hydroxyphenyl)-1-methylpropyl]amino}-7-hydroxy-4,5,6,7-tetrahydro-imidazo[4,5,1-*jk*[1]benzazepin-2(1*H*)-one,
(6*R*,7*R*)-6-{[(1*RS*)-3-(4,5-difluoro-2-hydroxyphenyl)-1-methylpropyl]amino}-7-hydroxy-4,5,6,7-tetrahydro-imidazo[4,5,1-*jk*][1]benzazepin-2(1*H*)-one,
(6*R*,7*R*)-6-{[(1*R*)-3-(4,5-difluoro-2-hydroxyphenyl)-1-methylpropyl]amino}-7-hydroxy-4,5,6,7-tetrahydro-imidazo[4,5,1-*jk*][1]benzazepin-2(1*H*)-one, and
(6*R*,7*R*)-6-{[(1*S*)-3-(4,5-difluoro-2-hydroxyphenyl)-1-methylpropyl]amino}-7-hydroxy-4,5,6,7-tetrahydro-imidazo[4,5,1-*jk*][1]benzazepin-2(1*H*)-one.

The compounds of formula (I) are agonists at the beta-2 adrenoceptor. In particular they have good efficacy at the bovine and/or porcine beta-2 adrenoceptor, as demonstrated in the assays set out below in the Examples.

The compounds of formula (I) may be used to improve meat production in livestock animals. Examples of livestock animals include ruminants such as cows, bulls, heifers, steers, goats, sheep and minor species such as buffalo, bison and antelopes. Other examples include pigs, boars, gilts, sows and avians such as chickens, ducks, geese and turkeys. A preferred use is in the improvement of meat production in cattle, swine and poultry.

Beta-2 agonists have also been reported to improve muscle production and feed efficiency in farmed fish. Accordingly, the compounds of formula (I) may find use in the production of fish such as, for example, tuna, salmon and trout.

The compounds of formula (I) may be administered to the animal by any suitable route. A preferred route of administration for improving meat production in livestock animals is the oral route. For such administration, the compounds of formula (I) may be added to the animals' food, drinking water, or any other material ingested by the animals, such as a salt lick.

The compounds of formula (I) may be added directly to the feed or drinking water, or may be presented as a concentrate for addition to the feed or drinking water.

The concentrate may be a solid or a liquid. Solid concentrates include simple mixtures of the compounds with a solid diluent such as corn starch, and compositions wherein the compounds are adsorbed onto the diluent. Examples of other diluents include alfalfa meal, rice hulls, corncob grits, bone meal, soybean meal, ground corn; inorganic diluents such as limestone, sodium chloride; vitamin and mineral mixes. Liquid concentrates include solutions and suspensions in water or another suitable vehicle, such as an oil, especially a vegetable oil.

A suitable concentrate for addition to feed comprises:

| | | |
|---|---|---|
| Active agent | 0.1 to 2wt% | for example 0.5wt% |
| Crushed limestone | 0.5 to 9wt% | for example 4.5wt% |
| Rice hulls | 90 to 99wt% | for example 94.5wt% |
| Mineral oil | 0.1 to 3wt% | for example 1wt% |

The concentration of the compound of formula (I) in the feed or water should be adjusted such that each animal receives a maximally effective amount. For cattle, an intake of between 0.1 and 1000mg/animal/day, particularly 0.1 to 100mg/animal/day, may be suitable. Preferably the amount may be between 0.5 and 50mg/animal/day, and more preferably between 1 and 25mg/animal/day. For cattle consuming 10kg of feed per day, these administration rates can be achieved by adding the compounds of formula (I) to the feed at an inclusion rate of 0.01 to 100ppm, 0.01 to 10ppm, 0.05 to 5ppm, and 0.1 to 2.5ppm respectively.

Compounds of the present invention may be administered alone or in combination with one or more other compounds of the invention or in combination with one or more other drugs (or as any combination thereof).

For example, compounds of formula (I) may be used in combination with other feed additives used in livestock production; for example, polyether ionophores such as monensin, salinomycin, narasin, lasalocid and laidlomycin; antibiotics such as the tetracyclines, bacitracin, tylosin, tiamulin, lincomycin, virginiamycin, quinolone antibacterials and carbadox; melengesterol acetate; agents for the prevention or treatment of sub-acute rumen acidosis such as sodium bicarbonate, acarbose and other amylase or glucosidase inhibitors; carcass quality / anabolic agents such as ractopamine, salbutamol, almeterol and other beta adrenergic ligands; enzymes, minerals, vitamins and other supplements. The man skilled in the art will recognise that the agents listed above are examples of a wide range of feed additives which may be used in combination with compounds of formula (I). Other examples are referred to in "2006 Feed Additive Companion" and "Handbook of Feed Additives 2006".

Compounds of formula (I) may also be used in combination with anabolic agents such as zearanol, trenbolone acetate and oestradiol; and growth hormones such as bovine somatotropin and porcine somatotropin. Compounds of formula (I) may also be used in combination with agents used in animal welfare; for example endectocides such as ivermectin, doramectin, moxidectin, abamectin and other macrocyclic lactones; anthelmintics such as levamisole, albendazole and other benzimidazole carbamates, morantel, pyrantel; ectoparasiticides such as pyrethroids, arylpyrazoles, neonicotinoids.

The compounds of formula (I) may also be used in the treatment of diseases of animals in which beta-2 agonists have, or may have, a beneficial effect. In particular, the compounds of formula (I) may be used in the treatment of respiratory diseases of animals, including the treatment of heaves in horses.

The compounds of formula (I) also have agonist activity at the human beta-2 adrenoceptor and so are potentially useful in human medicine.

Beta-2 agonists are currently used to treat allergic and non-allergic airways diseases such as asthma and chronic obstructive airways disease (COPD). Treatment guidelines for these diseases include both short and long acting inhaled beta-2 agonists. Short acting, rapid onset beta-2 agonists are used for "rescue" bronchodilation, whereas, long-acting forms provide sustained relief and are used as maintenance therapy.

Bronchodilation is mediated via agonism of the beta-2 adrenoceptor expressed on airway smooth muscle cells, which results in relaxation and hence bronchodilation. Thus, as functional antagonists, beta-2 agonists can prevent and reverse the effects of all bronchoconstrictor substances, including leukotriene D4 (LTD4), acetylcholine, bradykinin, prostaglandins, histamine and endothelins. Because beta-2 receptors are so widely distributed in the airway, beta-2 agonists may also affect other types of cells that play a role in asthma. For example, it has been reported that beta-2 agonists may stabilize mast cells. The inhibition of the release of bronchoconstrictor substances may be how beta-2 agonists block the bronchoconstriction induced by allergens, exercise and cold air. Furthermore, beta-2 agonists inhibit cholinergic neurotransmission in the human airway, which can result in reduced cholinergic-reflex bronchoconstriction.

Therefore, a further aspect of the present invention relates to the compounds of formula (I), or pharmaceutically acceptable salts thereof, for use in the treatment of diseases, disorders, and conditions in which the beta-2 receptor is involved. More specifically, the present invention also concerns the compounds of formula (I), or pharmaceutically acceptable salts thereof, for use in the treatment of diseases, disorders, and conditions selected from the group consisting of :
- asthma of whatever type, etiology, or pathogenesis, in particular asthma that is a member selected from the group consisting of atopic asthma, non-atopic asthma, allergic asthma, atopic bronchial IgE-mediated asthma, bronchial asthma, essential asthma, true asthma, intrinsic asthma caused by pathophysiologic disturbances, extrinsic asthma caused by environmental factors, essential asthma of unknown or inapparent cause, bronchitic asthma, emphysematous asthma, exercise-induced asthma, allergen induced asthma, cold air induced asthma, occupational asthma, infective asthma caused by bacterial, fungal, protozoal, or viral infection, non-allergic asthma, incipient asthma, wheezy infant syndrome and bronchiolytis,
- chronic or acute bronchoconstriction, chronic bronchitis, small airways obstruction, and emphysema,
- obstructive or inflammatory airways diseases of whatever type, etiology, or pathogenesis, in particular an obstructive or inflammatory airways disease that is a member selected from the group consisting of chronic eosinophilic pneumonia, chronic obstructive pulmonary disease (COPD), COPD that includes chronic bronchitis, pulmonary emphysema or dyspnea associated or not associated with COPD, COPD that is characterized by irreversible, progressive airways obstruction, adult respiratory distress syndrome (ARDS), exacerbation of airways hyper-reactivity consequent to other drug therapy and airways disease that is associated with pulmonary hypertension,
- bronchitis of whatever type, etiology, or pathogenesis, in particular bronchitis that is a member selected from the group consisting of acute bronchitis, acute laryngotracheal bronchitis, arachidic bronchitis, catarrhal bronchitis, croupus bronchitis, dry bronchitis, infectious asthmatic bronchitis, productive bronchitis, staphylococcus or streptococcal bronchitis and vesicular bronchitis,
- acute lung injury,
- bronchiectasis of whatever type, etiology, or pathogenesis, in particular bronchiectasis that is a member selected from the group consisting of cylindric bronchiectasis, sacculated bronchiectasis, fusiform bronchiectasis, capillary bronchiectasis, cystic bronchiectasis, dry bronchiectasis and follicular bronchiectasis.

In addition to the airways, it has also been established that beta-2 adrenoceptors are also expressed in other organs and tissues and thus the compounds of formula (I) may have application in the treatment of other diseases such as, but not limited to those of the nervous system, premature labor, congestive heart failure, depression, inflammatory and allergic skin diseases, psoriasis, proliferative skin diseases, glaucoma and in conditions where there is an advantage in lowering gastric acidity, particularly in gastric and peptic ulceration.

When used in human therapy, the compounds of formula (I) and their pharmaceutically acceptable salts will generally be administered as a formulation in association with one or more pharmaceutically acceptable excipients. The term "excipient" is used herein to describe any ingredient other than the compound of the invention. The choice of excipient will to a large extent depend on the particular mode of administration.

The compounds of the invention may be administered orally. Oral administration may involve swallowing, so that the compound enters the gastrointestinal tract, or buccal or sublingual administration may be employed by which the compound enters the blood stream directly from the mouth.

Formulations suitable for oral administration include: solid formulations such as tablets; capsules containing particulates, liquids, or powders; lozenges'(including liquid-filled); and chews; multi- and nano-particulates; gels; solid solutions; liposomes; films, ovules, sprays and liquid formulations.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986, by Liang and Chen (2001).

For tablet dosage forms, depending on dose, the drug may make up from 1 weight % to 80 weight % of the dosage form, more typically from 5 weight % to 60 weight % of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet.

Other possible ingredients include anti-oxidants, colourants, flavouring agents, preservatives and taste-masking agents.

Exemplary tablets contain up to about 80% drug, from about 10 weight % to about 90 weight % binder, from about 0 weight % to about 85 weight % diluent, from about 2 weight % to about 10 weight % disintegrant, and from about 0.25 weight % to about 10 weight % lubricant.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in Pharmaceutical Dosage Forms: Tablets, Vol. 1, by H. Lieberman and L. Lachman (Marcel Dekker, New York, 1980).

Consumable oral films for human use are typically pliable water-soluble or water-swellable thin film dosage forms which may be rapidly dissolving or mucoadhesive and typically comprise a compound of formula (I), a film-forming polymer, a binder, a solvent, a humectant, a plasticiser, a stabiliser or emulsifier, a viscosity-modifying agent and a solvent. Some components of the formulation may perform more than one function.

The compound of formula (I) may be water-soluble or insoluble. A water-soluble compound typically comprises from 1 weight % to 80 weight %, more typically from 20 weight % to 50 weight %, of the solutes. Less soluble compounds may comprise a greater proportion of the composition, typically up to 88 weight % of the solutes. Alternatively, the compound of formula (I) may be in the form of multiparticulate beads.

The film-forming polymer may be selected from natural polysaccharides, proteins, or synthetic hydrocolloids and is typically present in the range 0.01 to 99 weight %, more typically in the range 30 to 80 weight %.

Other possible ingredients include anti-oxidants, colorants, flavourings and flavour enhancers, preservatives, salivary stimulating agents, cooling agents, co-solvents (including oils), emollients, bulking agents, anti-foaming agents, surfactants and taste-masking agents.

Films in accordance with the invention are typically prepared by evaporative drying of thin aqueous films coated onto a peelable backing support or paper. This may be done in a drying oven or tunnel, typically a combined coater dryer, or by freeze-drying or vacuuming.

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in US Patent No. 6,106,864. Details of other suitable release technologies such as high energy dispersions and osmotic and coated particles are to be found in Pharmaceutical Technology On-line, 25(2), 1-14, by Verma et al (2001). The use of chewing gum to achieve controlled release is described in WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, ' carbohydrates and buffering agents (preferably to a pH of from 3 to 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of formula (I) used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLApoly(dl-lactic-coglycolic)acid (PGLA) microspheres.

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958 by Finnin and Morgan (October 1999).

Other means of topical administration include delivery by electroporation, iontophoresis, phonophoresis, sonophoresis and microneedle or needle-free (e.g. Powderject™, Bioject™, etc.) injection.

Formulations for topical administration may be formulated to be immediate and/or modified release. Modified release formulations include delayed-, sustained-, pulsed-, controlled-, targeted and programmed release.

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or hydroxypropylmethylcellulose), blisters and cartridges for use in an inhaler or insufflator may be formulated to contain a powder mix of the compound of the invention, a suitable powder base such as lactose or starch and a performance modifier such as I-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1µg to 20mg of the compound of the invention per actuation and the actuation volume may vary from 1µl to 100µl. A typical formulation may comprise a compound of formula (I), propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

Formulations for inhaled/intranasal administration may be formulated to be immediate and/or modified release using, for example, PGLA. Modified release formulations include delayed-, sustained-, pulsed-, controlled-, targeted and programmed release.

In the case of dry powder inhalers and aerosols, the dosage unit is determined by means of a valve which delivers a metered amount. Units in accordance with the invention are typically arranged to administer a metered dose or "puff" containing from 0.001mg to 10mg of the compound of formula (I). The overall daily dose will typically be in the range 0.001mg to 40mg which may be administered in a single dose or, more usually, as divided doses throughout the day.

The compounds of formula (I) are particularly suitable for an administration by inhalation.

The compounds of the invention may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

The compounds of the invention may also be administered directly to the eye or ear, typically in the form of drops of a micronised suspension or solution in isotonic, pH-adjusted, sterile saline. Other formulations suitable for ocular and aural administration include ointments, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

The compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, i.e. as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

For administration to human patients, the total daily dose of the compounds of the invention is typically in the range 0.001 mg to 5000mg depending, of course, on the mode of administration. For example, an intravenous daily dose may only require from 0.001 mg to 40mg. The total daily dose may be administered in single or divided doses and may, at the physician's discretion, fall outside of the typical range given herein.

These dosages are based on an average human subject having a weight of about 65kg to 70kg. The physician will readily be able to determine doses for subjects whose weight falls outside this range, such as infants and the elderly.

When used for the treatment of human airway disease, the compounds of formula (I) and their pharmaceutically acceptable salts may advantageously be used in combination with a second pharmacologically active agent. Examples of such agents include: H3 antagonists, muscarinic M3 receptor antagonists, PDE4 inhibitors, glucocorticosteroids, adenosine A2a receptor agonists, modulators of cytokine signalling pathyways such as p38 MAP kinase or syk kinase, and leukotriene antagonists (LTRAs) including antagonists of LTB₄, LTC₄, CTD₄, and LTE₄.

Particularly preferred agents for such combination therapy are:
- glucocorticosteroids, in particular inhaled glucocorticosteroids with reduced systemic side effects, including prednisone, prednisolone, flunisolide, triamcinolone acetonide, beclomethasone dipropionate, budesonide, fluticasone propionate, ciclesonide, and mometasone furoate, and
- muscarinic M3 receptor antagonists or anticholinergic agents including in particular ipratropium salts, namely bromide, tiotropium salts, namely bromide, oxitropium salts, namely bromide, perenzepine, and telenzepine.

### EXAMPLES

The following non-limiting Examples illustrate the preparation of compounds of the formula (I).

In the following experimental details, nuclear magnetic resonance spectral data were obtained using Varian Inova 300, Varian lnova 400, Varian Mercury 400, Varian Unityplus 400, Bruker AC 300MHz, Bruker AM 250MHz or Varian T60 MHz spectrometers, the observed chemical shifts being consistent with the proposed structures. N.m.r chemical shifts are quoted in p.p.m downfield from tetramethylsilane. In the data presented below, for some compounds, only key n.m.r. signals are listed. In the following Examples, where an Example is indicated as being a mixture of diastereoisomers, then the n.m.r. integrals shown refer to the relative ratio of integrals for the quoted chemical shift. Mass spectral data were obtained on a Finnigan Masslab Navigator, a Fisons Instrument Trio 1000, or a Hewlett Packard GCMS System Model 5971 spectrometer. The calculated and observed ions quoted refer to the isotopic composition of lowest mass. HPLC means high performance liquid chromatography. Where indicated, the following analytical HPLC methods have been used:
*HPLC Method A*:
   Gilson system, 150 x 4.6 mm Gemini C18 5µm column;
   Acetonitrile : 0.1% aqueous ammonia [5:95 to 95:5], 1 ml/min.
*HPLC Method B:*
   Gilson system, 150 x 4.6 mm LUNA C18(2) 5µm column;
   Acetonitrile : ammonium formate (20 mM) [5:95 to 98:2], 1 ml/min.
*HPLC Method C*:
   Gilson system, 250 x 4.6 mm Chiralcel OD-H 5µm column;
   Ethanol : hexane [20:80], 1 ml/min.
*HPLC Method D:*
   Gilson system, 250 x 4.6 mm ID Chiralpak AD-H, 5µm column;
   Methanol : ethanol : hexane [5:15:80] with 0.1% v/v triethylamine, 1 ml/min.
*HPLC Method E:*
   Gilson system, 250 x 4.6 mm ID Chiralpak OD-H, 5µm column;
   Ethanol : hexane [20:80] with 0:1 % v/v triethylamine, 1 ml/min
*HPLC Method F:*
   Gilson system, 250 x 4.6 mm ID Chiralpak OD-H, 5µm column;
   Ethanol : hexane [20:80], 1 ml/min.

### Biological Test

Compounds of the present invention have been found to display activity in a cAMP assay selective for the bovine and porcine beta-2 adrenoceptors.

CHO cells transfected with the bovine or porcine beta-2 adrenceptors were maintained in culture in DMEM/HAMS F12 + 10% FBS + 2mM glutamine + 500µg/ml geneticin (for the porcine receptor the medium was supplement with 1.5mM HEPES) at 37°C with a 5% CO₂ atmosphere.

Cells were plated into 96 well viewplates in medium and incubated overnight at 37°C with a 5% CO₂ atmosphere. The cells were pre-incubated with 0.5mM IBMX in PBS for 30 minutes prior to incubation with increasing concentrations of experimental compound (5 x 10⁻¹² to 10⁻⁵M) for 30 minutes at 37°C with a 5% CO₂ atmosphere. At the end of the incubation time the compound was removed and the cells assayed for cAMP using the DiscoveRx Hit Hunter cAMP II™ assay kit.

Duplicate samples were run for each experimental compound and the data generated was analysed using Echo analysis software in Graphpad Prism.

Room temperature means 20 to 25°C. N/A indicates no data available.

In the following Examples, structures are depicted as follows:

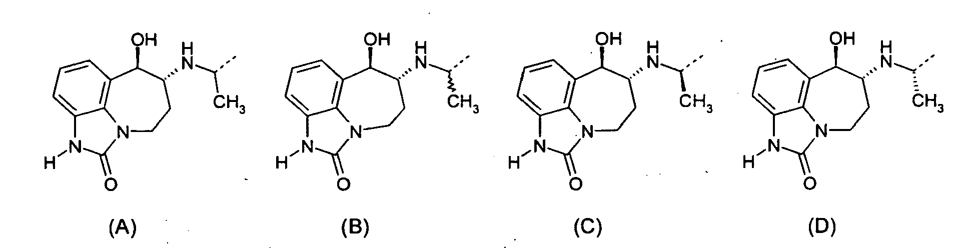

Unless specified otherwise, the wedge and dashed bonds indicate relative stereochemistry only. In particular, the 7-hydroxyl and the 6-N-substituent-are oriented in a *trans* configuration, but the structures encompass both the 6*R*,7*R* and 6*S*,7*S* stereoisomers. Formula (A) represents a compound which is a mixture of epimers at the carbon atom bearing the methyl substituent. Formula (B) represents a compound which is a single, unidentified epimer at the carbon atom bearing the methyl substituent. Formulae (C) and (D) represent single epimers of known relative configuration. Thus, formula (A) represents a compound that is a mixture of (C) and (D), while (B) represents a compound that is either (C) or (D).

### Example 1

### 6-({3[4-(Cyclopropylmethoxy)phenyl]-1-methylpropyl}amino)-7-hydroxy-4,5,6,7-tetrahydro-imidazo[4,5,1-jk][1]benzazepin-2(1H)-one

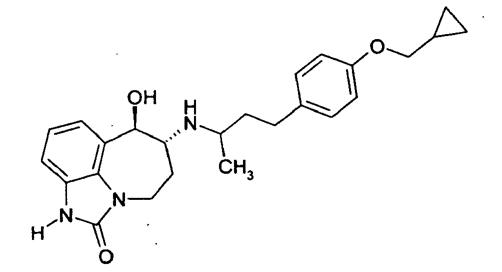

A mixture of the compound of Preparation 1 (100 mg, 0.4 mmol), triethylamine (80 µl, 0.6 mmol) and the compound of Preparation 12 (204 mg, 0.9 mmol) in methanol (2 ml) was stirred at room temperature for 18 h. Sodium borohydride (44 mg, 1.2 mmol) was added carefully and the reaction mixture was stirred at room temperature for another 18 h. The mixture was diluted with methanol (8 ml) and Amberlyst® 15 ion-exchange resin (3 g, prepared according to J. Org. Chem. 1998, 63, 3471-3473) was added. The mixture was shaken overnight and the solution was filtered off. The resin was washed with methanol (3 x 20 ml) and treated with ammonia in methanol (2N, 5 ml) to release the captured amino-alcohols. After shaking for 2 h, the solution was filtered off and the resin was washed with ammonia in methanol (2N, 2 x 5 ml). The combined methanol/ammonia washings were concentrated *in* vacuo to give the crude product. The residue was dissolved in acetonitrile : water (1:1, 1 ml) and purified by automated preparative liquid chromatography (Gilson system, 150 mm x 21.4 mm Gemini 5µm column), 20 ml/min, using an acetonitrile : 0.1% aqueous ammonia (1:9) : acetonitrile : 0.1% aqueous ammonia (9:1) gradient [100:0 to 20:80 from 2 to 20 min; 20:80 to 0:100 from 20 to 25 min]. The appropriate fractions were combined and concentrated to give the compound of Example 1a (60 mg) as a mixture of 4 diastereoisomers.

### Alternative route

To a suspension of the compound of Preparation 1 (15.5 g, 60.5 mmol) in methanol (80 ml) was added potassium hydroxide (1.0 g, 18.2 mmol). After stirring for 30 min, a solution of the compound of Preparation 12 (14.5 g, 66.5 mmol), in methanol (80 ml), was added and the reaction mixture was cooled to 0°C. Sodium cyanoborohydride (5.7 g, 90.7 mmol) was added and the reaction mixture was stirred at 0°C for 30 min and then at room temperature for 48 h. The reaction mixture was quenched with water (30 ml) and concentrated *in vacuo.* A portion of the residue was purified by automated flash chromatography (Biotage™ 40M cartridge, wet with dichloromethane) with gradient elution, dichloromethane : methanol [1:0 to 8:2]. The appropriate fractions were combined and concentrated to give the compound of Example 1b (540 mg) as a pair of enantiomers.

To a solution of the compound of Example 1b (540 mg, 1.3 mmol) in methanol (5 ml) was added dropwise hydrogen chloride in diethyl ether (1M, 1.3 ml). After stirring for 1 h, diethyl ether (3 ml) was added dropwise and the precipitate was collected by filtration and washed with diethyl ether. The solid was re-dissolved in warm methanol (8 ml) and precipitated with diethyl ether. After washing with diethyl ether, the solid was dried in a vacuum oven to give the compound of Example 1c (255 mg).

| Example | Structure Comment | MH⁺ exper | MH⁺ expect | Bovine EC₅₀ nM | Porcine EC₅₀ nM |
|---|---|---|---|---|---|
| **1a** | Mixture of 4 diastereoisomers | 422.5 | 422.2 | 14 | 5.9 |
| **1c** | Second eluting pair of enantiomers - HPLC method A | 422.4 | 422.2 | 12.2 | 4.8 |

### Example 1a

¹H-NMR (CD₃OD): 0.30 - 0.35 (2H), 0.49 - 0.60 (2H), 1.10 -1.15 (3H), 1.20 - 1.22 (1H), 4.60 - 4.68 (1H), 6.75-6.85 (2H), 7.00-7.10 (4H), 7.14-7.20 (1H)

### Example 1c

¹H-NMR (CD₃OD): 0.30 - 0.35 (2H), 0.58 - 0.62 (2H), 1.17 - 1.22 (1H), 1.35-1.43 (3H), 2.03 - 2.11 (2H), 4.80 - 4.84 (1H), 6.80 - 6.85 (2H), 7.04 - 7.06 (1H), 7.10 - 7.18 (3H), 7.29 - 7.32 (1H)

### Example 2

### 7-Hydroxy-6-{[3-(4-hydroxyphenyl)-1-methylpropyl]amino}-4,5,6,7-tetrahydroimidazo[4,5,1-jk][1]benzazepin-2(1H)-one

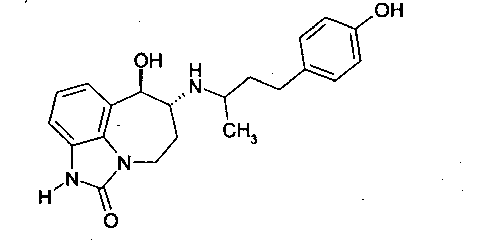

To a suspension of the compound of Preparation 1 (18.3 g, 71.5 mmol) in methanol (300 ml) was added triethylamine (24.9 ml, 179 mmol) and the compound of Preparation 106 (12.9 g, 78.6 mmol) in methanol. The reaction mixture was stirred at room temperature for 60 h, cooled to 0°C, and sodium borohydride (4.1 g, 107.2 mmol) was added portionwise. After stirring at room temperature for 18 h, the reaction mixture was quenched with water and silica (100 g) was added. The mixture was concentrated *in vacuo* and the product/silica mix was loaded onto a silica column (pre-wet with dichloromethane); the column was eluted with dichloromethane : methanol [1:0 to 4:6]. The appropriate fractions were combined and concentrated to give the compound of Example 2a (25.2 g) as a mixture of 4 diastereoisomers.

The compound of Example 2a (3.0 g, 8.2 mmol) was purified using a Biotage™ system with gradient elution, dichloromethane : methanol [1:0 to 7:3]. The appropriate fractions were combined and concentrated to give a solid. This solid was further purified using a Biotage system with gradient elution, dichloromethane : methanol [85:15 to 65:35]. The appropriate fractions were combined and concentrated to give the compound of Example 2b (300 mg) as a pair of enantiomers. HPLC Method B - retention time 11.74 min. Other appropriate fractions were combined and concentrated to give the compound of Example 2c (323 mg) as a pair of enantiomers. HPLC Method B - retention time 12.00 min.

The compound of Example 2c (148 mg, 0.4 mmol) was dissolved in ethanol : hexane (1:4) and the enantiomers were separated by automated preparative liquid chromatography (Gilson system, 250 x 21.4 mm Chiralcel OD-H, 10 µm column, 10 ml/min) using ethanol : hexane [1:4] as the mobile phase. The appropriate fractions were combined and concentrated to give the,compound of Example 2d (68 mg) as a single enantiomer. HPLC Method C - retention time 14.96 min.

| Example | Structure Comment | MH⁺ exper | MH⁺ expect | Bovine EC₅₀ nM | Porcine EC₅₀ nM |
|---|---|---|---|---|---|
| **2a** | Mixture of 4 diastereoisomers | N/A | | 13.4 | 6.1 |
| **2b** | First eluting pair of enantiomers - HPLC method A | 368.1 | 368.2 | 298 | 121 |
| **2c** | Second eluting pair of enantiomers - HPLC method A | 368.2 | 368.2 | 13.1 | 2.9 |
| **2d** | Single enantiomer | 368.1 | 368.2 | 3.6 | 1.9 |

### Example 2a

¹H-NMR (*d*₆-DMSO): 1.00 - 1.18 (3H), 4.60 - 4.65 (1H), 6.60 - 6.66 (2H), 6.84 - 6.89 (1H), 6.90 - 7.00 (3H), 7.00 - 7.11 (1H)

### Example 2b

¹H-NMR (CD₃OD): 1.21 - 1.28 (3H), 4.72 - 4.77 (1H), 6.62 - 6.68 (2H), 6.96 - 7.04 (3H), 7.08 - 7.10 (1H), 7.11 - 7.13 (1H)

### Example 2c

¹H-NMR (d₆-DMSO) 0.90 - 1.00 (3H), 4.50 - 4.55 (1H), 6.79 - 6.85 (2H), 6.80 - 6.87 (1H), 6.90 - 6.96 (3H), 7.00 - 7.05 (1H)

### Example 2d

¹H-NMR (CD₃OD): 1.10 - 1.13 (3H), 1.74 - 1.82 (2H), 4.63 - 4.66 (1H), 6.62 - 6.66 (2H), 6.97 - 7.09 (4H), 7.20-7.22(1H)

### Example 3

### 6-{[3-(2,3-Dihydro-1-benzofuran-5-yl)-1-methylpropyl]amino}-7-hydroxy-4,5,6,7-tetrahydro-imidazo[4,5,1-jk][1]benzazepin-2(1H)-one

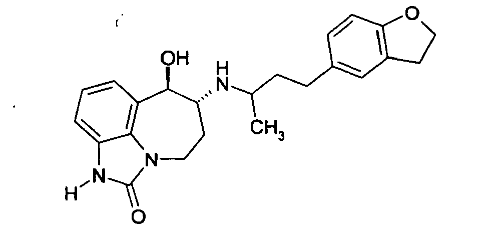

A mixture of the compound of Preparation 1 (117 mg, 0.5 mmol), triethylamine (100 µl, 0.7 mmol) and the compound of Preparation 170 (190 mg, 1.0 mmol) in methanol (2 ml) was heated at 80°C in a microwave oven (300W) for 40 min. The reaction mixture was stirred overnight at room temperature, before addition of sodium borohydride (120 mg, 3.2 mmol). After stirring at room temperature for 18 h, the mixture was diluted with methanol (8 ml) and Amberlyst® 15 ion-exchange resin (4 g, prepared according to (J. Org. Chem. 1998, 63, 3471-3473) was added. The mixture was shaken overnight and the solution was filtered off. The resin was washed with methanol (3 x 20 ml) and treated with ammonia in methanol (2N, 15 ml). After shaking for 2 h, the solution was filtered off and the resin was washed with ammonia in methanol (2N, 2 x 15 ml). The combined methanol/ammonia washings were concentrated *in vacuo* and the residue was re-dissolved in methanol (5 ml). This solution was filtered and concentrated *in vacuo.* The residue was dissolved in acetonitrile : water (1:1, 1.4 ml) and purified by automated preparative liquid chromatography (Gilson system, 150 mm x 19 mm XTERRA C18 5µm column), 20 ml/min, using an acetonitrile : 0.1% aqueous ammonia (1 :9) : acetonitrile : 0.1% aqueous ammonia (9:1) gradient [1:0 to 0:1 from 0 - 20 min; 0:1 from 20 - 25 min]. The appropriate fractions were combined and concentrated to give the compound of Example 3a (74 mg) as a mixture of 4 diastereoisomers.

### Alternative synthesis

To a suspension of the compound of Preparation 1 (0.95 g, 3.7 mmol) in methanol (25 ml) was added the compound of Preparation 170 (776 mg, 4.1 mmol) in methanol (25 ml), followed by triethylamine (0.16 1ml, 1.1 mmol). The reaction mixture was stirred at room temperature for 1 h and sodium cyanoborohydride (0.58 g, 9.27 mmol) was added portionwise. After stirring at room temperature for 60 h, the reaction mixture was quenched with water (1 ml) and concentrated *in vacuo.* The residue was purified by automated flash chromatography (Biotage™ 40M cartridge, wet with dichloromethane) with gradient elution, dichloromethane : 2.5% ammonia in methanol [100:0 to 84:16]. The appropriate fractions were combined and concentrated to give the compound of Example 3b (578 mg) as a pair of enantiomers. HPLC Method A - retention time 13.97 min. Other appropriate fractions were combined and concentrated to give the compound of Example 3c (563 mg) as a pair of enantiomers. HPLC Method A - retention time 14.25 min.

| Example | Structure Comment | MH⁺ exper | MH⁺ expect | Bovine EC₅₀ nM | Porcine EC₅₀ nM |
|---|---|---|---|---|---|
| **3a** | Mixture of 4 diastereoisomers | 394.5 | 394.2 | 34.3 | 8.1 |
| **3b** | First eluting pair of enantiomers - HPLC method A | N/A | | 456 | 108 |
| **3c** | Second eluting pair of enantiomers - HPLC method A | N/A | | 27.1 | 5.6 |

### Example 3a

¹H-NMR (CD₃OD): 1.08 - 1.18 (3H), 3.16 - 3.21 (2H), 4.42 - 4.48 (2H), 4.61 - 4.64 (1H), 6.55 - 6.60 (1H), 6.80 - 6.84 (1H), 6.99 - 7.05 (3H), 7.18 - 7.20 (1H)

### Example 3b

¹H-NMR (CD₃OD): 1.14 - 1.19 (3H), 3.75 - 3.84 (1H), 3.93 - 4.00 (1H), 4.44 - 4.51 (2H), 4.63 - 4.66 (1H), 6.54 - 6.58 (1H), 6.80 - 6.85 (1H), 6.98 - 7.08 (3H), 7.15 - 7.19 (1H)

### Example 3c

¹H-NMR (CD₃OD): 1.10.- 1.15 (3H), 3.81 - 4.00 (2H), 4.41 - 4.50 (2H), 4.63 - 4.69 (1H), 6.56 - 6.60 (1H), 6.83 - 6.90 (1H), 7.00 - 7.10 (3H), 7.19 - 7.21 (1H)

### Example 4

### 7-Hydroxy-6-{[3-(2-hydroxyphenyl)-1,3-dimethyl)butyl]amino)-4,5,6,7-tetrahydroimidazo[4,5,1-jk][1]benzazepin-2(1H)-one

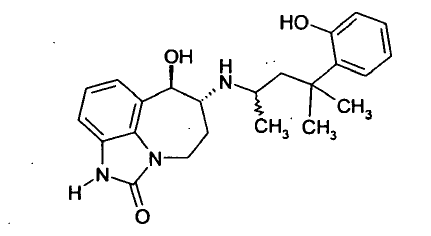

To a solution of the compound of Preparation 1 (144 mg, 0.6 mmol) and the compound of Preparation 13 (130 mg, 0.7 mmol) in methanol (10 ml) was added triethylamine (24 µI, 0.2 mmol). The mixture was .stirred at room temperature for 1 h before addition of sodium cyanoborohydride (53 mg, 0.9 mmol). After stirring at room temperature for 60 h, the reaction mixture was heated at 60°C for 48 h and then quenched with water and concentrated *in vacuo.* The residue was purified by automated flash chromatography (Biotage™ 25+M cartridge) with gradient elution, dichloromethane : 2.5% ammonia in methanol [96:4 to 80:20]. The appropriate fractions were combined and concentrated and the residue was dissolved in acetonitrile : water (9:1, 1 ml) and purified by automated preparative liquid chromatography (Gilson system, 150 mm x 21.4 mm Gemini C18 5µm column), 20 ml/min using an acetonitrile : 0.1% aqueous ammonia (5:95): acetonitrile : 0.1% aqueous ammonia (95:5) gradient [1:0 to 0:1 from 0 - 20 min; 0:1 from 20 - 25 min]. The appropriate fractions were combined and concentrated to give the compound of Example 4 (3 mg) as a pair of enantiomers.

| Example | Structure Comment | MH⁺ exper | MH⁺ expect | Bovine EC₅₀ nM | Porcine EC₅₀ nM |
|---|---|---|---|---|---|
| **4** | Second eluting pair of enantiomers - HPLC method A | 396.5 | 396.2 | 1.0 | 2.2 |

### Example 4

¹H-NMR (CD₃OD): 0.90 - 0.97 (3H), 1.22 - 1.28 (6H), 4.47 - 4.50 (1H), 6.19 - 6.21 (1H), 6.50 - 6.53 (1H), 6.67 - 6.71 (1H), 6.99 - 7.02 (4H)

### Example 5

### 6-{[3-{3-Chloro-2-hydroxyphenyl}-1-methylpropyl]amino}-7-hydroxy-4,5,6,7-tetrahydro-imidazo[4,5,1-jk][1]benzazepin-2(1H)-one

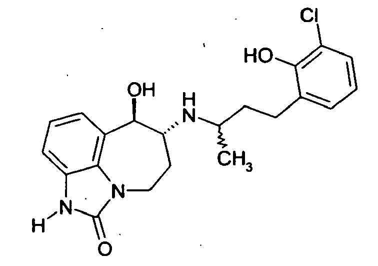

To a solution of the compound of Preparation 1 (591 mg, 2.3 mmol) in methanol (10 ml) was added the compound of Preparation 16 (500 mg, 2.5 mmol) in methanol (10 ml), followed by triethylamine (97 µl, 0.7 mmol). The mixture was stirred at room temperature for 1 h before addition of sodium cyanoborohydride (363 mg, 5.8 mmol). After stirring at room temperature for 5 days, the reaction mixture was quenched with water (2 ml), before first the addition of citric acid (1 g) and then sodium hydrogen carbonate (3 g). The mixture was concentrated *in vacuo* and the residue was mixed with silica, dissolved in methanol (50 ml) and re-concentrated. The residue was purified by automated flash chromatography (Biotage™ 40M cartridge, conditioned with dichloromethane : 2.5% ammonia in methanol with gradient elution, dichloromethane : 2.5% ammonia in methanol [96:4 to 91:9]. The appropriate fractions were combined and concentrated to give the compound of Example 5a (166 mg) as a pair of enantiomers. HPLC Method A - retention time 13.43 min. Other appropriate fractions were combined and concentrated to give the compound of Example 5b (50 mg) as a pair of enantiomers. HPLC Method A - retention time 14.36 min.

A portion of the compound of Example 5b (150 mg, 0.4 mmol) was dissolved in ethanol : methanol (1:1, 4 ml) and heated at 120°C in a microwave oven (CEM, 300W) for 2 min to aid solubility. The enantiomers were separated by automated preparative liquid chromatography (Gilson system, 500 x 50 mm ID Chiralpak AD-H, 20 µm column, 50 ml/min) using methanol : ethanol : hexane [5:15:80] with 0.1% v/v triethylamine as the mobile phase. The appropriate fractions were combined and concentrated to give the compound of Example 5c (100 mg) as a single enantiomer. HPLC Method D - retention time 17.97 min.

| Example | Structure Comment | MH⁺ exper | MH⁺ expect | Bovine EC₅₀ nM | Porcine EC₅₀ nM |
|---|---|---|---|---|---|
| **5a** | First eluting pair of enantiomers - HPLC method A | 402.2 | 402.2 | 307 | 473 |
| **5b** | Second eluting pair of enantiomers - HPLC method A | 402.2 | 402.2 | 1.6 | 2.9 |
| **5c** | Single enantiomer | N/A | | 2.7 | 6.0 |

### Example 5a

¹H-NMR (CD₃OD): 1.14 - 1.18 (3H), 1.71 - 1.86 (2H), 4.66 - 4.69 (1H), 6.64 - 6.69 (1H), 6.93 - 7.05 (3H), 7.07- 7.10 (1H), 7.14 - 7.18 (1H)

### Example 5b

¹H-NMR (CD₃OD): 1.07 - 1.10 (3H), 1.75 - 1.85 (2H), 4.74 - 4.77 (1H), 6.65 - 6.70 (1H), 6.96 - 7.10 (4H), 7.23 - 7.27 (1H)

### Example 5c

### (6R,7R)-6{[3-(3-Chloro-2-hydroxyphenyl)-(1R)-1-methylpropyl]amino}-7-hydroxy-4,5,6,7-tetrahydroimidazo[4,5,1-jk][1]benzazepin-2(1H)-one

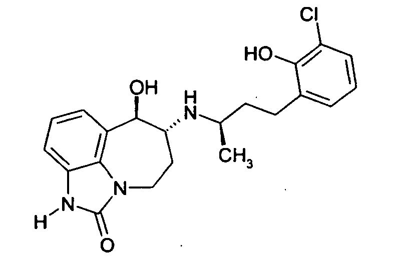

¹H-NMR (CD₃OD): 1.10 - 1.15 (3H), 4.77 - 4.81 (1H), 6.68 - 6.73 (1H), 6.98 - 7.03 (2H), 7.06 - 7.13 (2H), 7.26- 7.30 (1H)

### Example 6

### 7-Hydroxy-6-{[3-(2-hydroxyphenyl)-1-methylpropyl]amino}-4,5,6,7-tetrahydroimidazol[4,5,1-jk][1]benzazepin-2(1H)-one

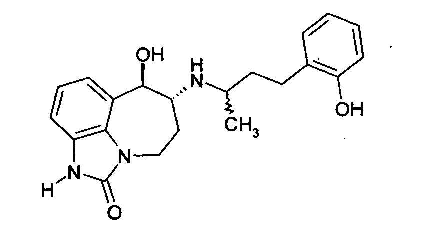

To a solution of the compound of Preparation 100 (7.0 g, 43.2 mmol) in methanol (200 ml) was added the compound of Preparation 1 (10.0 g, 39.2 mmol), followed by potassium hydroxide (0.7 g, 43.2 mmol). After stirring for 20 min, sodium cyanoborohydride (6.2 g, 98.1 mmol) was added and the reaction mixture was heated at 60°C for 60 h. The mixture was quenched with water (10 ml) and concentrated *in vacuo.* The residue was azeotroped with methanol, pre-absorbed onto silica (60 g) and purified by column chromatography (silica, 280 g) with gradient elution, 2% ammonia in methanol : dichloromethane [0:1 to 4:96]. The appropriate fractions were combined and concentrated to give the compound of Example 6a (2.0 g), as a pair of enantiomers. HPLC Method A - retention time 13.38 min.

To a solution of the compound of Example 6a (5.0 g, 13.7 mmol) in methanol (30 ml) was added dropwise hydrogen chloride in diethyl ether (1M, 13.7 ml). After stirring for 2 h, diethyl ether (200 ml) was added and the precipitate was collected by filtration, washing through with methanol : diethyl ether (15:85, 100 ml). The solid was dissolved in isopropyl alcohol (22 ml) and water (1.3 ml) at 98°C, then cooled to 0°C and stirred for 45 min. The resulting solid was collected by filtration, washed with cold isopropyl alcohol and diethyl ether and dried in a vacuum oven to give the hydrochloride salt, the compound of Example 6b (1.21 g).

The compound of Example 6a (1.1 g, 3.1 mmol) was dissolved in ethanol : methanol (3:1,20 ml) and heated to aid solubility. The enantiomers were separated by automated preparative liquid chromatography (Gilson system, 500 x 50 mm ID Chiralcel OD, 20 µm column, 50 ml/min) using methanol : ethanol : hexane [5:5:90] with 0.1% v/v triethylamine as the mobile phase. The appropriate fractions were combined and concentrated to give the compound of Example 6c (432 mg) as a single enantiomer. HPLC Method E - retention time 8.46 min. Other appropriate fractions were combined and concentrated to give the compound of Example 6d (502 mg) as a single enantiomer. HPLC Method E - retention time 9.88 min.

To a solution of the compound of Example 6c (378 mg, 1.0 mmol) in methanol (6 ml), at 0°C, was added dropwise hydrogen chloride in diethyl ether (1M, 1.2 ml). After stirring for 1.5 h, diethyl ether (34 ml) was added and the precipitate was collected by filtration. The resulting solid was washed with diethyl ether (2 x 40 ml) and dried in a vacuum oven at 50°C to give the hydrochloride salt, the compound of Example 6e (394 mg).

To a solution of the compound of Example 6d (419 mg, 1.1 mmol) in methanol (6 ml), at 0°C, was added dropwise hydrogen chloride in diethyl ether (1 M, 1.3 ml). After stirring for 1.5 h, diethyl ether (34 ml) was added and the precipitate was collected by filtration. The resulting solid was washed with diethyl ether (2 x 40 ml) and dried in a vacuum oven at 50°C to give the hydrochloride salt, the compound of Example 6f (418 mg).

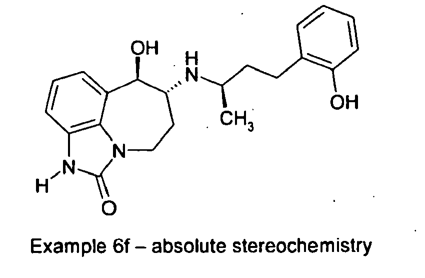

### Alternative synthesis

To a solution of the compound of Preparation 1 (5.0 g, 19.6 mmol) in methanol (100 ml), under nitrogen, was added the compound of Preparation 41 (5.5 g, 33.5 mmol), followed by triethylamine (0.8 ml, 5.9 mmol). After stirring for 20 min, sodium cyanoborohydride (1.8 g, 29.3 mmol) was added and the reaction mixture was heated at 50°C for 40 h. The mixture was concentrated *in vacuo* and the residue was dissolved in dichloromethane : 2% ammonia in methanol (4:1, 50 ml) and filtered through a silica plug. The filtrate was concentrated *in vacuo* and the residue was purified by automated flash chromatography (Biotage™ 65i cartridge, conditioned with dichloromethane : 2% ammonia in methanol with gradient elution, dichloromethane : 2% ammonia in methanol [94:6 to 87:13]. The appropriate fractions were combined and concentrated to give the compound of Example 6a (2.14 g), as a pair of enantiomers.

| Example | Structure Comment | MH⁺ exper | MH⁺ expect | Bovine EC₅₀ nM | Porcine EC₅₀ nM |
|---|---|---|---|---|---|
| **6a** | Second eluting pair of enantiomers, free base - HPLC method A | 368.1 | 368.2 | 1.5 | 1.5 |
| **6b** | Second eluting pair of enantiomers, HCI salt-HPLC method A | 368.3 | 368.2 | 2.0 | 1.4 |
| **6d** | Single enantiomer, HCl salt | 368.0 | 368.2 | 315 | 410 |
| **6f** | Single enantiomer, HCl salt | 368.1 | 368.2 | 1.0 | 1.9 |

### Example 6a

¹H-NMR (CD₃OD): 1.08 - 1.12 (3H), 4.63 - 4.66 (1H), 6.65 - 6.70 (2H), 6.92 - 7.00 (3H), 7.02 - 7.06 (1H), 7.19-7.22 (1H)

### Example 6b

¹-NMR (CD₃OD): 1.38 - 1.42 (3H), 4.87 - 4.91 (1H), 6.72 - 6.77 (2H), 6.99 - 7.04 (2H), 7.07 - 7.12 (2H), 7.26 - 7.30 (1H)

### Example 6d

¹H-NMR (CD₃OD): 1.38 - 1.44 (3H), 2.01 - 2.18 (2H), 4.87 - 4.91 (1H), 6.72 - 6.77 (2H), 6.98 - 7.04 (2H), 7.06 - 7.11 (2H), 7.25 - 7.29 (1H)

### Example 6f

¹H-NMR (CD₃OD): 1.38 - 1.41 (3H), 2.01 - 2.17 (2H), 4.88 - 4.92 (1H), 6.74 - 6.78 (2H), 6.99 - 7.04 (2H), 7.06 - 7.10 (2H), 7.25 - 7.29 (1H)

### Example 7

### 6-{[3-(5-Fluoro-2-hydroxypheny))-1-methylpropyl]amino}-7-hydroxy-4,5,6,7-tetrahydro-imidazo[4,5,1-jk][1]benzazepin-2(1H)-one

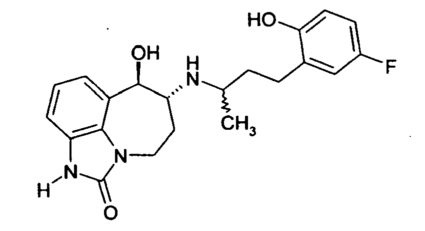

To a solution of the compound of Preparation 1 (1.7 g, 6.6 mmol) in methanol (34 ml) was added the compound of Preparation 42 (1.2 g, 6.6 mmol), followed by triethylamine (0.3 ml, 2.0 mmol). After stirring at 50°C for 30 min, sodium cyanoborohydride (662 mg, 10.5 mmol) was added and the reaction mixture was heated at 50°C for 18 h. The mixture was concentrated *in vacuo* and the residue was dissolved in methanol : dichloromethane (1:4) and purified by automated flash chromatography (Biotage™ 65i cartridge, conditioned with dichloromethane : 2% ammonia in methanol with gradient elution, dichloromethane : 2% ammonia in methanol [96:4 to 85:15]. The appropriate fractions were combined and concentrated to give the compound of Example 7a (900 mg) as a pair of enantiomers. Retention time 13.23 min. Other appropriate fractions were combined and concentrated to give the compound of Example 7b (1.07 g) as a pair of enantiomers. HPLC Method A - retention time 13.89 min.

| Example | Structure Comment | MH⁺ exper | MH⁺ expect | Bovine EC₅₀ nM | Porcine EC₅₀ nM |
|---|---|---|---|---|---|
| **7a** | First eluting pair of enantiomers - HPLC method A | 386.0 | 386.2 | 77.7 | 104 |
| **7b** | Second eluting pair of enantiomers - HPLC method A | 386.0 | 386.2 | 0.3 | 0.5 |

### Example 7a

¹H-NMR (CD₃OD): 1.21 - 1.26 (3H), 2.52 - 2.64 (2H), 4.67 - 4.70 (1H), 6.60 - 6.71 (2H), 6.79 - 6.82 (1H), 6.98 - 7.06 (2H), 7.18 - 7.21 (1H)

### Example 7b

¹H-NMR (CD₃OD): 1.09 - 1.13 (3H), 1.70 - 1.85 (2H), 4.62 - 4.66 (1H), 6.60 - 6.66 (2H), 6.77 - 6.80 (1H), 6.95 - 7.05 (2H), 7.18 - 7.21 (1H)

### Example 8

### 7-Hydroxy-6-{[3-(2-hydroxy-3-methoxyphenyl)-1-methylpropyl]amino}-4,5,6,7-tetrahydro-imidazo[4,5,1-jk][1]benzazepin-2(1H)-one

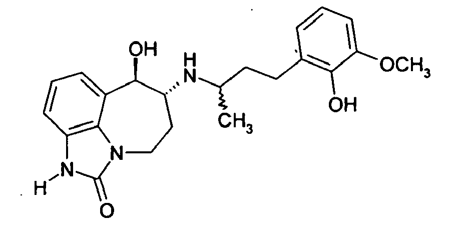

To a solution of the compound of Preparation 1 (0.6 g, 2.4 mmol) in methanol (10 ml) was added the compound of Preparation 20 (500 mg, 2.6 mmol) in methanol (5 ml), followed by triethylamine (99 gl, 0.7 mmol). After stirring for 1 h, sodium cyanoborohydride (372 mg, 5.2 mmol) was added and the reaction mixture was stirred at room temperature for 18 h. The reaction mixture was quenched with water (1 ml), stirred for 60 min and concentrated *in vacuo.* The residue was dissolved in acetonitrile : water (9:1, 4 ml) and purified by automated preparative liquid chromatography (Gilson system, 250 x 50 mm Gemini C18(2) column), 120 ml/min, using an acetonitrile : 0.1% aqueous ammonia (5:95): acetonitrile : 0.1% aqueous ammonia (95:5) gradient [100:0 to 75:25 from 0 to 32 min; 75:25 to 0:100 from 32 to 33 min; 0:100 from 33 to 36 min]. The appropriate fractions were combined and concentrated to give the compound of Example 8a (118 mg) as a pair of enantiomers. HPLC Method A - retention time 12.99 min. Other appropriate fractions were combined and concentrated to give the compound of Example 8b (150 mg) as a pair of enantiomers HPLC Method A - retention time 13.46 min.

| Example | Structure Comment | MH⁺ exper | MH⁺ expect | Bovine EC₅₀ nM | Porcine EC₅₀ nM |
|---|---|---|---|---|---|
| **8a** | First eluting pair of enantiomers - HPLC method A | 398.5 | 398.2 | 200 | 172 |
| **8b** | Second eluting pair of enantiomers - HPLC method A | 398.5 | 398.2 | 0.7 | 1.1 |

### Example 8a

¹H-NMR (CD₃OD 1.12 - 1.16 (3H), 3.79 - 3.80 (3H), 4.58 - 4.61 (1H), 6.58 - 6.66 (2H), 6.70 - 6.73 (1H), 6.95-7.05(2H),7.16-7.19(1H)

### Example 8b

¹H-NMR (CD₃OD): 1.09 - 1.12 (3H), 3.79 - 3.80 (3H), 4.63 - 4.66 (1H), 6.62 - 6.68 (2H), 6.70 - 6.73 (1H), 6.98-7.06 (2H), 7.19-7.21 (1H)

### Example 9

### 7-Hydroxy-6-[(1-methyl-3-phenylpropyl)amino]-4,5,6,7-tetrahydroimidazo[4,5,1-jk][1]benzazepin-2(1H)-one

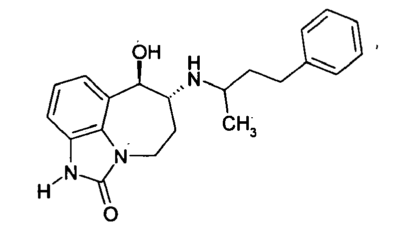

A mixture of the compound of Preparation 1 (117 mg, 0.5 mmol), triethylamine (100 µl, 0.7 mmol) and the compound of Preparation 112 (135 mg, 1.0 mmol) in methanol (2 ml) was heated at 80°C in a microwave oven (300W) for 40 min. The reaction mixture was stirred overnight at room temperature, before addition of sodium borohydride (120 mg, 3.2 mmol). After stirring at room temperature for 18 h, the mixture was diluted with methanol (8 ml) and AmberIyst® 15 ion-exchange resin (4 g, prepared according to (J. Org. Chem. 1998, 63, 3471-3473) was added. The mixture was shaken overnight and the solution was filtered off. The resin was washed with methanol (3 x 20 ml) and treated with ammonia in methanol (2N, 15 ml). After shaking for 2 h, the solution was filtered off and the resin was washed with ammonia in methanol (2N, 2 x 15 ml). The combined methanol/ammonia washings were concentrated *in vacuo* and the residue was re-dissolved in methanol (5 ml). This solution was filtered and concentrated *in vacuo.* The residue was dissolved in acetonitrile : water (1:1, 1.2 ml) and purified by automated ' preparative liquid chromatography (Gilson system, 150 mm x 19 mm XTERRA C18 5µm column), 20 ml/min, using an acetonitrile : 0.1% aqueous ammonia (1 :9) : acetonitrile : 0.1% aqueous ammonia (9:1) gradient [1:0 to 0:1 from 0 - 20 min; 0:1 from 20 - 25 min]. The appropriate fractions were combined and concentrated to give the compound of Example 9a (97 mg) as a mixture of 4 diastereoisomers.

To a solution of the compound of Example 9a (350 mg, 1.0 mmol) in methanol (7 ml) was added dropwise hydrogen chloride in diethyl ether (1M, 1.0 ml). After stirring for 45 min, diethyl ether (30 ml) was added dropwise and the solution was allowed to stand overnight. To the mixture was added methanol:diethyl ether (1:4, 20 ml) and the precipitate was collected by filtration. The resulting solid was washed with methanol:diethyl ether (1:4,3 x 15 ml) and diethyl ether (3 x 15 ml) and dried in a vacuum oven at 50°C to give the hydrochloride salt, the compound of Example 9b (183 mg).

The compound of Example 9a (3.0 g. 8.6 mmol) was dissolved in dichloromethane : methanol (9:1, 12 ml) and purified by automated flash chromatography (Biotage™ 65i cartridge) with gradient elution, dichloromethane : 2% ammonia in methanol [94:6 to 90:10]. The appropriate fractions were combined and concentrated to give the compound of Example 9c (0.8 g), as a pair of enantiomers.

To a solution of the compound of Example 9c (2.5 g, 7.1 mmol) in methanol (38 ml), at 0°C, was added dropwise hydrogen chloride in diethyl ether (1M, 7.1 ml). After stirring for 45 min, diethyl ether (205 ml) was added slowly and the precipitate was collected by filtration. The resulting solid was washed with diethyl ether (3 x 200 ml) and dried in a vacuum oven to give the hydrochloride salt, the compound of Example 9d (2.6 g).

The compound of Example 9c (1.0 g, 2.9 mmol) was dissolved in ethanol (30 ml) and heated to aid solubility. The enantiomers were separated by automated preparative liquid chromatography (Gilson system, 500 x 50 mm ID Chiralcel OD-H, 20 µm column) using ethanol : hexane [15:85] as the mobile phase. The appropriate fractions were combined and concentrated to give the compound of Example 9e (527 mg) as a single enantiomer HPLC Method F - retention time 12.43 min.

| Example | Structure Comment | MH⁺ exper | MH⁺ expect | Bovine EC₅₀ nM | Porcine EC₅₀ nM |
|---|---|---|---|---|---|
| **9b** | Mixture of 4 diastereoisomers - HCI salt | 352.2 | 352.2 | 179 | 34.3 |
| **9d** | Second eluting pair of enantiomers - HPLC method A | N/A | | 5.9 | 2.3 |
| **9e** | Single enantiomer | 352.4 | 352.2 | 7.0 | 2.3 |

### Example 9b

¹H-NMR (d₆-DMSO): 1.00 - 1.05 (3H), 4.50 - 4.57 (1H), 6.80 - 6.95 (2H), 7.01 - 7.04 (1H), 7.10 - 7.15 (3H), 7.20 - 7.25 (2H)

### Example 9d

¹H-NMR (CD₃OD): 1.41 - 1.43 (3H), 2.69 - 2.85 (2H), 4.95 - 4.98 (1H), 7.02 - 7.04 (1H), 7.10 - 7.15 (1H), 7.19 - 7.21 (1H), 7.23 - 7.30 (5H)

### Example 9e

¹H-NMR (CD₃OD): 1.10 - 1.13 (3H), 4.59 - 4.61 (1H), 6.98 - 7.05 (2H), 7.08 - 7.21 (6H)

### Example 10

### 6-{[3-(3,5-Difluoro-2-hydroxyphenyl)-1-methylpropyl)amino}-7-hydroxy-4,5,6,7-tetrahydro-imidazo[4,5,1-jk][1]benzazepin-2(1H)-one

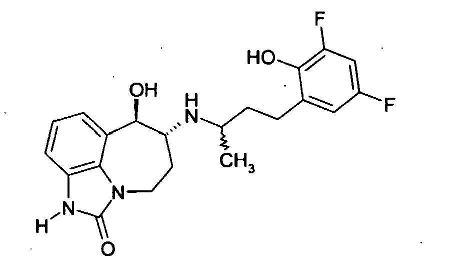

To a solution of the compound of Preparation 1 (2.0 g, 7.9 mmol) in methanol (40 ml) was added the compound of Preparation 48 (1.6 g, 7.9 mmol), followed by triethylamine (330 µl, 2.4 mmol). After stirring for 25 min, sodium cyanoborohydride (744 mg, 11.8 mmol) was added and the reaction mixture was stirred at 50°C for 80 h. The mixture was concentrated *in vacuo* to give the compound of Example 10a (4.8 g) as a mixture of 4 diastereoisomers

A solution of the compound of Example 10a (4.8 g, 11.8 mmol) in dichloromethane : 2.5% ammonia in methanol (2:1, 45 ml) was purified by automated flash chromatography (Biotage™ 65i cartridge, conditioned with dichloromethane : 2.5% ammonia in methanol [98:2]) with gradient elution, dichloromethane : 2.5% ammonia in methanol [97:3 to 88:12]. The appropriate fractions were combined and concentrated to give the compound of Example 10b (1.1 g), as a pair of enantiomers HPLC Method A - retention time 12.69 min.

| Example | Structure Comment | MH⁺ exper | MH⁺ expect | Bovine EC₅₀ nM | Porcine EC₅₀ nM |
|---|---|---|---|---|---|
| **10b** | Second eluting pair of enantiomers - HPLC method A | 404.4 | 404.2 | 0.8 | 1.7 |

### Example 10b

¹H-NMR (CD₃OD): 1.11 - 1.14 (3H), 1.74 - 1.80 (2H), 4.70 - 4.74 (1H), 6.63 - 6.71 (2H), 6.99 - 7.01 (1H), 7.04-7.08(1H), 7.22-7.24(1H).

### Example 11

### 6-{[3-(4,5-Difluoro-2-hydroxyphenyl)-1-methylpropyl)amino}-7-hydroxy-4,5,6,7-tetrahydro-imidazo[4,5,1-jk][1]benzazepin-2(1H)-one

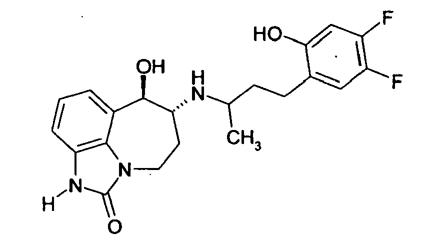

To a solution of the compound of Preparation 1 (2.7 g, 10.6 mmol) in methanol (25 ml) was added the compound of Preparation 18 (2.9 g, 11.7 mmol) in methanol (25 ml). To the suspension was added triethylamine (0.5 ml, 3.2 mmol) and the mixture was stirred at 45°C for 1 h, before addition of sodium cyanoborohydride (1.7 g, 26.6 mmol). The reaction mixture was heated at 45°C for 5 days and then quenched by addition of water (4 ml), citric acid (2.0 g, 10.4 mmol) and then sodium hydrogen carbonate (6.0 g). The mixture was concentrated *in vacuo* and to the residue was added methanol (100 ml) and silica. The slurry was concentrated *in vacuo* and the product/silica mix was eluted with dichloromethane : 2.5% ammonia in methanol [4:1]. The appropriate fractions were combined and concentrated and the residue was purified by automated flash chromatography (Biotage™ 40M cartridge, conditioned with dichloromethane : 2.5% ammonia in methanol [96:4]) with gradient elution, dichloromethane : 2.5% ammonia in methanol [96:4 to 91:9]. The appropriate fractions were combined and concentrated and the residue was dissolved in acetonitrile and dimethyl sulphoxide (1:1, 4 ml) and further purified by automated preparative liquid chromatography (Gilson system, 250 mm x 50 mm Gemini C18(2) column, 20 ml/min) using an acetonitrile : 0.1% aqueous ammonia (5:95): acetonitrile : 0.1% aqueous ammonia (95:5) gradient [9:1 to 7:3 (over 10 min) then 7:3 to 5:95 (over 16 min)]. The appropriate fractions were combined and concentrated to give the compound of Example 11a (44 mg) as a pair of enantiomers. HPLC Method A - retention time 13.6 min.

| Example | Structure Comment | MH⁺ exper | MH⁺ expect | Bovine EC₅₀ nM | Porcine EC₅₀ nM |
|---|---|---|---|---|---|
| **11a** | Second eluting pair of enantiomers - HPLC method A | 404.4 | 404.2 | 1.21 | 1.15 |

¹H-NMR (CD₃OD) 1.32 - 1.35 (3H), 1.98 - 2.05 (2H), 4.18 - 4.22 (1H), 6.59 - 6.64 (1H), 6.99 - 7.06 (2H), 7.09- 7.12 (1H), 7.29 - 7.32 (1H)

### The following Examples were prepared by similar methods to those described above for Examples 1 -11:

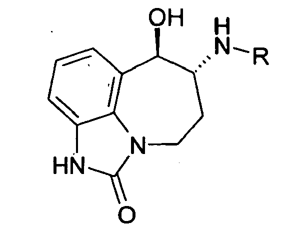

| Example | R | Structure Comment | MH⁺ exper | MH⁺ expect | Bovine EC₅₀ nM | Porcine EC₅₀ nM | From the compound of Preparation: |
|---|---|---|---|---|---|---|---|
| **12** | 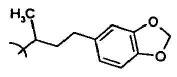 | Mixture of 4 diastereoisomers | 396.5 | 396.2 | 23.1 | 11.7 | 114 |
| **13a** | 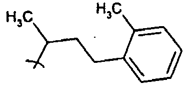 | Mixture of 4 diastereoisomers | 366.5 | 366.3 | 4.3 | 3.9 | 43 |
| **13b** | 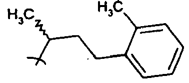 | Second eluting pair of enantiomers - HPLC method A | 366.5 | 366.2 | 2.0 | 0.9 | 43 |
| **14a** | 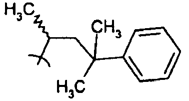 | Pair of enantiomers, methyl fixed but unknown | N/A | 428 | | 385 | 116 |
| **14b** | 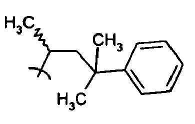 | Pair of enantiomers, methyl fixed but unknown - methyl opposite stereochemistry to 14a | 380.5 | 380.2 | 3.4 | 4.2 | 116 |
| **15** | 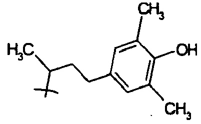 | Mixture of 4 diastereoisomers | 396.5 | 396.2 | 221 | 48.7 | 55 |
| **16** | 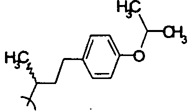 | Second eluting pair of enantiomers - HPLC method A | 410.6 | 410.2 | 17.3 | 5.5 | 104 |
| **17** | 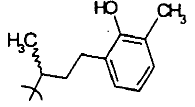 | Second eluting pair of enantiomers - HPLC method A | 382.1 | 382.2 | 3.3 | 3.0 | 23 |
| **18** | 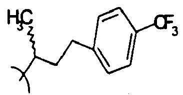 | Second eluting pair of enantiomers - HPLC method A | 420.4 | 420.2 | 29.6 | 10.7 | 44 |
| **19a** | 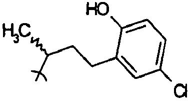 | Second eluting pair of enantiomers - HPLC method A | 402.2 | 402.2 | 0.6 | 0.9 | 168 |
| **19b** | 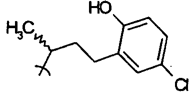 | First eluting pair of enantiomers - HPLC method A | 402.2 | 402.2 | 123 | 101 | 168 |
| **20** | 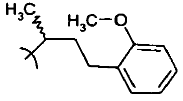 | Second eluting pair of enantiomers - HPLC method A | 382.3 | 382.2 | 7.5 | 35.8 | 24 |
| **21** | 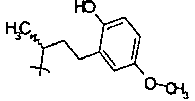 | First eluting pair of enantiomers - HPLC method A | N/A | | 3.2 | 3.1 | 25 |
| **22** | 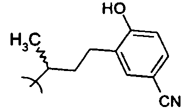 | Second eluting pair of enantiomers - HPLC method A | 393.1 | 393.2 | 1.2 | 2.3 | 22 |
| **23** | 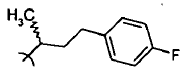 | Second eluting pair of enantiomers - HPLC method A | 370.0 | 370.2 | 15.8 | 10.5 | 26 |
| **24** | 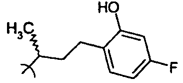 | Second eluting pair of enantiomers - HPLC method A | 386.4 | 386.2 | 2.7 | 4.2 | 27 |
| **25** | 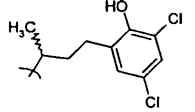 | Second eluting pair of enantiomers - HPLC method A | 436.1 | 436.3 | 2.2 | 2.7 | 28 |
| **26** | 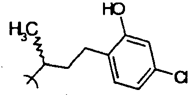 | Second eluting pair of enantiomers - HPLC method A | 402.4 | 402.2 | 3.1 | 1.7 | 29 |
| **27** | 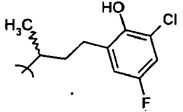 | Second eluting pair of enantiomers - HPLC method A | 420.4 | 420.1 | 0.6 | 2.2 | 30 |
| **28a** | 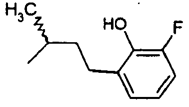 | Second eluting pair of enantiomers - HPLC method A | 386.4 | 386.2 | 1.5 | 1.8 | 31 |
| **28b** | 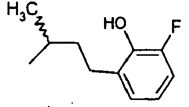 | First eluting pair of enantiomers - HPLC method A | 386.4 | 386.2 | 55 | 80 | 31 |
| **29** | 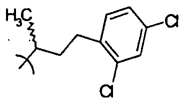 | Second eluting pair of enantiomers- HPLC method A | N/A | | 6.9 | 6.4 | 45 |
| **30** | 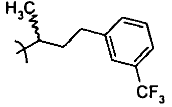 | Second eluting pair of enantiomers - HPLC method A | 420.5 | 420.2 | 37.1 | 5.5 | 46 |
| **31** | 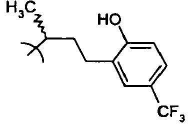 | Second eluting pair of enantiomers - HPLC method A | 436.4 | 436.2 | 27.2 | 8.1 | 32 |
| **32** | 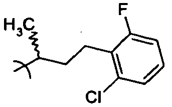 | Second eluting pair of enantiomers - HPLC method A | 404.4 | 404.2 | 8.6 | 2.9 | 47 |
| **33** | 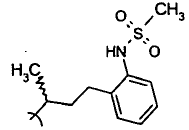 | Second eluting pair of enantiomers - HPLC method A | 445.4 | 445.2 | N/A | 11.9 | 62 |
| **34** | 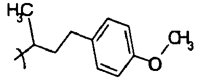 | Mixture of 4 diastereoisomers | 382.1 | 382.2 | 20.3 | 30.4 | 136 |
| **35** | 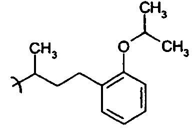 | Mixture of 4 diastereoisomers | 410.4 | 410.2 | 7.3 | 1.8 | 66 |
| **36** | 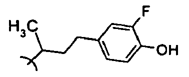 | Second eluting pair of enantiomers - HPLC method A | 386.4 | 386.2 | 1.4 | 2.2 | 56 |
| **37** | 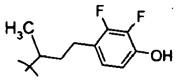 | Second eluting pair of enantiomers HPLC method A | 404.3 | 404.2 | 2.8 | 1.9 | 57 |
| **38** | 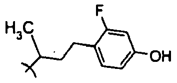 | Second eluting pair of enantiomers HPLC method A | N/A | N/A | 5.0 | 3.6 | 58 |
| **39** | 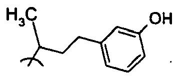 | Second eluting pair of enantiomers HPLC method A | 368.1 | 368.2 | 7.5 | 5.6 | 63 |

### Examples 12

### 6-{[3-(1,3-Benzodioxol-5-yl)-1-methylpropyl]amino}-7-hydroxy-4,5,6,7-tetrahydroimidazo[4,5,1-jk][1]benzazepin-2(1H)-one

¹H-NMR (CD₃OD): 1.08 - 1.20 (3H), 4.61 - 4.68 (1H), 5.82 - 5.90 (2H), 6.50 - 6.70 (3H), 6.98 - 7.10 (2H), 7.17-7.22 (1H)

### Example 13a

### 7-hydroxy-6-{[1-Methyl-3-(2-methylphenyl)propyl]amino)-4,5,6,7-tetrahydroimidazo[4,5,1-jk][1]benzazepin-2(1H)-one

¹H-NMR (CD₃OD): 1.17 - 1.22 (3H), 2.21 - 2.24 (3H), 3.80 - 4.00 (2 H), 4.65 - 4.70 (1H), 7.00 - 7.12 (6H), 7.18-7.21 (1H)

### Example 13b

### 7-hydroxy-6-{[1-Methyl-3-(2-methylphenyl)propyl]amino}-4,5,6,7-tetrahydroimidazo[4,5,1-jk][1]benzazepin-2(1H)-one

¹H-NMR (d₆-DMSO): 1.00 - 1.09 (3H), 2.22 - 2.23 (3H), 4.50 - 4.52 (1H), 6.81 - 6.83 (1H), 6.90 - 6.94 (1H), 7.01 -7.11 (5H)

### Example 14a

### 6-[(1,3-Dimethyl-3-phenylbutyl)amino]-7-hydroxy-4,5,6,7-tetrahydroimidazo]4,5,1-jk][1]benzazepin-2(1H)-one

¹H-NMR (CD₃OD): 0.99 - 1.05 (3H), 1.13 - 1.19 (6H), 4.69 - 4.73 (1H), 6.96 - 7.03 (5H), 7.04 - 7.08 (1H), 7.08-7.12 (2H)

### Example 14b

### 6-[(1,3-Dimethyl-3-phenylbutyl)amino]-7-hydroxy-4,5,6,7-tetrahydroimidazo[4,5,1-jk][1]benzazepin-2(1H)-one

¹H-NMR (CD₃OD): 0.89 - 0.95 (3H), 1.20 - 1.23 (3H), 1.35 - 1.40 (3H), 4.45 - 4.50 ( H), 6.80 - 6.85 (1H), 6.95 -7.10 (5H), 7.20-7.26 (2H)

### Example 15

### 7-Hydroxy-6-{[3-(4-hydroxy-3,5-dimethylphenyl)-1-methylpropyl]amino}-4,5,6,7-tetrahydro-imidazo[4,5,1-jk][1]benzazepin-2(1H)-one

¹H-NMR (CD₃OD): 1.09 - 1.15 (3H), 1.70 - 1.81 (2H), 2.15 - 2.17 (6H), 4.60 - 4.62 (1H), 6.70 - 6.73 (2H), 6.99 - 7.07 (2H), 7.18 - 7.20 (1H)

### Example 16

### 7-Hydroxy-6-{[3-(4-isopropoxyphenyl)-1-methylpropyl]amino}-4,5,6,7-tetrahydroimidazo[4,5,1-jk][1]benzazepin-2(1H)-one

¹H-NMR (CD₃OD): 1.10 - 1.18 (3H), 1.25 - 1.29 (6H), 4.62 - 4.68 (1H), 6.75 - 6.81 (2H), 6.99 - 7.10 0 (4H), 7.19-7.22 (1H)

### Example 17

### 7-Hydroxy-6-{[3-(2-hydroxy-3-methylphenyl)-1-methylpropyl]amino}-4,5,6,7-tetrahydro-imidazo[4,5,1-jk][1]benzazepin-2(1H)-one

¹H-NMR (CD₃OD): 1.39 - 1.43 (3H), 2.16 - 2.19 (3H), 4.86 - 4.90 (1H), 6.67 - 6.72 (1H), 6.90 - 6.96 (2H), 7.00 - 7.04 (1H), 7.06 - 7.12 (1H), 7.25 - 7.29 (1H)

### Example 18

### 7-Hydroxy-8-({1-methyl-3-[4-trifluoromethyl)phenyl]propyl}amino)-4,5,6,7-tetrahydro-imidazo[4,5,1-jk][1]benzazepin-2(1H)-one

¹H-NMR (CD₃OD): 1.15 - 1.17 (3H), 1.78 - 1.86 (2H), 4.62 - 4.64 (1H), 6.98 - 7.06 (2H), 7.18 - 7.21 (1H), 7.38 - 7.41 (2H), 7.55 - 7.59 (2H)

### Example 19a

### 6-{[3-(5-Chloro-2-hydroxyphenyl)-1-methylpropyl]amino}-7-hydroxy-4,5,6,7-tetrahydro-imidazo[4,5,1-jk][1]benzazepin-2(1H)-one

¹H-NMR (CD₃OD): 1.39 - 1.43 (3H), 2.61 - 2.80 (2H), 4.88 - 4.92 (1H), 6.70 - 6.73 (1H), 6.97 - 7.04 (2H), 7.09 - 7.12 (2H), 7.27 - 7.31 (1H)

### Example 19b

### 6-{[3-(5-Chloro-2-hydroxyphenyl)-1-methylpropyl]amino}-7-hydroxy-4,5,6,7-tetrahydro-imidazo[4,5,1-jk][1]benzazepin-2(1H)-one

¹H-NMR (CD₃OD): 1.15-1.19 (3H), 1.70 - 1.87 (2H), 4.63 - 4.67 (1H), 6.63 - 6.66 (1H), 6.91 - 7.05 (4H), 7.15-7.18 (1H)

### Example 20

### 7-Hydroxy-6-{[3-(2-methoxyphenyl)-1-methylpropyl]amino}-4,5,6,7-tetrahydroimidazo[4,5,1-jk][1]benzazepin-2(1H)-one

¹H-NMR (CD₃OD): 1.13 - 1.17 (3H), 3.73 - 3.75 (3H), 4.65 - 4.68 (1H), 6.78 - 6.87 (2H), 6.98 - 7.15 (4H), 7.17-7.21 (1H)

### Example 21

### 7-Hydroxy-6-{[3-(2-hydroxy-5-methoxyphenyl)-1-methylpropyl]amino}-4,5,6,7-tetrahydro-imidazo[4,5,1-jk][1]benzazepin-2(1H)-one

¹H-NMR (CD₃OD): 1.16 - 1.19 (3H), 3.66 - 3.69 (3H), 4.69 - 4.74 (1H), 6.58 - 6.61 (1H), 6.63 - 6.66 (2H), 6.99 - 7.09 (2H), 7.22 - 7.24 (1H)

### Example 22

### 4-Hydroxy-3-(3-{[7-hydroxy-2-oxo-1,2,4,5,6,7-hexahydroimidazo[4,5,1-jk][1]benzazepin-6-yl]amino}butyl)benzonitrile

¹H-NMR (CD₃OD): 1.15-1.19 (3H), 1.74 - 1.82 (2H), 4.71 - 4.75 (1H), 6.79 - 6.82 (1H), 6.99 - 7.09 (2H), 7.25 - 7.28 (1H), 7.35 - 7.39 (1H), 7.42 - 7.44 (1H)

### Example 23

### 6-{[3-(4-Fluorophenyl)-1-methylpropyl]amino}-7-hydroxy-4,5,6,7-tetrahydroimidazo[4,5,1-jk][1]benzazepin-2(1H)-one

¹H-NMR (CD₃OD): 1.08 - 1.13 (3H), 1.71 - 1.81 (2H), 4.60 - 4.64 (1H), 6.88 - 6.99 (3H), 7.00 - 7.06 (1H), 7.12-7.19 (3H)

### Example 24

### 6-{[3-(4-Fluoro-2-hydroxyphenyl)-1-methylpropyl]amino}-7-hydroxy-4,5,6,7-tetrahydro-imidazo[4,5,1-jk][1]benzazepin-2(1H)-one

¹H-NMR (CD₃OD): 1.09-1.12 (3H), 1.70 -1.80 (2H), 4.65 - 4.70 (1H), 6.40 - 6.48 (2H), 6.99 - 7.11 (3H), 7.22 - 7.25 (1H)

### Example 25

### 6-{[3-(3,5-Dichloro-2-hydroxyphenyl)-1-methylpropyl]amino}-7-hydroxy-4,5,6,7-tetrahydro-imidazo[4,5,1-jk][1]benzazepin-2(1H)-one

¹H-NMR (CD₃OD): 1.09 - 1.12 (3H), 1.80.1.88 (2H), 4.80 - 4.83 (1H), 7.00 - 7.03 (2H), 7.04 - 7.12 (2H), 7.27 - 7.29 (1H)

### Example 26

### 6-{[3-(4-Chloro-2-hydroxyphenyl)-1-methylpropyl]amino}-7-hydroxy-4,5,6,7-tetrahydro-imidazo[4,5,1jk][1]benzazepin-2(1H)-one

¹H-NMR (CD₃OD): 1.12 - 1.17 (3H), 1.70 - 1.80 (2H), 4.63 - 4.66 (1H), 6.61 - 6.63 (1H), 6.77 - 6.79 (1H), 6.99 - 7.10 (3H), 7.20 - 7.22 (1H)

### Example 27

### 6-{[3-(3-Chloro-5-fluoro-2-hydroxyphenyl)-1-methylpropyl]amino}-7-hydroxy-4,5,6,7-tetrahydro-imidazo[4,5,1-jk][1]benzazepin-2(1H)-one

¹H-NMR(CD₃OD): 1.09-1.12 (3H), 4.75 - 4.78 (1H), 6.80 - 6.82 (1H), 6.91 - 6.94 (1H), 7.00 - 7.03 (1H), 7.07 - 7.10 (1H), 7.25 - 7.30 (1H)

### Example 28a

### 6{[3-(3-Fluoro-2-hydroxyphenyl)-1-methylpropyl]amino}-7-hydroxy-4,5,6,7-tetrahydro-imidazo[4,5,1-jk][1]benzazepin-2(1H)-one

¹H-NMR (CD₃OD): 1.12 - 1.15 (3H), 1.74 - 1.81 (2H), 4.70 - 4.72 (1H), 6.63 - 6.66 (1H), 6.80 - 6.87 (2H), 6.99 - 7.10 (2H), 7.23 - 7.25 (1H)

### Example 28b

### 6-{[3-(3-Fluoro-2-hydroxyphenyl)-1-methylpropyl]amino}-7-hydroxy-4,5,6,7-tetrahydro-imidazo[4,5,1-jk]benzazepin-2(1H)-one

¹H-NMR (CD₃OD): 1.16 - 1.20 (3H), 1.70 1.80 (2H), 4.65 - 4.68 (1H), 6.62 - 6.66 (1H), 6.80 - 6.89 (2H), 6.99 - 7.08 (2H), 7.18 - 7.21 (1H)

### Example 29

### 6{[3-(2,4-Dichlorophenyl)-1-methylpropyl]amino}-7-hydroxy-4,5,6,7-tetrahydroimidazo[4,5,1-jk][1]benzazepin-2(1H)-one

¹H-NMR (CD₃OD): 1.12 - 1.15 (3H), 1.70 - 1.80 (2H), 4.61 - 4.63 (1H), 6.96 - 7.04 (2H), 7.16 - 7.20 (2H), 7.22 - 7.24 (1H), 7.36 - 7.38 (1H)

### Example 30

### 7-Hydroxy-6-({1-methyl-3-[3-(trifluoromethyl)phenyl]propyl}amino)-4,5,6,7-tetrahydro-imidazo[4,5,1-jk][1]benzazepin-2(1H)-one

¹H-NMR (CD₃OD): 1.14 - 1.17 (3H), 1.78 - 1.87 (2H), 4.62 - 4.64 (1H), 6.99 - 7.09 (2H), 7.20 - 7.22 (1H), 7.41 - 7.50 (3H), 7.51 - 7.52 (1H)

### Example 31

### 7-Hydroxy-6-({3-[2-hydroxy-5-(trifluoromethyl)phenyl]-1-methylpropyl}amino)-4,5,6,7-tetrahydro-imidazo[4,5,1-jk][1]benzazepin-2(1H)-one

¹H-NMR (CD₃OD): 1.38 - 1.41 (3H), 2.00 - 2.10 (2H), 4.19 - 4.23 (1H), 6.88 - 6.91 (1H), 7.01 - 7.04 (1H), 7.10 - 7.14 (1H), 7.30 - 7.35 (2H), 7.40 - 7.44 (1H)

### Example 32

### 6-{[3-(2-Chloro-6-fluorophenyl)-1-methylpropyl]amino}-7-hydroxy-4,5,6,7-tetrahydroimidazo(4,5,1-jk][1]benzazepin-2(1H)-one

¹H-NMR (d₆-DMSO): 1.00 - 1.06 (3H), 4.50 - 4.55 (1H), 6.81 - 6.85 (1H), 6.90 - 6.96 (1H), 7.01 - 7.05 (1H), 7.12 - 7.19 (1H), 7.20 - 7.26 (2H)

### Example 33

### N-[2-(3-{[7-Hydroxy-2-oxo-1,2,4,5,6,7-hexahydroimidazo[4,5,1-jk][1]benzazepin-6-yl]amino}butyl)phenyl]methanesulfonamide

¹H-NMR (CD₃OD): 1.10 - 1.13 (3H), 2.90 - 2.92 (3H), 4.90 - 4.92 (1H), 6.99 - 7.01 (1H), 7.06 - 7.08 (1H), 7.16 - 7.21 (2H), 7.25 - 7.34 (2H), 7.39 - 7.41 (1H)

### Example 34

### 7-Hydroxy-6-{[3-(4-methoxyphenyl)-1-methylpropyl]amino}-4,5,6,7-tetrahydroimidazo[4,5,1-jk][1]benzazepin-2(1H)-one

¹H-NMR (CD₃OD): 1.07 - 1.15 (3H), 3.69 - 3.72 (3H), 4.59 - 4.64 (1H), 6.71 - 6.77 (2H), 6.96-7.05 (4H), 7.12-7.17 (1H)

### Example 35

### 7-Hydroxy-6-{[3-(2-isopropoxyphenyl)-1-methylpropyl]amino}-4,5,6,7-tetrahydroimidazo[4,5,1-jk][1]benzazepin-2(1H)-one

¹H-NMR (CD₃OD): 1.10 - 1.14 (3H), 1.25 - 1.30 (6H), 4.50 - 4.52 (1H), 4.62 - 4.64 (1H), 6.75 - 6.79 (1H), 6.83 - 6.86 (1H), 7.00 - 7.11 (4H), 7.18 - 7.20 (1H)

### Example 36

### 6-{[3-(3-Fluoro-4-hydroxyphenyl)-1-methylpropyl]amino}-7-hydroxy-4,5,6,7-tetrahydro-imidazo[4,5,1-jk][1]benzazepin-2(1H)-one

¹H-NMR (CD₃OD): 1.15 - 1.18 (3H), 1.75 - 1.82 (2H), 4.63 - 4.65 (1H), 6.77 - 6.80 (2H), 6.85 - 6.87 (1H), 7.00 - 7.02 (1H), 7.05 - 7.07 (1H), 7.20 - 7.22 (1H)

### Example 37

### 6-{[3-(2,3-Difluoro-4-hydroxyphenyl-1-methylpropyl]amino}-7-hydroxy-4,5,6,7-tetrahydro-imidazo[4,5,1-jk][1]benzazepin-2(1H)-one

¹H-NMR (CD₃OD): 1.20 - 1.22 (3H), 1.80 - 1.86 (2H), 4.73 - 4.75 (1H), 6.60 - 6.64 (1H), 6.79 - 6.82 (1H), 7.00 - 7.02 (1H), 7.05 - 7.07 (1H), 7.21 - 7.23 (1H)

### Example 38

### 6{[3-(2-Fluoro-4-hydroxyphenyl)-1-methylpropyl]amino}-7-hydroxy-4,5,6,7-tetrahydro-imidazo[4,5,1-jk][1]benzazepin-2(1H)-one

¹H-NMR (CD₃OD): 1.14 - 1.16 (3H), 1.75 - 1.81 (2H), 4.63 - 4.65 (1H), 6.78 - 6.80 (2H), 6.86 - 6.88 (1H), 7.00 - 7.02 (1H), 7.03 - 7.05 (1H), 7.20 - 7.22 (1H)

### Example 39

### 7-Hydroxy-6-{[3-(3-hydroxyphenyl)-1-methylpropyl]amino}-4,5,6,7-tetrahydroimidazo[4,5,1-jk][1]benzazepin-2(1H)-one

¹H-NMR (CD₃OD): 1.10 - 1.13 (3H), 1.75 - 1.82 (2H), 4.62 - 4.64 (1H), 6.57 - 6.59 (1H), 6.71 - 6.74 (2H), 6.98 - 7.05 (3H), 7.19 - 7.22 (1H)

### Example 40

### 6-{[3-(2-Fluorophenyl)-1-methylprop-2-en-1-yl]amino}-7-hydroxy-4,5,6,7-tetrahydroimidazo[4,5,1-jk][1]benzazepin-2(1H)-one

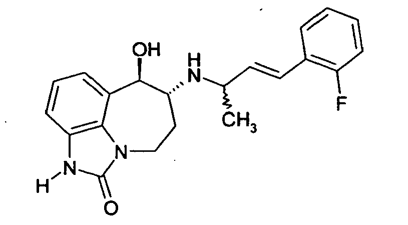

To a solution of the compound of Preparation 1 (535 mg, 2.1 mmol) in methanol (10 ml) was added the compound of Preparation 33 (378 mg, 2.3 mmol) in methanol (5 ml), followed by triethylamine (88 µl, 0.6 mmol). The reaction mixture was stirred at room temperature for 1 h, before addition of sodium cyanoborohydride (329 mg, 5.2 mmol). After stirring for 72 h, sodium borohydride (40 mg, 1.1 mmol) was added and the mixture was quenched by addition of water. The mixture was stirred for a further 1 h and then concentrated *in vacuo.* The residue was extracted with ethyl acetate (3 x 15 ml) and the combined extracts were dried (MgSO₄) and concentrated *in vacuo.* The residue was dissolved in dichloromethane and purified by automated flash chromatography (Biotage™ 25M cartridge conditioned with dichloromethane : 2.5% ammonia in methanol with gradient elution, dichloromethane : 2.5% ammonia in methanol [96:4 to 93:7]. The appropriate fractions were combined and concentrated and the residue was dissolved in acetonitrile : water (9:1, 4 ml) and purified by automated preparative liquid chromatography (Gilson system, 150 mm x 21.4 mm Gemini C18 5µm column), 20 ml/min, using an acetonitrile : 0.1% aqueous ammonia (5:95) : acetonitrile : 0.1% aqueous ammonia (95:5) gradient [30:70 from 0 to 32 min; 30:70 to 5:95 from 32 to 33 min; 5:95 from 33 to 36 min]. The appropriate fractions were combined and concentrated to give the compound of Example 40a (9 mg) as a pair of enantiomers HPLC Method A - 14.14min. Other appropriate fractions were combined and concentrated to give the compound of Example 40b (14 mg) as a pair of enantiomers. HPLC Method A - retention time 14.41min.

| Example | Structure Comment | MH⁺ exper | MH⁺ expect | Bovine EC₅₀ nM | Porcine **EC₅₀** nM |
|---|---|---|---|---|---|
| **40a** | First eluting pair of enantiomers - HPLC method A | 368.4 | 368.2 | 242 | 65.1 |
| **40b** | Second eluting pair of enantiomers - HPLC method A | 368.4 | 368.2 | 33.2 | 18 |

### Example 40a

¹H-NMR (CD₃OD): 1.21 - 1.24 (3H), 4.62 - 4.66 (1H), 6.10 - 6.16 (1H), 6.62 - 6.69 (1H), 6.95 - 7.10 (4H), 7.15 - 7.21 (2H), 7.45 - 7.50 (1H)

### Example 40b

¹H-NMR (CD₃OD): 1.19 - 1.23 (3H), 4.73 - 4.77 (1H), 6.20 - 6.28 (1H), 6.70 - 6.76 (1H), 6.96 - 7.10 (4H), 7.18 - 7.22 (2H), 7.45 - 7.50 (1H)

### Similarly prepared were:

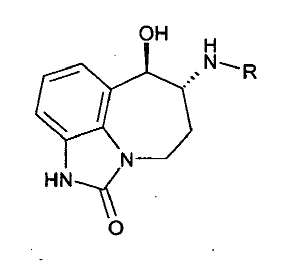

| Example | R | Structure Comment | MH⁺ exper | MH⁺ expect | Bovine EC₅₀ nM | Porcine EC₅₀ | From the compound of Preparation |
|---|---|---|---|---|---|---|---|
| **41** | 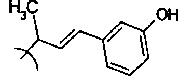 | Mixture of 4 diastereoisomers | 366.5 | 366.2 | 13.3 | 6.6 | 171 |
| **42** | 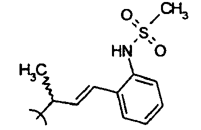 | Second eluting pair of enantiomers - HPLC method A | 443.0 | 443.2 | 258 | 104 | 65 |

### Example 41

### 7-Hydroxy-6-{[3-(3-hydroxyphenyl)-1-methylprop-2-en-1-yl]amino}-4,5,6,7-tetrahydroimidazo[4,5,1-jk][1]benzazepin-2(1H)-one

¹H-NMR (CD₃OD): 1.20 - 1.30 (3H), 4.65 - 4.76 (1H), 5.95 - 6.10 (1H), 6.40 - 6.50 (1H), 6.60 - 6.70 (1H), 6.80 - 6.90 (2H), 7.00 - 7.15 (3H), 7.20 - 7.26 (1H)

### Example 42

### N-{2-[3-{[7-Hydyroxy-2-oxo-1,2,4,5,6,7-hexahydroimidazo[4,5,1-jk][1]benzazepin-6-yl]amino}but-1-en-1-yl]phenyl}methanesulfonamide

¹H-NMR (CD₃OD): 1.24 - 1.28 (3H), 2.90 - 2.92 (3H), 4.66 - 4.68 (1H), 6.03 - 6.09 (1H), 6.97 - 7.05 (3H), 7.20 - 7.26 (3H), 7.30 - 7.32 (1H), 7.61 - 7.63 (1H)

### Example 43

### 7-Hydroxy-6-{[3-(3-hydroxyphenyl)-1-methylpropyl]amino}-4,5,6,7-tetrahydroimidazo[4,5,1-jk][1]benzazepin-2(1H)-one

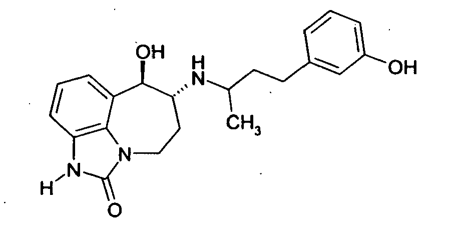

A mixture of the compound of Example 41 (15 mg, 40.2 µmol) and palladium (10% on carbon, 4 mg) in methanol (2 ml) was hydrogenated (60 psi) for 1 h. The reaction mixture was filtered through Celite® and the filtrate was concentrated *in vacuo* to give the compound of Example 43 (15 mg) as a mixture of 4 diastereoisomers.

| Example | Structure Comment | MH⁺ exper | MH⁺ expect | Bovine EC₅₀ nM | Porcine EC₅₀ nM |
|---|---|---|---|---|---|
| **43** | Mixture of 4 diastereoisomers | 368.3 | 368.2 | 7.2 | 5.6 |

### Example 43

¹H-NMR (CD₃OD): 1.10 - 1.20 (3H), 4.60 - 4.70. (1H), 6.58 - 6.70 (3H), 6.95 - 7.10 (3H), 7.15-7.22 (1H)

### Example 44

### 7-Hydroxy-6-({3-[3-(hydroxymethyl)phenyl]-1-methylpropyl}amino)-4,5,6,7-tetrahydro-imidazo[4,5,1-jk][1]benzazepin-2(1H)-one

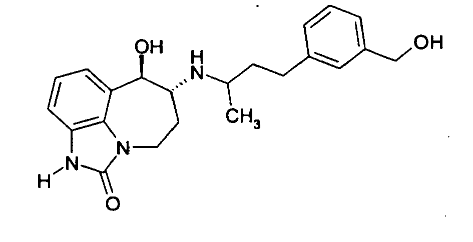

A mixture of the compound of Preparation 1 (100 mg, 0.4 mmol), triethylamine (0.1 ml, 0.6 mmol) and the compound of Preparation 34 (138 mg, 0.8 mmol) in methanol (3 ml) was stirred at room temperature for 18 h. Sodium borohydride (44 mg, 1.2 mmol) was then added and the reaction mixture was stirred at room temperature for 1 h. The mixture was diluted with methanol (8 ml) and Amberlyst® 15 ion-exchange resin (3.5 g, prepared according to J. Org. Chem. 1998, 63, 3471-3473) was added. The mixture was shaken overnight and the solution was filtered off. The resin was washed with methanol (100 ml) and treated with ammonia in methanol (2N, 5 ml). After shaking for 2 h, the solution was filtered off and the resin was washed with ammonia in methanol (2N, 2 x 5 ml). The combined methanol/ammonia washings were concentrated *in vacuo* and the residue was dissolved in acetonitrile : water (1:1, 1.5 ml) and purified by automated preparative liquid chromatography (Gilson system, 150 mm x 21.4 mm Gemini 5µm column, 20 ml/min) using a 0.1% aqueous ammonia : acetonitrile (9:1): 0.1% aqueous ammonia : acetonitrile gradient (1:9) [1:0 to 0:1 from 0 - 20 min; 0:1 from 20 - 25 min]. The appropriate fractions were combined and concentrated to give the unsaturated intermediate of the title compound (14 mg).
A mixture of the unsaturated intermediate of the title compound (14 mg, 0.04 mmol) and platinum (IV) oxide (1 mg) in water (0.5 ml) and isopropyl alcohol (0.5 ml) was shaken under hydrogen (60 psi) for 30 min. The reaction mixture was filtered through Arbocel®, washing through with isopropyl alcohol (0.5 ml), and the filtrate was concentrated *in vacuo* to give the compound of Example 44 (10 mg).

| Example | Structure Comment | MH⁺ exper | MH⁺ expect | Bovine EC₅₀ nM | Porcine EC₅₀ nM |
|---|---|---|---|---|---|
| **44** | Mixture of 4 diastereoisomers | 382.1 | 382.2 | 75.7 | 61.7 |

### Example 44

¹H-NMR (CD₃OD): 1.10 - 1.20 (3H), 4.50 - 4.60 (2H), 4.63 - 4.66 (1H), 6.90 - 7.10 (3H), 7.11 7.25 (3H)

### Example 45

### 6-{[3-(4-Aminophenyl)-1-methylpropyl]amino}-7-hydroxy-4,5,6,7-tetrahydroimidazo[4,5,1-jk][1]benzazepin-2(1H)-one

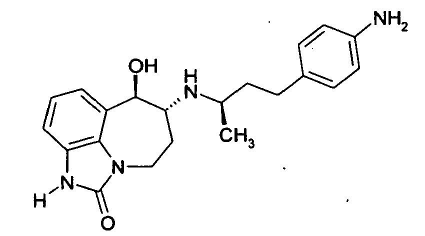

A solution of the compound of Preparation 67 (500 mg, 1.3 mmol) in methanol (5 ml) was flowed through an H-Cube hydrogenator (8 ml/min, 1 atm, 55°C) using a palladium catalyst (10% on carbon). The solution was concentrated *in vacuo* and the residue was dissolved in acetonitrile (2 ml) and purified by automated preparative liquid chromatography (Gilson system, 150 mm x 21 mm Gemini C18 5µm column, 25 ml/min) using a 1% aqueous ammonia : acetonitrile gradient [5:95 to 40:60 (from 0 to 6 min) to 98:2 (from 10 to 10.5 min)]. The appropriate fractions were combined and concentrated to give the the compound of Example 45 (4 mg) as a single enantiomer.

| Example | Structure Comment | MH⁺ exper | MH⁺ expect | Bovine EC₅₀ nM | Porcine EC₅₀ nM |
|---|---|---|---|---|---|
| **45** | Single enantiomer | 367.2 | 367.2 | 30.3 | 21 |

### Example 45

¹H-NMR (*d*₆-DMSO): 0.97 - 1.00 (3H), 2.61 - 2.65 (1H), 2.98 - 3.01 (1H), 4.77 - 4.79 (1H), 6.42 - 6.45 (2H), 6.79 - 6.82 (2H), 6.82 - 6.84 (1H), 6.90 - 6.92 (1H), 7.03 - 7.05 (1H)
HPLC Method A - retention time 11.80 min

### Similarly prepared was:

### Example 46

### 6-{[3-(3-Aminophenyl)-1-methylpropyl]amino}-7-hydroxy-4,5,6,7-tetrahydroimidazo[4,5,1-jk][1]benzazepin-2(1H)-one

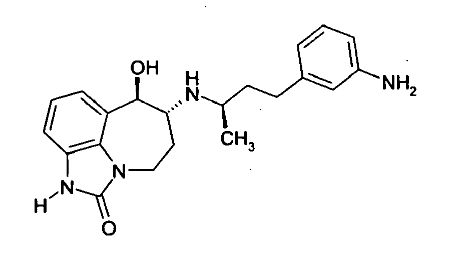

| Example | Structure Comment | MH⁺ exper | MH⁺ expect | Bovine EC₅₀ nM | Porcine EC₅₀ nM | From the compound of Preparation |
|---|---|---|---|---|---|---|
| **46** | Single enantiomer | 367.2 | 367.2 | 33.8 | 28.9 | 68 |

HPLC Method A - retention time 11.98 min

### Example 47

### 6-{[3-(3,5-Dibromo-2-hydroxyphenyl)-1-methylpropyl]amino}-7-hydroxy-4,5,6,7-tetrahydro-imidazo[4,5,1-jk][1]benzazepin-2(1H)-one

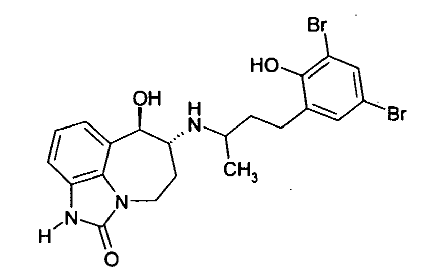

To a solution of the compound of Preparation 69 (2.1 g, 5.7 mmol) in N,N-dimethylformamide (10 ml) and dichloromethane (40 ml) was added dropwise the compound of Preparation 148 (3.6 g, 17.2 mmol) in N,N-dimethylformamide (20 ml). The reaction mixture was stirred for 1.5 h, before addition of dichloromethane (100 ml) and saturated aqueous sodium hydrogen carbonate solution (10 ml). After stirring for 10 min, the solution was adjusted to pH 7 by addition of solid citric acid and aqueous sodium thiosulphate solution (50 ml) was added. The solution was concentrated *in vacuo* and to the residue was added methanol (150 ml) and silica (50 g). The slurry was concentrated *in vacuo* and the silica/product mix was passed through a silica plug, eluting with dichloromethane : 2.5% ammonia in methanol [4:1]. The filtrate was concentrated *in vacuo* and the residue was triturated with dichloromethane (50 ml). To the residue was added methanol (50 ml) and silica (10 g) and the slurry was concentrated *in vacuo.* The product/silica mix was purified by automated flash chromatography (Biotage™, 65i silica cartridge) with gradient elution, dichloromethane : 2.5% ammonia in methanol [96:4 to 90:10]. The appropriate fractions were combined and concentrated to give the compound of Example 48 (300 mg) as a pair of enantiomers. HPLC Method A - retention time 12.8 min
¹H-NMR (d₆-DMSO): 0.99 - 1.02 (3H), 4.64 - 4.68 (1H), 6.85 - 6.87 (1H), 6.92 - 6.94 (1H), 7.15 - 7.17 (1H), 7.20 - 7.22 (1H), 7.42 - 7.43 (1H)

| Example | Structure Comment | MH⁺ exper | MH⁺ expect | Bovine EC₅₀ nM | Porcine EC₅₀ nM |
|---|---|---|---|---|---|
| **47** | Second eluting pair of enantiomers - HPLC method A | 523.9 | 524.0 | >200 | >300 |

### Example 48

### Hydrochloride salt of 7-hydroxy-6-{[3-(2-hydroxyphenyl)-1-methylpropyl]amino}-4,5,6,7-tetrahydroimidazo[4,5,1-jk][1]benzazepin-2(1H)-one

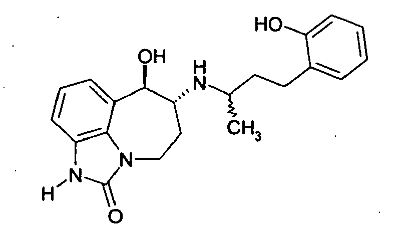

To a mixture of the compound of Preparation 9 (7.2 g, 28.2 mmol) and triethylamine (1.2 ml, 8.5 mmol) in methanol (40 ml) was added the compound of Preparation 41 (5.6 g, 33.8 mmol) followed by sodium cyanoborohydride (2.7 g, 42.3 mmol). The reaction mixture was heated at 50°C for 18 h, before addition of water (3 ml), citric acid (3.0 g) and sodium hydrogen carbonate (3.0 g). After stirring for 30 min, the solution was concentrated *in vacuo* and to the residue was added methanol (250 ml) and silica. The slurry was concentrated *in vacuo* and the silica/product mix was passed through a silica plug, eluting with dichloromethane : 2.5% ammonia in methanol [4:1]. The filtrate was concentrated *in vacuo* and purified by automated flash chromatography (Biotage™, 65M silica cartridge) with gradient elution, dichloromethane : 2.5% ammonia in methanol [95:5 to 93:7]. The appropriate fractions were combined and concentrated and the residue was dissolved in acetonitrile (4 ml) and purified by automated preparative liquid chromatography (Gilson system, 250 mm x 50 mm Gemini C18 10µm column, 120 ml/min) using an acetonitrile : 0.1% aqueous ammonia (5:95) : acetonitrile : 0.1% aqueous ammonia (95:5) gradient [90:10 to 50:50 (from 3 to 10 min) to 40:60 (from 18 to 20 min) to 5:95 (from 20 to 21 min)]. The appropriate fractions were combined and concentrated to give the free base of the compound of Example 48 (1.1 g) as a single enantiomer. To a solution of the free base the compound of Example 48 (419 mg, 1.1 mmol) in methanol (6 ml), at 0°C, was added dropwise hydrogen chloride in diethyl ether (1M, 1.30 ml). After stirring for 1.5 h, diethyl ether (34 ml) was added and the precipitate was collected by filtration. The resulting solid was washed with diethyl ether (2 x 40 ml) and dried in a vacuum oven at 50°C to give the hydrochloride salt, the compound of Example 48 (418 mg). HPLC method A - retention time 13.1 min

| Example | Structure Comment | MH⁺ exper | MH⁺ expect | Bovine EC₅₀ nM | Porcine EC₅₀ nM |
|---|---|---|---|---|---|
| **48** | Single enantiomer. HCl salt | 368.1 | 368.2 | 1.0 | 1.9 |

### Example 48

¹H-NMR (CD₃OD): 1.38 - 1.41 (3H), 2.01 - 2.17 (2H), 4.88 - 4.92 (1H), 6.74 - 6.78 (2H), 6.99 - 7.04 (2H), 7.06 - 7.10 (2H), 7.25 - 7.29 (1H)

### Example 49

### 6-{[3-(3,5-Dibromo-2-hydroxyphenyl)-1-methylpropyl]amino}-7-hydroxy-4,5,6,7-tetrahydro-imidazo[4,5,1-jk][1]benzazepin-2(1H)-one

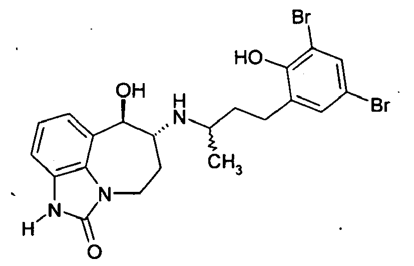

To a mixture of the compound of Preparation 9 (2.5 g, 9.8 mmol) and triethylamine (0.4 ml, 2.9 mmol) in methanol (15 ml) was added the compound of Preparation 70 (3.7 g, 11.0 mmol), followed by sodium cyanoborohydride (0.9 g, 14.7 mmol). The reaction mixture was heated at 50°C for 18 h, cooled and quenched with citric acid. The mixture was adjusted to pH 7 by addition of sodium hydrogen carbonate and concentrated *in vacuo.* The residue was pre-absorbed onto silica and passed through a silica plug, eluting with dichloromethane : 2.5% ammonia in methanol [4:1]. The filtrate was concentrated *in vacuo* and purified by automated flash chromatography (Biotage™, 65i silica cartridge) with gradient elution, dichloromethane : 2.5% ammonia in methanol [97:3 to 94:6]. The appropriate fractions were combined and concentrated to give the compound of Example 49 (765 mg) as a single enantiomer. HPLC Method A - retention time 12.70 min.

| Example | Structure Comment | MH⁺ exper | MH⁺ expect | Bovine EC₅₀ nM | Porcine EC₅₀ nM |
|---|---|---|---|---|---|
| **49** | Single enantiomer | N/A | N/A | 1.6 | 2.3 |

### Example 49

¹H-NMR (*d*₆-DMSO): 0.97 - 1.00 (3H), 4.68 - 4.70 (1H), 6.85 - 6.87 (1H), 6.94 - 6.97 (1H), 7.17-7.19 (1H), 7.20 - 7.22 (1H), 7.40-7.41 (1H)

### Example 50

### 6-{[3-(2-Chloro-3-hydroxyphenyl)-1-methylpropyl]amino}-7-hydroxy-4,5,6,7-tetrahydro-imidazo[4,5,1-jk][1]benzazepin-2(1H)-one

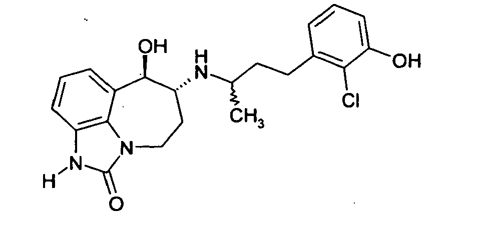

To a mixture of the compound of Preparation 9 (300 mg, 1.2 mmol) and the compound of Preparation 49 (280 mg. 1.4 mmol) in methanol (5 ml), under nitrogen, was added triethylamine (0.05 ml, 0.4 mmol). After stirring for 20 min, sodium cyanoborohydride (111 mg, 1.8 mmol) was added and the reaction mixture was heated at 60°C, under nitrogen, for 18 h. After cooling, citric acid (500 mg) was added and the mixture was heated at 60°C for 3 h. To the mixture was added water (0.2 ml), followed by excess sodium hydrogen carbonate and the mixture was stirred at room temperature for 18 h. The mixture was concentrated *in vacuo* and the residue was purified by automated flash chromatography (Biotage™, 40+M silica cartridge) with gradient elution, dichloromethane : 2.5% ammonia in methanol [96:4 to 89:11]. The appropriate fractions were combined and concentrated and further purified by automated preparative liquid chromatography (Gilson system, 150 mm x 21.4 mm Gemini C18 5µm column, 20 ml/min) using an acetonitrile : 0.1% aqueous ammonia (5:95) : acetonitrile : 0.1% aqueous ammonia (95:5) gradient [90:10 to 78:22 (from 2 to 8 min) to 50:50 (from 15 to 20 min) to 5:95 (from 21 to 22 min)]. The appropriate fractions were combined and concentrated to give the compound of Example 50 (61 mg) as a single enantiomer. HPLC method A - retention time 11.51 min.

| Example | Structure Comment | MH⁺ exper | MH⁺ expect | Bovine EC₅₀ nM | Porcine EC₅₀ nM |
|---|---|---|---|---|---|
| **50** | Single enantiomer | 402.3 | 402.2 | 1.7 | 1.3 |

### Example 50

¹H-NMR (CD₃OD): 1.26 - 1.28 (3H), 1.78 - 1.85 (2H), 4.65 - 4.67 (1H), 6.73 - 6.76 (2H), 6.98 - 7.03 (2H), 7.04-7.07 (1H), 7.19-7.21 (1H)

### Example 51

### 2,2,2-Trifluoro-N-{2-[3-{[7-hydroxy-2-oxo-1,2,4,5,6,7-hexahydroimidazo[4,5,1-jk][1]benzazepin-6-yl]amino}butyl]phenyl}acetamide

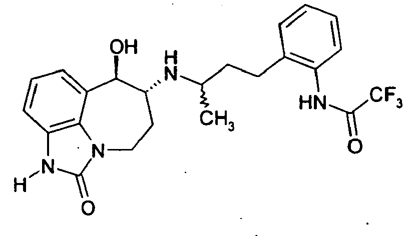

To a mixture of the compound of Preparation 9 (247 mg, 1.0 mmol) and the compound of Preparation 71 (250 mg, 1.0 mmol) in methanol (8 ml), under nitrogen, was added triethylamine (0.04 ml, 0.4 mmol). After stirring for 20 min, sodium cyanoborohydride (91 mg, 1.5 mmol) was added and the reaction mixture was heated at 60°C under nitrogen for 18 h. After cooling, citric acid (500 mg) was added and the mixture was heated at 60°C for 3 h. To the mixture was added water (0.2 ml), followed by excess sodium hydrogen carbonate and the mixture was stirred at room temperature for 18 h. The mixture was concentrated *in vacuo* and the residue was pre-absorbed onto silica (10 g) and passed through a silica plug (10 g), eluting with dichloromethane : 2.5% ammonia in methanol [4:1]. The filtrate was concentrated *in vacuo* and the residue was dissolved in acetonitrile : water (9:1, 4 ml) and purified by automated preparative liquid chromatography (Gilson system, 150 mm x 21.4 mm Gemini C18 5µm column, 20 ml/min) using an acetonitrile : 0.1% aqueous ammonia (5:95) : acetonitrile : 0.1% aqueous ammonia (95:5) gradient [90:10 to 80:20 (from 2 to 15 min) to 50:50 (from 24 to 26 min) to 5:95 (from 26 to 28 min)]. The appropriate fractions were combined and concentrated to give the compound of Example 51 (61 mg) as a single enantiomer.. HPLC method A - retention time 12.72 min.

| Example | Structure Comment | MH⁺ exper | MH⁺ expect | Bovine EC₅₀ nM | Porcine EC₅₀ nM |
|---|---|---|---|---|---|
| **51** | Single enantiomer | N/A | N/A | 3.6 | 11 |

### Example 51

¹H-NMR (CD₃OD): 1.15-1.17 (3H), 2.57-2.60 (2H), 4.64 - 4.66 (1H), 6.59-6.61 (1H), 6.67 - 6.69 (1H), 6.91-6.97 (2H), 6.99 - 7.01 (1H), 7.05-7.07 (1H), 7.20-7.22 (1H)

### Example 52

### 4-[-3-{[7-Hydroxy-2-oxo-1,2,4,5,6,7-hexahydroimidazo[4,5,1-jk][1]benzazepin-6-yl]amino}butyl]-N-(2,2,2-trifluoroethyl)benzamide

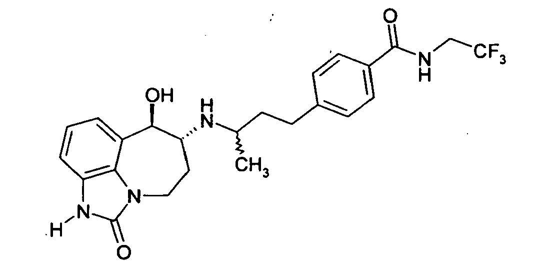

To a mixture of the compound of Preparation 9 (293 mg, 1.1 mmol) and triethylamine (0.05 ml, 0.4 mmol) in methanol (15 ml) was added the compound of Preparation 89 (172 mg, 0.6 mmol), followed by sodium cyanoborohydride (108 mg, 1.6 mmol) and the reaction mixture was heated at 50°C for 18 h. After cooling, the mixture was quenched by addition of water (3 ml) and citric acid was added, followed by excess sodium hydrogen carbonate. The mixture was stirred at room temperature for 30 min and then passed through a silica plug, eluting with dichloromethane : 2.5% ammonia in methanol [3:1]. The filtrate was concentrated *in vacuo* and the residue was dissolved in acetonitrile : water (9:1, 6 ml) and purified by automated preparative liquid chromatography (Gilson system, 250 mm x 50 mm Gemini C18 10µm column, 120 ml/min) using an acetonitrile : 0.1% aqueousammonia (5:95) : acetonitrile : 0.1% aqueous ammonia (95:5) gradient [90:10 to 65:35 (from 2 to 5 min) to 50:50 (from 11 to 12 min) to 5:95 (from 14 to 15 min)]. The appropriate fractions were combined and concentrated to give the compound of Example 52 (120 mg) as a single enantiomer. HPLC method A - retention time 13.47 min

| Example | Structure Comment | MH⁺ exper | MH⁺ expect | Bovine EC₅₀ nM | Porcine EC₅₀ nM |
|---|---|---|---|---|---|
| **52** | Single enantiomer | 477.1 | 477.2 | 7.7 | 38.4 |

### Example 52

¹H-NMR (CD₃OD): 1.16-1.19 (3H), 2.75 - 2.79 (2H), 4.04 - 4.10 (2H), 4.63 - 4.65 (1H), 6.98 - 7.00 (1H), 7.01-7.03 (1H), 7.19-7.21 (1H), 7.30-7.33 (2H), 7.75-7.77 (2H)

### Example 53

### 3-[[3-{[7-Hydroxy-2-oxo-1,2,4,5,6,7-hexahydroimidazo[4,5,1-jk][1]benzazepin-6-yl]amino}butyl]benzoic acid

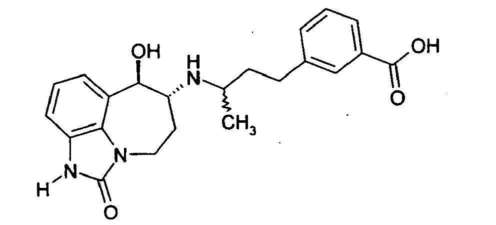

To a mixture of the compound of Preparation 9 (277 mg, 1.1 mmol) and triethylamine (0.05 ml, 0.3 mmol) in methanol (15 ml) was added the compound of Preparation 51 (250 mg, 1.3 mmol), followed by sodium cyanoborohydride (102 mg, 1.6 mmol) and the reaction mixture was heated at 50°C for 18 h. After cooling, the mixture was quenched by addition of water (3 ml) and citric acid was added, followed by excess sodium hydrogen carbonate. The mixture was stirred at room temperature for 30 min and then passed through a silica plug, eluting with dichloromethane : 2.5% ammonia in methanol [3:1]. The filtrate was concentrated *in vacuo* and the residue was dissolved in acetonitrile : water (9:1, 6 ml) and purified by automated preparative liquid chromatography (Gilson system, 150 mm x 21 mm Gemini C18 5µm column, 25 ml/min) using a 0.1% aqueous ammonia : acetonitrile gradient [5:95 to 20:80 (from 0 to 6 min) to 98:2 (from 8 to 8.5 min)]. The appropriate fractions were combined and concentrated to give the compound of Example 53 (72 mg) as a single enantiomer. HPLC method A - retention time 8.11 min.

| Example | Structure Comment | MH⁺ exper | MH⁺ expect | Bovine EC₅₀ nM | Porcine EC₅₀ nM |
|---|---|---|---|---|---|
| **53** | Single enantiomer | 396.1 | 396.2 | 167 | 78.8 |

### Example 53

¹H-NMR (CD₃OD): 1.38-1.40 (3H), 4.90-4.92 (2H), 7.00-7.02 (1H), 7.08-7.11 (1H), 7.27-7.33 (3H), 7.78-7.80 (1H), 7.84-7.86 (1H)

### Example 54

### 6-{[3-(4,5-Difluoro-2-hydroxyphenyl)-1-methylpropyl]amino}-7-hydroxy-4,5,6,7-tetrahydro-imidazo[4,5,1-jk][1]benzazepin-2(1H)-one

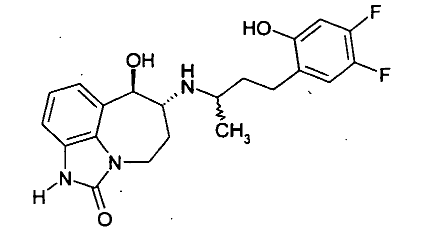

To a mixture of the compound of Preparation 9 (445 mg, 1.7 mmol) and the compound of Preparation 52 (418 mg, 2.1 mmol) in methanol (5 ml), under nitrogen, was added triethylamine (0.07 ml, 0.5 mmol). After stirring for 20 min, sodium cyanoborohydride (164 mg, 2.6 mmol) was added and the reaction mixture was heated at 60°C under nitrogen for 18 h. After cooling, citric acid (500 mg) was added and the mixture was heated at 60°C for 3 h. To the mixture was added water (0.2 ml), followed by excess sodium hydrogen carbonate and the mixture was stirred at room temperature for 18 h. The mixture was concentrated *in vacuo* and the residue was pre-absorbed onto silica (10 g) and passed through a silica plug (10 g), eluting with dichloromethane : 2.5% ammonia in methanol [4:1]. The filtrate was concentrated *in vacuo* and the residue was purified by automated flash chromatography (Biotage™, 40+M silica cartridge) with gradient elution, dichloromethane : 2.5% ammonia in methanol [96:4 to 89:11]. The appropriate fractions were combined and concentrated to give the compound of Example 54 (69 mg) as a single enantiomer. HPLC method A - retention time 14.49 min

| Example | Structure Comment | MH⁺ exper | MH⁺ expect | Bovine EC₅₀ nM | Porcine EC₅₀ nM |
|---|---|---|---|---|---|
| **54** | Single enantiomer | N/A | N/A | | |

### Example 54

¹H-NMR (CD₃OD): 1.15 - 1.17 (3H), 1.70 - 1.76 (2H), 4.65 - 4.67 (1H), 6.58 - 6.60 (1H), 6.93 - 6.99 (2H), 7.02 - 7.04 (1H), 7.22 - 7.24 (1H)

**The following were prepared by methods similar to those used for Examples 48 - 54:**

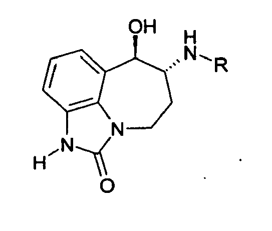

| Example | R | Structure Comment | MH⁺ exper | MH⁺ expect | Bovine EC₅₀ nM | Porcine EC₅₀ nM | From the compound of Preparation |
|---|---|---|---|---|---|---|---|
| **55** | 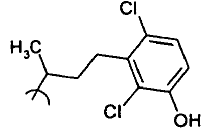 | Single enantiomer | 436.3 | 436.1 | 4.3 | 2.7 | 53 |
| **56** | 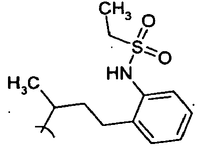 | Single enantiomer | 459.2 | 459.2 | 5.2 | 2.8 | 72 |
| **57** | 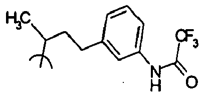 | Single N/A enantiomer | N/A | N/A | 6.1 | 7.1 | 73 |
| **58** | 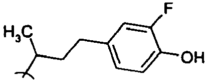 | Single enantiomer | N/A | N/A | 1.1 | 2.4 | 56 |
| **59** | 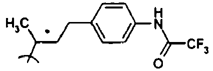 | Single enantiomer | N/A | N/A | 10.4 | 12.1 | 74 |
| **60** | 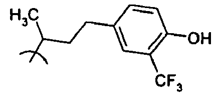 | Single enantiomer | 436.2 | 436.2 | 10.5 | 15.9 | 54 |
| **61** | 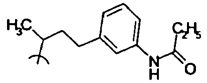 | Single enantiomer | 423.2 | 423.2 | 12 | 12.5 | 75 |
| **62** | 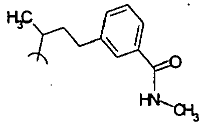 | Single enantiomer | 409.2 | 409.2 | 24.3 | 18.9 | 90 |
| **63** | 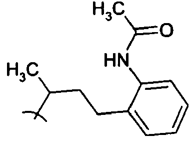 | Single enantiomer | 409.2 | 409.2 | 39.2 | 40.8 | 76 |
| **64** | 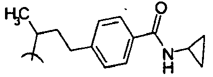 | Single enantiomer | 435.2 | 435.2 | 46.4 | 42.9 | 95 |
| **65** | 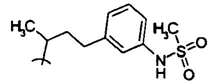 | Single enantiomer | 445.2 | 445.2 | 74.7 | 31.5 | 77 |
| **66** | 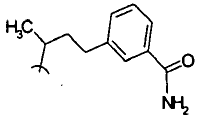 | Single enantiomer | 395.2 | 395.2 | 137 | 141 | 96 |
| **67** | 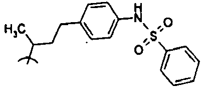 | Single enantiomer | 507.1 | 507.2 | 156 | N/A | 101 |
| **68** | 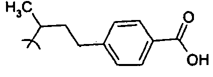 | Single enantiomer | 396.2 | 396.2 | 198 | 128 | 50 |
| **69** | 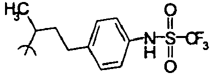 | Single enantiomer | 499.1 | 499.2 | 234 | 150.3 | 102 |

### Example 55

### 6-{[3-(2,6-Dichloro-3-hydroxyphenyl)-1-methylpropyl]amino}-7-hydroxy-4,5,6,7-tetrahydro-imidazo[4,5,1-jk][1]benzazepin-2(1H)-one

¹H-NMR (CD₃OD): 1.18 - 1.20 (3H), 4.67 - 4.69 (1H), 6.74 - 6.76 (1H), 6.99 - 7.01 (1H), 7.03 - 7.05 (1H), 7.08 - 7.10 (1H), 7.20 - 7.22 (1H)
HPLC method A - retention time 11.28 min

### Example 56

### N-{2-[3-{[7-Hydroxy-2-oxo-1,2,4,5,6,7-hexahydroimidazo[4,5,1-jk][1]benzazepin-6-yl]amino}butyl]phenyl}ethanesulfonamide

Experimental MH⁺ 459.2; expected 459.2
¹H-NMR (CD₃OD): 1.10 1.12 (3H), 1.28 - 1.31 (3H), 3.05 - 3.11 (2H), 4.15 - 4.19 (1H), 6.98 - 7.00 (1H), 7.06 - 7.08 (1H), 7.12 - 7.18 (2H), 7.24 - 7.26 (1H), 7.30 - 7.32 (1H), 7.38 - 7.40 (1H)
HPLC method A - retention time 12.77 min

### Example 57

### 2,2,2-Trifluoro-N-{3-[3-{[7-Hydroxy-2-oxo-1,2,4,5,6,7-hexahydroimidazo[4,5,1-jk][1]benzazepin-6-yl]amino}butyl]phenyl}acetamide

HPLC method A - retention time 12.19 min

### Example 58

### 6-{[3-(3-Fluoro-4-hydroxyphenyl)-1-methylpropyl]amino}-7-hydroxy-4,5,6,7-tetrahydro-imidazo[4,5,1-jk][1]benzazepin-2(1H)-one

¹H-NMR (CD₃OD): 1.40 - 1.42 (3H), 2.05 - 2.11 (2H), 4.95 - 4.97 (1H), 6.83 - 6.87 (2H), 6.97 - 7.00 (1H), 7.03-7.05(1H), 7.10-7.13(1H), 7.30-7.32 (1H)

### Example 59

### 2,2,2-Trifluoro-N-{4-[(3-{[7-Hydroxy-2-oxo-1,2,4,5,6,7-hexahydroimidazo[4,5,1-jk][1]benzazepin-6-yl]amino}butyl]phenyl}acetamide

¹H-NMR (CD₃OD): 1.09 - 1.12 (3H), 1.70 - 1.80 (2H), 4.61 - 4.63 (1H), 6.61 - 6.64 (2H), 6.88 - 6.91 (2H), 6.99 - 7.01 (1H), 7.03 - 7.05 (1H). 7.18 - 7.20 (1H)
HPLC method A - retention time 11.90 min

### Example 60

### 7-Hydroxy-6-({3-[4-hydroxy-3-(trifluoromethyl)phenyl]-1-methylpropyl}amino)-4,5,6,7-tetrahydro-imidazo[4,5,1-jk][1]benzazepin-2(1H)-one

¹H-NMR (CD₃OD): 1.14 - 1.16 (3H), 1.76 - 1.84 (2H), 4.64 - 4.66 (1H), 6.80 - 6.82 (1H), 6.99 - 7.01 (1H), 7.02 - 7.04 (1H), 7.20 - 7.23 (2H), 7.29 - 7.31 (1H)
HPLC method A - retention time 12.50 min

### Example 61

### N-{2-[3-{[7-Hydroxy-2-oxo-1,2,4,5,6,7-hexahydroimidazo[4,5,1-jk][1]benzazepin-6-yl]amino}butyl]phenyl}propanamide

¹H-NMR (CD₃OD): 1.10 - 1.12 (3H), 1.17 - 1.20 (3H), 2.36 - 2.40 (2H), 4.62 - 4.64 (1H), 6.89 - 6.91 (1H), 6.99 - 7.01 (1H), 7.02 - 7.05 (1H), 7.17 - 7.20 (2H), 7.30 - 7.32 (1H), 7.40 - 7.42 (1H)
HPLC method A - retention time 12.67 min

### Example 62

### 3-[3-{[7-Hydroxy-2-oxo-1,2,4,5,6,7-hexahydroimidazo[4,5,1-jk][1]benzazepin-6-yl]amino}butyl]-N-methylbenzamide

¹H-NMR (CD₃OD): 1.16 - 1.18 (3H), 2.90 - 2.91 (3H), 4.63 - 4.65 (1H), 6.98 - 7.01 (1H), 7.03 - 7.06 (1H), 7.18 - 7.20 (1H), 7.32 - 7.37 (2H), 7.38 - 7.40 (1H), 7.65 - 7.67 (1H)
HPLC method A - retention time 11.40 min

### Example 63

### N-{2-[3-{[7-Hydroxy-2-oxo-1,2,4,5,6,7-hexahydroimidazo[4,5,1-jk][1]benzazepin-6-yl]amino}butyl]phenyl}acetamide

¹H-NMR (CD₃OD): 1.11 - 1.14 (3H), 2.07 - 2.09 (3H), 4.62 - 4.64 (1H), 6.99 - 7.01 (1H), 7.04 - 7.07 (1H), 7.15 - 7.22 (3H), 7.22 - 7.27 (2H)
HPLC method A - retention time 11.46 min

### Example 64

### N-Cyclopropyl-4-[3-{[7-Hydroxy-2-oxo-1,2,4,5,6,7-hexahydroimidazo[4,5,1-jk][1]benzazepin-6-yl]amino}butyl]benzamide

¹H-NMR (*d₆*-DMSO): 0.54 - 0.59 (2H), 0.62 - 0.66 (2H), 0.98 - 1.02 (3H), 3.00 - 3.03 (1H), 4.47 - 4.50 (1H), 6.81 - 6.83 (1H), 6.89 - 6.92 (1H), 7.01 - 7.04 (1H). 7.20 - 7.23 (2H), 7.67 - 7.70 (2H)
HPLC method A - retention time 12.01 min

### Example 65

### N-{3-[3-{[7-Hydroxy-2-oxo-1,2,4,5,6,7-hexahydroimidazo[4,5,1-jk][1]benzazepin-6-yl]amino}butyl]phenyl}methanesulfonamide

¹H-NMR (CD₃OD): 1.14 - 1.16 (3H), 2.90.- 2.92 (3H), 4.62 - 4.64 (1H). 6.97 - 7.01 (2H), 7.02 - 7.07 (2H), 7.09-7.11 (1H). 7.19 - 7.22 (2H)
HPLC method A - retention time 10.00 min

### Example 66

### 3-[3-{[7-Hydroxy-2-oxo-1,2,4,5,6,7-hexahydroimidazo[4,5,1-jk][1]benzazepin-6-yl]amino}butyl]benzamide

¹H-NMR (CD₃OD): 1.37 - 1.39 (3H), 4.86 - 4.88 (1H), 7.00 - 7.02 (1H), 7.07 - 7.10 (1H), 7.26 - 7.31 (3H), 7.78 - 7.80 (1H), 7.85 - 7.86 (1H)
HPLC method A - retention time 8.02 min

### Example 67

### N-{4-[3-{[7-Hydroxy-2-oxo-1,2,4,5,6,7-hexahydroimidazo[4,5,1-jk][1]benzazepin-6-yl]amino}butyl]phenyl}benzenesulfonamide

¹H-NMR (CD₃OD): 1.06 - 1.09 (3H), 4.60 - 4.62 (1H), 6.96 - 7.00 (2H), 7.00 - 7.06 (4H), 7.18 - 7.20 (1H), 7.41 - 7.45 (2H), 7.49 - 7.51 (1H), 7.70 - 7.72 (2H)
HPLC method A - retention time 11.41 min

### Example 68

### 4-[3-{[7-Hydroxy-2-oxo-1,2,4,5,6,7-hexahydroimidazo[4,5,1-jk][1]benzazepin-6-yl]amino}butyl]benzoic acid

¹H-NMR (CD₃OD): 1.25 - 1.27 (3H), 1.90 - 2.00 (2H), 4.78 - 4.80 (1H), 7.00 - 7.02 (1H), 7.06 - 7.09 (1H), 7.20 - 7.24 (3H), 7.84 - 7.86 (2H)
HPLC method A - retention time 8.02 min

### Example 69

### 1,1,1-Trifluoro-N-{4-[3-{[7-hydroxy-2-oxo-1,2,4,5,6,7-hexahydroimidazo[4,5,1-jk][1]benzazepin-6-yl]amino}butyl]phenyl}methanesulfonamide

¹H-NMR (CD₃OD): 1.36 - 1.38 (3H), 1.96 - 2.05 (2H), 4.80 - 4.82 (1H), 7.02 - 7.09 (5H), 7.09 - 7.12 (1H), 7.25 - 7.27 (1H)
HPLC method A - retention time 9.92 min

### Example 70

### Methyl 4-[3-{[7-hydroxy-2-oxo-1,2,4,5,6,7-hexahydroimidazo[4,5,1-jk][1]benzazepin-6-yl]amino}butyl]benzoate

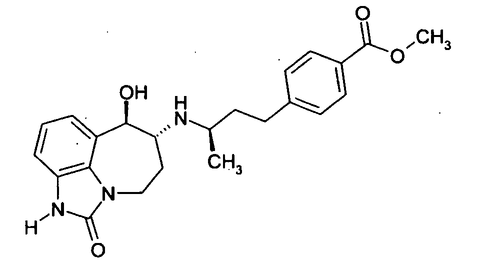

To a mixture of the compound of Preparation 9 (207 mg, 0.8 mmol) and triethylamine (0.03 ml, 0.2 mmol) in methanol (5 ml) was added the compound of Preparation 97 (186 mg, 1.0 mmol), followed by sodium cyanoborohydride (76 mg, 1.2 mmol) and the reaction mixture was heated at 50°C for 18 h. Additional sodium cyanoborohydride (76 mg, 1.2 mmol) was added and the reaction mixture was stirred at 50°c for another 18 days. After cooling, the mixture was quenched by addition of water (3 ml) and citric acid was added, followed by excess sodium hydrogen carbonate. The mixture was stirred at room temperature for 30 min and then concentrated *in vacuo.* To the residue was added methanol (250 ml) and silica and the mixture was concentrated *in vacuo.* The product/silica mix was passed through a silica plug, eluting with dichloromethane : 2.5% ammonia in methanol [4:1]. The filtrate was concentrated *in vacuo* and the residue was dissolved in acetonitrile : water (9:1, 4 ml) and purified by automated preparative liquid chromatography (Gilson system, 150 mm x 21.4 mm Gemini C18 5µm column, 20 ml/min) using acetonitrile : 0.1% aqueous ammonia (5:95) : acetonitrile : 0.1% aqueous ammonia (95:5) gradient [90:10 to 78:22 (from 2 to 5 min) to 70:30 (from 16 to 18 min) to 5:95 (from 30 to 31 min)]. The appropriate fractions were combined and concentrated to give the compound of Example 70 (6 mg) as a single enantiomer. HPLC method A - retention time 14.01 min.

| Example | Structure Comment | MH⁺ exper | MH⁺ expect | Bovine EC₅₀ nM | Porcine EC₅₀ nM |
|---|---|---|---|---|---|
| 70 | Single enantiomer | 410.2 | 410.2 | 8.2 | 6.2 |

### Example 70

¹H-NMR (*d*₆-DMSO): 1.00 - 1.03 (3H), 3.79 - 3.82 (3H), 4.48 - 4.50 (1H), 6.83 - 6.85 (1H), 6.88 - 6.91 (1H), 7.02 - 7.05 (1H), 7.30 - 7.33 (2H), 7.91 - 7.94 (2H)

### Preparation 1

### Hydrochloride salt of 6-amino-7-hydroxy-4,5,6,7-tetrahydroimidazo[4,5,1-jk][1]benzazepin-2(1H)-one

To a solution of the compound of Preparation 2 (53.5 g, 211 mmol) in methanol (2600 ml), at 0°C, was added sodium borohydride (8.8 g, 232 mmol), over 30 min. The reaction mixture was stirred at room temperature for 18 h, before addition of hydrochloric acid (2N, 120 ml). The mixture was concentrated *in vacuo* and the residue was re-crystallised from isopropanol : water (3:1, 700 ml). The solid was washed with diethyl ether and dried in a vacuum oven overnight to give the title compound (33.8 g).
¹H-NMR (*d₆*-DMSO): 2.00 - 2.10 (1H). 2.30 - 2.40 (1H). 3.60 - 3.70 (1H). 4.10 - 4.20 (1H), 4.85 - 4.95 (1H), 6.45 - 6.50 (1H), 6.90 - 6.95 (1H), 6.95 - 7.00 (1H), 7.15 - 7.20 (1H)

### Preparation 2

### Hydrochloride salt of 6-amino-5,6-dihydroimidazo[4,5,1-jk][1]benzazepine-2,7(1H,4H)-dione

A mixture of the compound of Preparation 3 (35.3 g, 153 mmol) and palladium (10% on carbon, 11g) and concentrated hydrochloric acid (25.5 ml) in methanol (300 ml) was stirred at room temperature under hydrogen (22 psi) for 3 h. The reaction mixture was filtered through Arbocel®, washing through with methanol and water, and ensuring the catalyst did not dry out. The filtrate was concentrated *in vacuo* and the residue was triturated with acetone to give the title compound (30.0 g).
¹H-NMR (*d₆*-DMSO): 2.20 - 2.30 (1H), 2.40 - 2.50 (1H), 3.70 - 3.80 (1H), 4.30 - 4.40 (1H), 4.60 - 4.70 (1H), 7.10 - 7.15 (1H). 7.25 - 7.30 (1H), 7.60 - 7.65 (1H)

### Preparation 3

### 4,5-Dihydroimidazo[4,5,1-jk][1]benzazepine-2,6,7(1H)-trione 6-oxime

To a solution of the compound of Preparation 4 (10.3 g, 51 mmol) in acetic acid (150 ml) was added *tert-*butyl nitrite (16 ml, 135 mmol), followed by hydrochloric acid (4N in dioxane, 33.4 ml). The reaction mixture was stirred at room temperature for 3 h and then filtered. The solid material was dried in a vacuum oven to give the title compound. (10.0 g).
Experimental MH⁺ 232.1; expected 232.1

### Preparation 4

### 5,6-Dihydroimidazo[4,5,1-jk][1]benzazepine-2,7(1H,4H)-dione

To a solution of the compound of Preparation 5 (45.0 g, 0.2 mol) in dichloromethane (150 ml) was added thionyl chloride (30 ml, 0.4 mol) and the reaction mixture was stirred at room temperature for 2 h. The mixture was concentrated *in vacuo* and to the residue was added dichloromethane (1000 ml) and aluminium chloride (84.0 g, 0.6 mol), added portionwise. After stirring at room temperature overnight, the reaction mixture was heated under reflux for 2 h and then concentrated *in vacuo.* To the residue was added ice water (2000 ml) and concentrated hydrochloric acid (50 ml), followed by additional ice water (2000 ml). The resulting precipitate was collected by filtration, washed with water (4 x 250 ml) and dissolved in sodium hydroxide solution (1N, 600 ml). The solution was washed with dichloromethane (2 x 150 ml) and cyclohexane (150 ml) and adjusted to pH 10 by addition of dry ice. The solid material was collected by filtration, washed with water (3 x 50 ml) and dried overnight at 40°C to give the title compound (30.0 g).
¹H-NMR (*d₆*-DMSO): 2.03 - 2.11 (2H), 2.90 - 3.00 (2H), 3.85 - 3.95 (2H), 7.02 - 7.10 (1H), 7.17 - 7.24 (1H), 7.50-7.58 (1H)

### Preparation 5

### 4-(2-Oxo-2,3-dihydro-1H-benzimidazol-1-yl)butanoic acid

To a solution of the compound of Preparation 6 (152.0 g, 0.6 mol) in tetrahydrofuran (600 ml) was added concentrated hydrochloric acid (75 ml). The reaction mixture was stirred for 2 h and then poured into water (700 ml). The mixture was filtered, washing through with water (750 ml) and the solid material was dried overnight at 40°C to give the title compound (155.5 g).
¹H-NMR (*d₆*-DMSO): 1.80 - 1.89 (2H), 2.20 - 2.25 (2H), 3.74 - 3.82 (2H), 6.96 - 7.01 (3H), 7.05 - 7.10 (1H)

### Preparation 6

### 4-(3-Isopropenyl-2-oxo-2,3-dihydro-1H-benzimidazol-1-yl)butanoic acid

To a solution of the compound of Preparation 7 (223.8 g, 0.7 mol) in tetrahydrofuran (500 ml) was added aqueous sodium hydroxide solution (15% w/w, 500 ml). The reaction mixture was heated under reflux for 4 h, cooled to room temperature and stirred overnight. The tetrahydrofuran was removed by vacuum distillation (38°C) and the aqueous layer was extracted with dichloromethane (2 x 400 ml) and cyclohexane (2 x 300 ml). To the aqueous layer was added glacial acetic acid (250 ml) and the solution was cooled to 2°C. After stirring for 30 min, the product was collected by filtration, washing through with water (3 x 250 ml) at 2°C. The solid was dried overnight at 40°C to give the title compound (307.5 g).
Experimental MH⁺ 261.2; expected 261.1

### Preparation 7

### Ethyl 4-(3-isopropenyl-2-oxo-2,3-dihydro-1H-benzimidazol-1-yl)butanoate

A mixture of the compound of Preparation 8 (114.0 g, 0.7 mol), potassium carbonate (136 mg, 1 mol) and the compound of Preparation 166 (167.4 g, 0.9 mol) in acetone (500 ml) was heated under reflux for 18 h. The reaction mixture was then cooled to room temperature and filtered, washing through with acetone (250 ml). The filtrate was concentrated *in vacuo* and the residue was dried overnight at 40°C to give the title compound (223.8 g).
¹H-NMR (*d₆*-DMSO): 1.10 - 1.20 (3H), 2.10 - 2.15 (3H), 3.95 - 4.07 (2H), 5.10 - 5.12 (1H), 5.35 - 5.39 (1H), 7.00-7.10 (3H), 7.20-7.26 (1H)

### Preparation 8

### 1-Isopropenyl-1,3-dihydro-2H-benzimidazol-2-one

To a solution of the compound of Preparation 140 (98.0 g, 0.9 mol) in xylene (420 ml), at 120°C, was added 1,8-diazobicylo[5.4.0]undec-7-ene (1.5 ml), followed by the compound of Preparation 141 (130.0 g, 1.0 mol), added over 30 min. The reaction mixture was heated at 150°C, using a Dean-Stark apparatus, for 60 h and then cooled to room temperature. The solid product was isolated by filtration, washing with cold xylene (250 ml), and dried in a vacuum oven to give the title compound (208.4 g). ¹H-NMR (*d₆*-DMSO): 2.08 - 2.11 (3H), 5.05 - 5.11 (1H), 5.34 - 5.37 (1H), 6.98 - 7.01 (3H), 7.01 - 7.06 (1H), 10.90-11.00 (1H)

### Preparation 9

### Hydrochloride salt of (6R,7R)-6-amino-7-hydroxy-4,5,6,7-tetrahydroimidazo[4,5,1-jk][1]benzazepin-2(1H)-one

To the compound of Preparation 10 (160 mg, 0.5 mmol) was added hydrogen chloride (4N in dioxane, 1.25 ml) and the mixture was stirred at room temperature for 1 h. The mixture was concentrated *in vacuo* and to the residue was added dioxane (10 ml). The solution was re-concentrated *in vacuo* to give the title compound as the hydrochloride salt (135 mg).
¹H-NMR (CD₃OD): 2.07 - 2.13 (1H), 2.41 - 2.44 (1H). 3.50 - 3.54 (1H), 3.78 - 3.82 (1H). 4.20 - 4.26 (1H). 7.01 - 7.04 (1H), 7.10 - 7.14 (1H), 7.35- 7.37 (1H)

### Alternative synthesis

A mixture of the compound of Preparation 3 (11.0 g, 48 mmol), rhodium chloro(norbornadiene) dimer (55 mg, 0.1 mmol) and 1-[(S)-ferrocenyl-2-(*R*)-ethyl-1-dimethylamino)phenyl]-(*S*)-phosphino-1'-dicyclohexylphosphino-ferrocene (Solvias AG) (187 mg, 0.3 mmol) in methanol (165 ml) and water (11 ml) was purged with nitrogen (x 3) and heated at 80°C under a hydrogen atmosphere (20 bar) for 16 h. The mixture was filtered, washing through with methanol and the filtrate concentrated *in vacuo.* To the residue was added hydrogen chloride (4M in dioxane 14ml). The solution was concentrated *in vacuo* and the residue was purified by azeotropic distillation with isopropanol (2 x 50 ml). The residue was re-crystallised from isopropanol : water (6:1, 150 ml) and again from isopropanol : water (6:1, 80 ml) to give the title compound (6.5 g) as the hydrochloride salt.
¹H-NMR (*d₆*-DMSO): 1.96 - 2.05 (1H), 2.30 - 2.38 (1H), 3.60 - 3.68 (1H). 4.08 - 4.15 (1H), 4.82 - 4.88 (1H), 6.45 - 6.50 (1H), 6.90 - 6.93 (1H), 6.97 - 7.01 (1H), 7.15 - 7.18 (1H)

### Preparation 10

### tert-Butyl [(6R,7R)-7-hydroxy-2-oxo-1,2,4,5,6,7-hexahydroimidazo[4,5,1-jk][1]benzazepin-6-yl]-carbamate

The compound of Preparation 11 (500 mg, 1.6 mmol) was dissolved in isopropanol containing 0.1 % diethylamine (100 ml), with heating and sonication. The solution was purified by supercritical fluid chromatography (Berger Multigram III, 250 x 30 mm Chiralcel OJ-H, 5 µm column, 35°C, 180 ml/min) using supercritical carbon dioxide/isopropanol containing 0.1% diethylamine [85:15] as the mobile phase. The appropriate fractions were combined and concentrated to give the title compound as the desired enantiomer, which was used directly.

### Preparation 11

### tert-Butyl [7-hydroxy-2-oxo-1,2,4,5,6,7-hexahydroimidazo[4,5,1-jk][1]benzazepin-6-yl]carbamate

To a solution of the compound of Preparation 1 (1.0 g, 3.9 mmol) in methanol (20 ml) was added triethylamine (1.1 ml, 7.8 mmol), followed by the compound of Preparation 142 (1.71 g, 7.8 mmol). The reaction mixture was stirred for 1 h, concentrated *in vacuo* and to the residue was added dichloromethane (50 ml). The solution was washed with water (50 ml) and the precipitate was collected by filtration and dried in a vacuum oven to give the title compound (500 mg), which was used directly.

### Preparation 12

### 4-[4-(Cyclopropylmethoxy)phenyl]butan-2-one

To a solution of the compound of Preparation 106 (5.0 g, 31 mmol) in acetone (75 ml) was added caesium carbonate (20.0 g, 61.0 mmol), followed by the compound of Preparation 107 (5.9 ml, 61 mmol) and sodium iodide (0.4.6 g, 31.0 mmol). The reaction mixture was heated under reflux for 24 h, concentrated *in vacuo* and the residue was partitioned between ethyl acetate (75 ml) and water (100 ml). The two layers were separated and the aqueous layer was extracted with ethyl acetate (75 ml). The combined organic phases were washed with brine (100 ml), dried (MgSO₄) and concentrated *in vacuo* to give the title compound (5.7 g), which was used directly for reductive amination with the compound of Preparation 1.

### Preparation 13

### 4-(2-Hydroxyphenyl)-4-methylpentan-2-one

To a solution of the compound of Preparation 14 (222 mg, 0.8 mmol) in tetrahydrofuran (2 ml), at -60°C and under nitrogen, was added methyllithium (1.6M in diethyl ether, 1.8 ml, 2.8 mmol) dropwise over 5 min. The reaction mixture was stirred at -60°C for 30 min, quenched with ethanol (0.2 ml) and allowed to warm to room temperature overnight. The mixture was partitioned between water and diethyl ether and the organic phase was separated, washed with brine, dried (MgSO₄) and concentrated *in vacuo* to give the title compound (130 mg).
Experimental (M-H⁺)⁻ 191.3; expected 191.1

### Preparation 14

### 2-(1,1-Dimethyl-3-morpholin-4-yl-3-oxopropyl)phenol

A solution of the compound of Preparation 15 (950 mg, 5.4 mmol) in morpholine (30 ml) was heated at 85°C for 18 h. To the reaction mixture was added water (5 ml) and the precipitate was collected by filtration, washed with water and dried in a vacuum oven to give the title compound (1.22 g). Experimental MH⁺ 264.0; expected 264.2

### Preparation 15

### 4,4-Dimethylchroman-2-one

To a solution of the compound of Preparation 105 (2.0 g, 11.5 mmol) in 1,2-dichloroethane (20 ml) was added aluminium chloride (2.3 g, 17.2 mmol) over 5 min and the reaction mixture was stirred at room temperature, under nitrogen, for 60 h. To the reaction mixture was added dichloromethane and the solution was concentrated *in vacuo.* The residue was partitioned between water and dichloromethane and the two layers were separated. The organic layer was further washed with water, dried (MgSO₄) and concentrated *in vacuo..*The residue was purified by automated flash chromatography (Biotage™ 40M cartridge) with gradient elution, cyclohexane : dichloromethane [3:1 to 1:3]. The appropriate fractions were combined and concentrated to give the title compound (1.4 g).
Experimental MH⁺ 177.2; expected 177.1

### Preparation 16

### 4-(3-Chloro-2-hydroxyphenyl)but-3-en-2-one

To a solution of the compound of Preparation 108 (17.5 g, 112.0 mmol) in acetone (49 ml), at 0°C, was added aqueous sodium hydroxide solution (5M, 33 ml) over 10 min. The reaction mixture was stirred at 0°C for 60 min and then at room temperature for 18 h. To the mixture was added water (500 ml) and the solution was extracted with dichloromethane (100 ml). The aqueous phase was acidified with excess aqueous citric acid solution and the resulting precipitate was collected by filtration. The solid was washed with water (100 ml), aqueous sodium hydrogen carbonate solution (100 ml), aqueous sodium metabisulfite solution (100 ml) and additional water (3 x 100 ml) and air dried to give the title compound (20g)
¹H-NMR (CDCl₃): 2.39 - 2.41 (3H), 6.78 - 6.93 (2H), 7.34 - 7.38 (1H). 7.41 -7.46 (1H), 7.77 - 7.83 (1H)

### Preparation 17

### 4-(5-Fluoro-2-hydroxyphenyl)but-3-en-2-one

To a solution of the compound of Preparation 110 (900 mg, 6.4 mmol) in acetone (20 ml), at 0°C. was added aqueous sodium hydroxide solution (5M, 1.9 ml). After stirring at 0°C for 1 h, water (50 ml) was added and the mixture was extracted with dichloromethane (50 ml). The aqueous phase was acidifed with hydrochloric acid (4M) and the solution was extracted with dichloromethane (100 ml). The combined organic phases were washed with water (20 ml), saturated aqueous sodium metabisulphite solution (30 ml), and additional water (3 x 20 ml), dried (MgSO₄) and concentrated *in vacuo* to give the title compound (900 mg).
¹H-NMR (CD₃OD): 2.31 - 2.34 (3H), 6.76 - 6.83 (2H), 6.92 - 6.99 (1H). 7.24 - 7.28 (1H). 7.82 - 7.88 (1H)

### Preparation 18

### 4-(4,5-Difluoro-2-hydroxyphenyl)but-3-en-2-one

To a solution of the compound of Preparation 60 (3.5 g, 22.0 mmol) in acetone (50 ml), at 0°C, was added aqueous sodium hydroxide solution (5M, 6.6 ml, 33.2 mmol). The reaction mixture was stirred at 0°C for 1 h and then at room temperature for 18 h. The mixture was diluted with water (200 ml) and acidifed with hydrochloric acid (4M). The mixture was extracted with diethyl ether (3 x 300 ml) and the combined extracts were dried (MgSO₄) and concentrated *in vacuo* to give the title compound (2.9 g).
¹H-NMR (CDCl₃): 2.39 - 2.43 (3H), 6.81 - 6.89 (1H), 7.25 - 7.31 (2H), 7.67 - 7.77 (1H)

### Similarly prepared were:

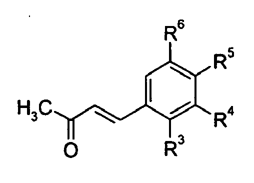

| Preparation | R³ | R⁴ | R⁵ | R⁶ | MH⁺ exper | MH⁺ expected | From the compound of Preparation |
|---|---|---|---|---|---|---|---|
| **19** | H | CF₃ | OH | H | N/A | | 157 |
| **20** | OH | OCH₃ | H | H | 193.1 | 193.1 | 111 |
| **21** | OH | F | H | F | 196.9 | 197.0 | 113 |
| **22** | OH | H | H | CN | N/A | | 59 |
| **23** | OH | CH₃ | H | H | N/A | | 119 |
| **24** | OCH₃ | H | H | H | N/A | | 121 |
| **25 -** | OH | H | H | OCH₃ | N/A | | 122 |
| **26** | H | H | F | H | N/A | | 124 |
| **27** | OH | H | F | H | N/A | | 125 |
| **28** | OH | Cl | H | Cl | 230.9 | 231.1 | 126 |
| **29** | OH | H | Cl | H | 194.9 | 195.0 | 127 |
| **30** | OH | Cl | H | F | 212.9 | 213.0 | 128 |
| **31** | OH | F | H | H | 181.1 | 181.1 | 129 |
| **32** | OH | H | H | CF₃ | N/A | | 61 |
| **33** | F | H | H | H | 164.9 | 165.2 | 138 |
| **34** | H | CH₂OH | H | H | N/A | | 172 |

### Preparation 19

### 4-[4-Hydroxy-3-(trifluoromethyl)phenyl]but-3-en-2-one

¹H-NMR (CDCl₃): 2.29 - 2.31 (3H), 6.60 - 6.64 (1H), 6.99 - 7.01 (1H), 7.43 - 7.46 (1H), 7.59 - 7.61 (1H), 7.70-7.72 (1H)

### Preparation 22

### 4-hydroxy-3-[3-oxobut-1-en-1-yl]benzonitrile

¹H-NMR (*d₆*DMSO): 2.31 - 2.33 (3H), 6.97 - 7.10 (2H), 7.60 - 7.70 (2H), 8.09 - 8.11 (1H)

### Preparation 23

### 4-(2-Hydroxy-3-methylphenyl)but-3-en-2-one

¹H-NMR (*d₆*-DMSO): 2.14 - 2.20 (3H), 2.25 - 2.32 (3H), 6.69 - 6.80 (2H), 7.11 - 7.17 (1H), 7.41 - 7.47 (1H), 7.88 - 7.94 (1H)

### Preparation 24

### 4-(2-Methoxyphenyl)but-3-en-2-one

¹H-NMR (*d₆*-DMSO): 2.28 - 2.30 (3H), 3.83 - 3.86 (3H), 6.78 - 6.84 (1H), 6.95 - 7.00 (1H), 7.05 - 7.09 (1H), 7.37 - 7.43 (1H), 7.64 - 7.69 (1H), 7.74 - 7.80 (1H)

### Preparation 25

### 4-(2-Hydroxy-5-methoxyphenyl)but-3-en-2-one

¹H-NMR (*d₆*-DMSO): 2.27 - 2.29 (3H), 3.68 - 3.71 (3H), 6.80 - 6.86 (3H), 7.11 - 7.15 (1H), 7.72 - 7.78 (1H)

### Preparation 26

### 4-(4-Fluorophenyl)but-3-en-2-one

¹H-NMR (CD₃OD): 2.33 - 2.34 (3H), 7.10 - 7.15 (3H), 7.61 - 7.68 (3H)

### Preparation 27

### 4-(4-Fluoro-2-hydroxyphenyl)but-3-en-2-one

¹H-NMR (CD₃OD): 2.37 - 2.39 (3H), 6.56 - 6.64 (2H), 6.79 - 6.82 (1H), 7.55 - 7.60 (1H), 7.82 - 7.85 (1H)

### Preparation 32

### 4-[2-Hydroxy-5-(trifluoromethyl)phenyl]but-3-en-2-one

¹H-NMR (CDCl₃): 2.44 - 2.47 (3H), 7.02 - 7.06 (1H), 7.13 - 7.19 (1H), 7.47 - 7.52 (1H), 7.70 - 7.73 (1H), 7.78-7.84 (1H)

### Preparation 35

### 4-(4-Nitrophenyl)but-3-en-2-one

To a solution of the compound of Preparation 145 (2.0 g, 13.2 mmol) in tetrahydrofuran (100 ml) was added the compound of Preparation 150 (8.4 g, 26.5 mmol). The reaction mixture was heated under reflux for 3 h and then cooled and concentrated *in vacuo.* The residue was triturated with diethyl ether and then loaded onto a silica plug and eluted with diethyl ether. The appropriate fractions were combined and concentrated and the residue was further purified using a silica plug, eluting with diethyl ether. The appropriate fractions were combined and concentrated to give the title compound (2.4 g). ¹H-NMR (CDCl₃): 2.39 - 2.41 (3H), 6.78 - 6.80 (1H), 7.48 - 7.50 (1H). 7.65 - 7.67 (2H), 8.20 - 8.22

### Similarly prepared were:

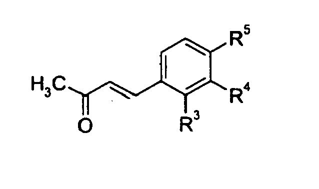

| Preparation | R³ | R⁴ | R⁵ | MH⁺ exper | MH⁺ expected | From the compound of preparation |
|---|---|---|---|---|---|---|
| **36** | CH₃ | H | H | 160.9 | 161.1 | 115 |
| **37** | H | NO₂ | H | N/A | | 146 |
| **38** | NO₂ | H | H | N/A | | 151 |
| **39** | H | H | CO₂H | N/A | | 153 |
| **40** | H | CO₂H | H | N/A | | 154 |

### Preparation 37

### 4-(3-Nitrophenyl)but-3-en-2-one

¹H-NMR (CDCl₃): 2.39 - 2.41 (3H), 6.78 - 6.82 (1H). 7.56 - 7.59 (1H). 7.66 - 7.68 (1H). 7.80 - 7.82 (1H), 8.18-8.21 (1H), 8.38-8.40 (1H)

### Preparation 38

### 4-(2-Nitrophenyl)but-3-en-2-one

¹H-NMR (CDCl₃): 2.40 - 2.41 (3H), 6.56 - 6.60 (1H), 7.42 - 7.46 (1H), 7.57 - 7.60 (1H). 7.62 - 7.65 (1H), 7.97 - 8.00 (1H), 8.02 - 8.04 (1H)

### Preparation 39

### 4-[3-Oxobut-1-en-1-yl]benzoic acid

¹H-NMR (*d₆*-DMSO): 2.17 - 2.19 (3H), 6.85 - 6.88 (1H), 7.61 - 7.64 (1H), 7.79 - 7.81 (2H), 7.91 - 7.93 (2H)

### Preparation 40

### 3-[3-Oxobut-1-en-1-yl]benzoic acid

¹H-NMR (CD₃OD): 2.39 - 2.41 (3H), 6.80 - 6.83 (1H), 7.51 - 7.54 (2H), 7.66 - 7.69 (1H), 7.85 - 7.87 (1H), 8.06 - 8.08 (1H), 8.23 - 8.25 (1H)

### Preparation 41

### 4-(2-Hydroxyphenyl)butan-2-one

A suspension of palladium (5% on alumina, 5.0 g, 47.0 mmol) in ethyl acetate (500 ml) was stirred under hydrogen (60 psi) for 40 min. To this suspension was added the compound of Preparation 100 (50.0 g, 308 mmol) and the reaction mixture was hydrogenated at 15 psi for 2 h. The mixture was filtered through Celite® and the filtrate was concentrated *in vacuo.* The residue was purified by column chromatography (silica, 1 kg), eluting with dichloromethane. The appropriate fractions were combined and concentrated and the residue was triturated with diethyl ether/pentane to give the title compound PF-01896702-00 (10.3 g).
¹H-NMR (CDCl₃): 2.18 - 2.19 (3H), 2.80 - 2.95 (4H), 6.75 - 6.85 (2H), 7.00 - 7.11 (2H)

### Preparation 42

### 4-(5-Fluoro-2-hydroxyphenyl)butan-2-one

A mixture of the compound of Preparation 17 (2.1 g, 11.4 mmol) and palladium (2 wt. % on strontium carbonate, 800 mg) in ethyl acetate (40 ml) was stirred under hydrogen (1 atm) at room temperature for 18 h. The mixture was filtered through Arbocel® and the filtrate was concentrated *in vacuo.* The residue was dissolved in ethyl acetate : cyclohexane [20:80, 20 ml] and purified by automated flash chromatography (Biotage™, 40M cartridge) with gradient elution, ethyl acetate : cyclohexane [20:80 to 30:70]. The appropriate fractions were combined and concentrated to give the title compound (1.2 g). ¹H-NMR (CD₃OD): 1.46 - 1 :51 (3H), 1.70 - 1.80 (1H), 1.90 - 2.00 (1H), 2.58 - 2.62 (1H), 2.85 - 2.95 (1H), 6.72 - 6.80 (3H)

### Preparation 43

### 4-(2-Methylphenyl)butan-2-one

A mixture of the compound of Preparation 36 (840 mg, 5.2 mmol) and chlorotris(triphenylphosphine) rhodium(l) (242 mg, 0.3 mmol) in ethyl acetate (25 ml) was stirred under hydrogen (5 atm) at 40°C for 60 h. The mixture was concentrated *in vacuo* and to the residue was added ethyl acetate : cyclohexane [1:3, 30 ml]. The solution was loaded on to a silica plug and eluted with ethyl acetate : cyclohexane [1:3, 300 ml]. The filtrate was concentrated *in vacuo* to give the title compound (556 mg).

### The following were prepared by methods similar to Preparations 41 - 43

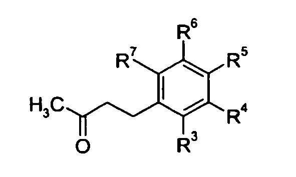

| Preparation | R³ | R⁴ | R⁵ | R⁶ | R⁷ | From the compound of Preparation |
|---|---|---|---|---|---|---|
| **44** | H | H | CF₃ | H | H | 120 |
| **45** | Cl | H | Cl | H | H | 130 |
| **46** | H | CF₃ | H | H | H | 131 |
| **47** | Cl | H | H | H | F | 134 |
| **48** | OH | F | H | F | H | 21 |
| **49** | Cl | OH | H | H | H | 98 |
| **50** | H | H | CO₂H | H | H | 39 |
| **51** | H | CO₂H | H | H | H | 40 |
| **52** | OH | H | F | F | H | 18 |
| **53** | Cl | OH | H | H | Cl | 99 |
| **54** | H | CF₃ | OH | H | H | 19 |

### Preparation 44

### 4-[4-(Trifluoromethyl)phenyl]butan-2-one

¹H-NMR (CD₃OD): 2.08 - 2.12 (3H), 2.79 - 2.84 (2H), 2.87 - 2.92 (2H), 7.34 - 7.38 (1H), 7.49 - 7.54 (1H)

### Preparation 45

### 4-(2,4-Dichlorophenyl)butan-2-one

¹H-NMR (CD₃OD): 2.10 - 2.12 (3H), 2.74 - 2.79 (2H), 2.88 - 2.93 (2H), 7.18 - 7.21 (1H), 7.23 - 7.27 (1H), 7.36 - 7.38 (1H)

### Preparation 46

### 4-[3-(Trifluoromethyphenyl]butan-2-one

¹H-NMR (CDCl₃): 2.14 - 2.16 (3H), 2.76 - 2.82 (2H), 2.92 - 2.98 (2H), 7.35 - 7.41 (2H), 7.42 - 7.48 (2H)

### Preparation 47

### 4-(2-Chloro-6-fluorophenyl)butan-2-one

¹H-NMR (CD₃OD): 2.15 - 2.17 (3H), 2.70 - 2.76 (2H), 2.98 -.3.04 (2H), 7.01 - 7.07 (1H), 7.19 - 7.23 (2H)

### Preparation 49

### 4-(2-Chloro-3-hydroxyphenyl)butan-2-one

¹H-NMR (CDCl₃): 2.11 - 2.13 (3H), 2.70 - 2.73 (2H), 2.95 - 2.99 (2H), 6.78 - 6.80 (1H), 6.87 - 6.89 (1H), 7.05-7.07 (1H)

### Preparation 50

### 4-(3-Oxobutyl)benzoic acid

¹H-NMR (CD₃OD): 2.16 - 2.18 (3H), 2.80 - 2.83 (2H), 2.89 - 2.92 (2H), -7.30 - 7.32 (2H), 7.90 - 7.93 (2H)

### Preparation 51

### 3-(3-oxobutyl)benzoic acid

¹H-NMR (CDCl₃): 2.16 - 2.18 (3H), 2.79 - 2.82 (2H), 2.95 - 2.98 (2H), 7.38 - 7.40 (1H), 7.41 - 7.44 (1H), 7.94 - 7.96 (2H)

### Preparation 52

### 4-(4,5-Difluoro-2-hydroxyphenyl)butan-2-one

¹H-NMR (CDCl₃): 2.20 - 2.22 (3H), 2.74 - 2.77 (2H), 2.87 - 2.89 (2H), 6.70 - 6.73 (1H), 6.79 - 6.82 (1H)

### Preparation 53

### 4-(2,6-Dichloro-3-hydroxyphenyl)butan-2-one

Experimental MH⁺ 233.2; expected 233.0

### Preparation 54

### 4-[4-Hydroxy-3-(trifluoromethyl)phenyl]butan-2-one

¹H-NMR (CDCl₃): 2.17 - 2.19 (3H), 2.76 - 2.79 (2H), 2.81 - 2.83 (2H), 6.80 - 6.82 (1H), 7.18 - 7.21 (1H), 7.29-7.31 (1H)

### Preparation 55

### 4-(4-Hydroxy-3,5-dimethylphenyl)butan-2-one

To a mixture of the compound of Preparation 117 (442 mg, 3.6 mmol) and concentrated sulfuric acid (conc., 0.9 ml) in toluene (4 ml), at 0°C, was added dropwise the compound of Preparation 118 (116 mg, 1.7 mmol) in toluene (2 ml). The reaction mixture was stirred at room temperature for 18 h and then partitioned between water and diethyl ether. The organic phase was separated, washed with water and brine, dried (MgSO₄) and concentrated *in vacuo.* The residue was purified by column chromatography (Isolute cartridge, 5g), with gradient elution, cyclohexane : dichloromethane [3:1 to 0:1]. The appropriate fractions were combined and concentrated to give the title compound (128 mg).
¹H-NMR (CDCl₃): 2.09 - 2.10 (3H), 2.18 - 2.20 (6H), 2.61 - 2.72 (4H), 4.61 - 4.62 (1H), 6.78 - 6.79 (2H)

### Preparation 56

### 4-(3-Fluoro-4-hydroxyphenyl)butan-2-one

To a solution of the compound of Preparation 156 (5.8 g, 51.9 mmol) in toluene (25 ml), at 0°C. was added concentrated sulphuric acid (1.1 ml, 21.6 mmol). To the mixture was added dropwise the compound of Preparation 118 (3.0 g, 43.2 mmol) in toluene (5 ml) over 1.5 h and the reaction mixture was stirred for a further 2 h. To the mixture was added water (25 ml) and the two layers were separated. The organic phase was washed with water (2 x 10 ml), dried (MgSO₄) and concentrated *in vacuo.* The residue was dissolved in ethyl acetate : cyclohexane [10:90, 5 ml] and purified by automated flash chromatography (Biotage™, 40M cartridge) with gradient elution, ethyl acetate : cyclohexane [5:95 to 20:80]. The appropriate fractions were combined and concentrated to give the title compound (747 mg). ¹H-NMR (CD₃OD): 2.10 - 2.12 (3H), 2.75 - 2.78 (4H), 6.77 - 6.80 (2H), 6.84 - 6.86 (1H)

### Similarly prepared were:

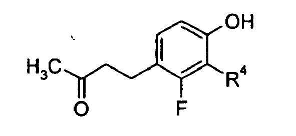

| Preparation | R⁴ | From the compound of Preparation |
|---|---|---|
| 57 | F | 163 |
| 58 | H | 164 |

### Preparation 57

### 4-(2,3-Difluoro-4-hydroxyphenyl)butan-2-one

¹H-NMR (CDCl₃): 2.15 - 2.18 (3H), 2.70 - 2.73 (2H), 2.82 - 2.85 (2H), 6.65 - 6.69 (1H), 6.80 - 6.84 (1H)

### Preparation 58

### 4-(2-Fluoro-4-hydroxyphenyl)butan-2-one

¹H-NMR (CDCl₃): 2.16 - 2.18 (3H), 2.71 - 2.75 (2H), 2.80 - 2.84 (2H), 6.50 - 6.54 (2H), 6.98 - 7.01 (1H)

### Preparation 59

### 3-Formyl-4-hydroxybenzonitrile

To a solution of the compound of Preparation 123 (2.5 g, 13.0 mmol) in anhydrous tetrahydrofuran (100 ml), at -78°C and under nitrogen, was added slowly n-butyllithium (1.6M, 16.2 mll) *via* syringe. The reaction mixture was stirred at -78°C for 30 min, before addition of *N,N*-dimethylformamide (2.1 ml). The reaction mixture was allowed to warm to room temperature overnight and then diluted with water (100 ml). The mixture was concentrated *in vacuo* and the residue was acidified by addition of hydrochloric acid (2M). The precipitate was collected by filtration and dried *in vacuo* to give the title compound (1.13 g).
¹H-NMR (CDCl₃): 7.02 - 7.17 (1H), 7.69 - 7.98 (2H), 9.86 9.97 (1H)

### Preparation 60

### 4_{.}5-Difluoro-2-hydroxvbenzaidehyde

To a solution of the compound of Preparation 132 (5.0 g, 20.0 mmol) in anhydrous tetrahydrofuran (100 ml), at -78°C and under nitrogen, was added n-butyllithium (1.6M in hexanes, 30.7 ml, 49.0 mmol), *via* syringe. The mixture was stirred at -78°C for 30 min, before addition of *N*,*N*-dimethylformamide (3.9 ml, 50 mmol). The reaction mixture was then stirred at room temperature for 18 h. To the mixture was added water (200 ml) and the solution was concentrated *in vacuo*. The residue was acidified with excess hydrochloric acid (2M) and extracted with diethyl ether (2 x 300 ml). The combined extracts were dried (MgSO₄) and concentrated *in vacuo* to give the title compound (3.0 g).
¹H-NMR (CDCl₃): 6.77 - 6.83 (1H), 7.34 - 7.40 (1H), 9.77 - 9.79 (1H)

### Similarly prepared was:

### Preparation 61

### 2-Hydroxy-5-trifluoromethylbenzaldehyde

### From the compound of Preparation 133

¹H-NMR (CDCl₃): 6.87 - 6.93 (1H), 7.09-7.13 (1H), 7.48 - 7.53 (1H), 9.94 - 9.97 (1H)

### Preparation 62

### N-[2-(3-Oxobutyl)phenyl]methanesulfonamide

To a mixture of the compound of Preparation 64 (200 mg, 0.7 mmol), palladium (II) acetate (10 mol%, 15 mg, 0.07 mmol) and lithium chloride (28.5 mg, 0.7 mmol), under nitrogen, was added triethylamine (0.34 ml, 2.4 mmol). The reaction vessel was purged with nitrogen and de-gassed, before addition of the compound of Preparation 139 (0.2 ml, 2.4 mmol). The reaction mixture was then heated in a microwave oven (300W) at 120°C for 20 min. The mixture was concentrated *in vacuo* and to the residue was added water (10 ml) The solution was extracted with ethyl acetate (3 x 25 ml). The combined extracts were washed with hydrochloric acid (0.1M) and aqueous sodium hydrogen carbonate solution, dried (MgSO₄) and concentrated *in vacuo.* The residue was purified by column chromatography (Biotage™ 40S cartridge, conditioned with cyclohexane : ethyl acetate [88:12]) with gradient elution, cyclohexane : ethyl acetate [88:12 to 0:100]. The appropriate fractions were combined and concentrated to give the title compound (160 mg).
¹H-NMR (CDCl₃): 2.12 - 2.15 (3H), 2.84 - 2.93 (4H), 3.02 - 3.06 (3H), 7.12 - 7.18 (1H), 7.19 - 7.25 (1H), 7.45 - 7.49 (1H), 8.24-8.33 (1H)

### Similarly prepared was:

### Preparation 63

### 4-(3-Hydroxyphenyl)butan-2-one

### From the compound of Preparation 165

¹H-NMR (CDCl₃): 2.17 - 2.19 (3H), 2.75 - 2.79 (2H), 2.81 - 2.85 (2H), 6.65 - 6.68 (2H), 6.76 - 6.78 (1H), 7.13-7.16 (1H)

### Preparation 64

### N-(2-Iodophenyl)methanesulfonamide

To a solution of the compound of Preparation 135 (1.0 g, 5.8 mmol) in pyridine (8 ml) was added dropwise the compound of Preparation 174 (0.7 ml, 8.7 mmol) and the reaction mixture was stirred at room temperature for 18 h. The mixture was concentrated *in vacuo* and to the residue was added water. The solution was extracted with dichloromethane and the organic extract was acidified with hydrochloric acid (2M), neutralised with sodium hydrogen carbonate, dried (MgSO₄) and concentrated *in vacuo.* To the residue was added dichloromethane (4 ml) and the supernatant liquid was removed and layered with cyclohexane from which a solid crystallised overnight. The crystalline solid was filtered and dried to give the title compound (1.2 g).
¹H-NMR (CDCl₃): 2.98 - 3.02 (3H), 6.91 - 6.96 (1H), 7.35 - 7.42 (1H), 7.63 - 7.67 (1H), 7.80 - 7.85 (1H)

### Preparation 65

### N-{2-[3-Oxobut-1-en-1-yl]phenyl}methanesulfonamide

To a solution of the compound of Preparation 64 (1.2 g, 3.9 mmol) in *N*,*N*-dimethylformamide (24 ml) was added palladium (II) acetate (10 mol%, 874 mg, 3.9 mmol), the compound of Preparation 118 (0.5 ml, 5.8 mmol), tetrabutylammonium chloride (1.08 g, 3.9 mmol) and sodium hydrogen carbonate (818 mg, 9.7 mmol) . The reaction vessel was purged with nitrogen and the mixture heated at 60°C for 2 h and then stirred at room temperature for 18 h. To the mixture was added water (50 ml) and the solution was extracted with ethyl acetate (4 x 100 ml). The combined extracts were concentrated *in vacuo* to give the crude product. The residue was purified by column chromatography (silica, 20 g, conditioned with dichloromethane) with gradient elution, cyclohexane : ethyl acetate [80:20 to 0:100]. The appropriate fractions were combined and concentrated to give the title compound (514 mg).
Experimental (M-H⁺)⁻ 237.9; expected 238.1

### Preparation 66

### 4-(2-Isopropoxyphenyl)butan-2-one

To a solution of the compound of Preparation 41 (31.7 g, 193 mmol) in acetone (750 ml) was added caesium carbonate (126.0 g, 386.0 mmol) and the compound of Preparation 167 (38.5 ml, 386 mmol). The reaction mixture was heated at 55°C for 18 h, cooled to room temperature and partitioned between water (700 ml) and ethyl acetate (600 ml). The two layers were separated and the aqueous layer was extracted with ethyl acetate (600 ml). The combined organic phases were washed with brine (500 ml) and concentrated *in vacuo* to give the title compound (41.0 g).
¹H-NMR (CDCl₃): 1.23 - 1.30 (6H), 2.09 - 2.10 (3H), 2.61 - 2.68 (2H), 2.90 - 2.98 (2H), 4.50 - 4.60 (1H), 6.78 - 6.82 (2H), 7.09 - 7.15 (2H)

### Preparation 67

### 7-Hydroxy-6-{[1-methyl-3-(4-nitrophenyl)propyl]amino}-4,5,6,7-tetrahydroimidazo[4,5,1-jk][1]benzazepin-2(1H)-one

To a mixture of the compound of Preparation 9 (300 mg, 1.2 mmol) and the compound of Preparation 35 (269 mg, 1.4 mmol) in methanol (5 ml), under nitrogen, was added triethylamine (0.05 ml, 0.4 mmol)). After stirring for 20 min, sodium cyanoborohydride (111 mg, 1.8 mmol) was added and the reaction mixture was heated at 60°C under nitrogen for 5 days. After cooling, citric acid (500 mg) was added and the mixture was heated at 60°C for 3 h. To the mixture was added water (0.2 ml), followed by excess sodium hydrogen carbonate and the mixture was stirred at room temperature for 18 h. The mixture was concentrated *in vacuo* and the residue was pre-absorbed onto silica (10 g) and passed through a silica plug (10 g), eluting with dichloromethane : 2.5% ammonia in methanol [4:1]. The filtrate was concentrated *in vacuo* and the residue was washed with toluene and dried to give the title compound (600 mg) which was used directly.

### Similarly prepared was:

### Preparation 68

### 7-Hydroxy-6-{[1-methyl-3-(3-nitrophenyl)propyl]amino}-4,5,6,7-tetrahydroimidazo[4,5,1-jk][1]benzazepin-2(1H)-one

From the compound of Preparation 37. The title compound was used directly in the next stage of reaction.

### Preparation 69

### 7-Hydroxy-6-{[3-(2-hydroxyphenyl)-1-methylpropyl]amino}-4,5,6,7-tetrahydroimidazo[4,5,1-jk][1]benzazepin-2(1H)-one

To a solution of the compound of Preparation 100 (7.1 g, 43.3 mmol) in methanol (200 ml) was added the compound of Preparation 1 (10.1 g, 39.4 mmol), followed by triethylamine (1.7 ml, 11.8 mmol). After stirring for 20 min, sodium cyanoborohydride (6.2 g, 98.5 mmol) was added and the reaction mixture was stirred at room temperature for 60 h. The mixture was quenched with water (10 ml) and concentrated in *vacuo.* The residue was azeotroped with methanol and the solution was concentrated *in vacuo* to give the title compound (22.5 g) as a mixture of 4 diastereoisomers
Experimental MH⁺ 368.1; expected 368.2

### Preparation 70

### 4-(3,5-Dibromo-2-hydroxyphenyl)butan-2-one

To a solution of the compound of Preparation 41 (2.0 g, 12.2 mmol) in dichloromethane (70 ml) was added dropwise the compound of Preparation 148 (4.3 g, 24.4 mmol) in *N*,*N*-dimethylformamide (8 ml). The reaction mixture was stirred at room temperature for 18 h, before addition of dichloromethane (100 ml). The solution was washed with water (3 x 100 ml) and concentrated under a stream of nitrogen. The residue was dissolved in ethyl acetate : cyclohexane [5:95] and purified by automated flash chromatography (Biotage™, 65i silica cartridge) with gradient elution, ethyl acetate : cyclohexane [2:98 to 20:80]. The appropriate fractions were combined and concentrated to give the title compound (3.7 g). ¹H-NMR (CDCl₃): 2.18 - 2.20 (3H), 2.77 - 2.80 (2H), 2.88 - 2.91 (2H), 7.15 - 7.17 (1H), 7.20 - 7.22 (1H)

### Preparation 71

### 2,2.2-Trifluoro-N-[2-(3-oxobutyl)phenyl]acetamide

To a solution of the compound of Preparation 78 (400 mg, 1.3 mmol) in methanol (10 ml) was added concentrated hydrochloric acid (0.1 ml) and the reaction mixture was stirred at room temperature for 2 h. The mixture was concentrated *in vacuo* and to the residue was added dichloromethane (15 ml). The solution was washed with water (2 x 5 ml), dried (MgSO₄) and concentrated *in vacuo* to give the title compound (250 mg).
¹H-NMR (CDCl₃): 2.16 - 2.19 (3H), 2.78 - 2.82 (2H), 2.97 - 3.00 (2H), 7.17 - 7.20 (2H), 7.60 - 7.65 (2H)

### Similarly prepared were:

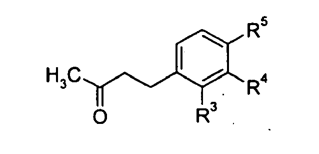

| Preparation | R³ | R⁴ | R⁵ | From the compound of Preparation |
|---|---|---|---|---|
| **72** | -NHSO₂Et | H | H | 93 |
| **73** | H | -NHCOCF₃ | H | 79 |
| **74** | H | H | -NHCOCF₃ | 80 |
| **75** | H | -NHCOEt | H | 81 |
| **76** | -NHCOCH₃ | H | H | 82 |
| **77** | H | -NHSO₂CH₃ | H | 94 |

### Preparation 72

### N-[2-(3-Oxobutyl)phenyl]ethanesulfonamide

¹H-NMR (CDCl₃): 1.40 - 1.44 (3H), 2.12 - 2.14 (3H), 2.86 - 2.89 (4H), 3.17 - 3.21 (2H), 7.11 - 7.16 (2H), 7.18 - 7.21 (1H), 7.41 - 7.43 (1H)

### Preparation 73

### 2,2,2-Trifluoro-N-[3-(3-oxobutyl)phenyl]acetamide

¹H-NMR (CDCl₃): 2.13 - 2.15 (3H), 2.76 - 2.80 (2H), 2.87 - 2.91 (2H), 7.04 - 7.06 (1H), 7.27 - 7.30 (1H), 7.39 - 7.43 (2H)

### Preparation 74

### 2,2,2-Trifluoro-N-[4-(3-oxobutyl)phenyl]acetamide

¹H-NMR (CDCl₃): 2.15 - 2.18 (3H), 2.76 - 2.80 (2H), 2.84 - 2.88 (2H), 7.20 - 7.23 (2H), 7.43 - 7.46 (2H)

### Preparation 75

### N-[3-(3-Oxobutyl)phenyl]propanamide

¹H-NMR (CDCl₃): 1.20 - 1.25 (3H), 2.10 - 2.13 (3H), 2.37 - 2.40 (2H), 2.76 - 2.79 (2H), 2.81 - 2.84 (2H), 6.90 - 6.93 (1H), 7.15 - 7.19 (2H ), 7.26 - 7.28 (1H)

### Preparation 76

### N-[2-(3-Oxobutyl)phenyl]acetamide

¹H-NMR (CDCl₃): 2:13 - 2.15 (3H), 2.28 - 2.30 (3H), 2.80 - 2.83 (2H), 2.89 - 2.92 (2H), 7.03 - 7.07 (1H), 7.19 - 7.22 (1H), 7.43 - 7.46 (1H), 7.62 - 7.64 (1H)

### Preparation 77

### N-[3-(3-Oxobutvl)phenyl]methanesulfonamide

¹H-NMR (CDCl₃): 2.16 - 2.18 (3H), 2.76 - 2.79 (2H), 2.84 - 2.87 (2H), 2.99 - 3.01 (3H), 7.00 - 7.05 (3H), 7.22 - 7.25 (1H)

### Preparation 78

### 2,2,2-Trifluoro-N-{2-[2-(2-methyl-1,3-dioxolan-2-yl)ethyl]phenyl}acetamide

To a solution of the compound of Preparation 83 (350 mg, 1.7 mmol) in dichloromethane (10 ml) was added pyridine (534 mg, 6.8 mmol) and the mixture was cooled in an acetonitrile/dry ice bath. To the mixture was added dropwise the compound of Preparation 173 (0.3 ml, 1.9 mmol) in dichloromethane (5 ml) and the reaction mixture was stirred for 10 min. The reaction mixture was allowed to warm to room temperature and stirred for 2 h, before addition of excess sodium hydrogen carbonate. After stirring for 18 h, water (5 ml) and dichloromethane (10 ml) were added and the two layers were separated. The organic phase was concentrated *in vacuo* to give the title compound (400 mg).
¹H-NMR (CDCl₃): 1.20 - 1.23 (3H), 1.94 - 1.98 (2H), 2.68 - 2.72 (2H), 3.94 - 4.00 (4H), 7.20 - 7.25 (2H), 7.40 - 7.45 (2H)

### Similarly prepared, using the appropriate acid chloride or acid anhydride, were:

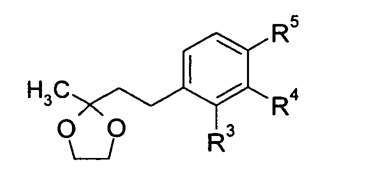

| Preparation | R³ | R⁴ | R⁵ | From the compound of Preparation |
|---|---|---|---|---|
| **79** | H | -NHCOCF₃ | H | 84 |
| **80** | H | H | -NHCOCF₃ | 85 |
| **81** | H | -NHCOEt | H | 84 and 158 |
| **82** | -NHCOCH₃ | H | H | 83 and 159 |

### Preparation 79

### 2,2,2-Trifluoro-N-{3-[2-(2-methyl-1,3-dioxolan-2-yl)ethyl]phenyl}acetamide

¹H-NMR (CDCl₃): 1.37 - 1.39 (3H), 1.95 - 1.99 (2H), 2.69 - 2.73 (2H), 3.96 - 4.00 (4H), 7.26 - 7.29 (1H), 6.38 - 6.42 (2H), 8.60 - 8.62 (1H)

### Preparation 80

### 2.2,2-Trifluoro-N-{4-[2-(2-methyl-1,3-dioxolan-2-yl)ethyl]phenyl}acetamid=H-NMR (CDCl₃):

1.37 - 1.39 (3H), 1.90 - 1.94 (2H), 2.68 - 2.73 (2H), 3.96 - 4.00 (4H), 7.19 - 7.22 (2H), 7.44-7.47 (2H)

### Preparation 81

### N-{3-[2-(2-Methyl-1,3-dioxolan-2-yl)ethyl]phenyl}propanamide

¹H-NMR (CDCl₃): 1.08 - 1.12 (3H), 1.38 - 1.40 (3H), 1.95 - 1.99 (2H)_{;} 2.36 - 2.40 (2H), 2.65 - 2.70 (2H), 3.90 - 3.96 (4H), 6.91 - 6.94 (1H), 7.10 - 7.15 (1H), 7.22 - 7.28 (2H)

### Preparation 82

### N-{2-[2-(2-Methyl-1,3-dioxolan-2-yl)ethyl]phenyl}acetamide

¹H-NMR (CDCl₃): 1.36 - 1.38 (3H), 1.94 - 1.98 (2H), 2.09 - 2.11 (3H), 2.60 - 2.64 (2H), 3.90 - 3.94 (2H), 4.00 - 4.03 (2H ), 7.01 - 7.04 (1H), 7.10 - 7.13 (1H), 7.42 - 7.45 (1H), 7.61 - 7.64 (1H)

### Preparation 83

### 2-[2-(2-Methyl-1,3-dioxolan-2-yl)ethyl]aniline

A solution of the compound of Preparation 86 (550 mg, 2.3 mmol) in methanol (75 ml) was flowed through an H-Cube hydrogenator (1 ml/min, 1 atm, room temperature) using a palladium catalyst (10% on carbon). The solution was concentrated *in vacuo* to give the title compound (500 mg).
¹H-NMR (CDCl₃): 1.38 - 1.40 (3H), 1.92 - 1.97 (2H), 2.58 - 2.62 (2H), 4.00 - 4.04 (4H), 6.67 - 6.73 (2H), 7.01 - 7.04 (2H)

### Similarly prepared were:

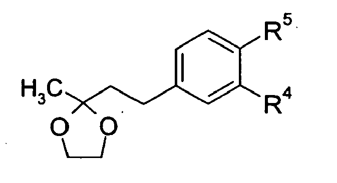

| Preparation | R⁴ | R⁵ | From the compound of Preparation |
|---|---|---|---|
| **84** | NH₂ | H | 87 |
| **85** | H | NH₂ | 88 |

### Preparation 84

### 3-[2-(2-Methyl-1,3-dioxolan-2-yl)ethyl]aniline

¹H-NMR (CDCl₃): 1.37 - 1.39 (3H), 1.90 - 1.94 (2H), 2.60 - 2.64 (2H), 3.96 - 4.00 (4H), 6.49 - 6.52 (2H), 6.60 - 6.62 (1H), 7.03 - 7.05 (1H)

### Preparation 85

### 4-[2-(2-Methyl-1,3-dioxolan-2-yl)ethyl]aniline

¹H-NMR (CDCl₃): 1.37 - 1.39 (3H), 1.88 - 1.92 (2H), 2.59 - 2.63 (2H), 3.97 - 4.00 (4H), 6.60 - 6.63 (2H), 6.98 - 7.00 (2H)

### Preparation 86

### 2-Methyl-2-[2-(2-nitrophenyl)vinyl]-1.3-dioxolane

A mixture of the compound of Preparation 38 (2.5 g, 13.1 mmol), ethylene glycol (1.6 g, 26.2 mmol) and p-toluenesulphonic acid monohydrate (25 mg, 0.1 mmol) in toluene (50 ml) was heated under reflux in a Dean Stark apparatus for 5 h. After cooling, the mixture was washed with saturated aqueous sodium carbonate solution (2 x 20 ml) and water, dried (K₂CO₃) and concentrated *in vacuo* to give the title compound (2.2 g).
¹H-NMR (CDCl₃): 2.58 - 2.61 (3H), 4.00 - 4.05 (4H), 6.05 - 6.09 (1H), 7.14 - 7.18 (1H), 7.55 - 7.57 (1H), 7.64 - 7.68 (2H), 7.95 - 7.97 (1H)

### Similarly prepared were:

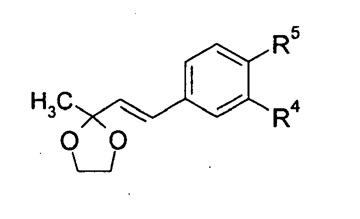

| Preparation | R⁴ | R⁵ | From the compound of Preparation |
|---|---|---|---|
| **87** | NO₂ | H | 37 |
| **88** | H | NO₂ | 35 |

### Preparation 87

### 2-Methyl-2-[2-(3-nitrophenyl)vinyl]-1,3-dioxolane

¹H-NMR (CDCl₃): 1.58 - 1.60 (3H), 3.92 - 3.97 (2H), 4.00 - 4.04 (2H), 6.26 - 6.30 (1H), 6.76 - 6.80 (1H), 7.48-7.51 (1H), 7.65 - 7.68 (1H), 8.08-8.11 (1H), 8.22 - 8.24 (1H)

### Preparation 88

### 2-Methyl-2-[2-(4-nitrophenyl)vinyl]-1,3-dioxolane

¹H-NMR (CDCl₃): 2.59 - 2.61 (3H), 3.95 - 3.99 (2H), 4.00 - 4.05 (2H), 6.30 - 6.34 (1H), 6.76 - 6.80 (1H), 7.52 - 7.55 (2H), 8.17 - 8.20 (2H)

### Preparation 89

### 4-(3-Oxobutyl)-N-(2,2,2-trifluoroethyl)benzamide

To a solution of the compound of Preparation 152 (0.6 ml, 7.0 mmol) in dichloromethane (15 ml) was added the compound of Preparation 91 (367 mg, 1.7 mmol). The reaction mixture was stirred for 15 min and then extracted with water (2 x 2ml). The organic phase was dried (MgSO₄) and concentrated in *vacuo* to give the title compound (670 mg).
¹H-NMR (CDCl₃): 4.05 - 4.11 (1H), 7.25 - 7.30 (2H), 7.69 - 7.75 (2H)

### Similarly prepared was:

### Preparation 90

### N-Methyl-3-(3-oxobutyl)benzamide from the compound of Preparation 92 and Preparation 175.

¹H-NMR (CDCl₃): 2.10 - 2.12 (3H), 2.75 - 2.79 (2H), 2.85 - 2.89 (2H), 2.96 - 2.98 (3H), 7.26 - 7.29 (2H), 7.54- 7.57 (1H), 7.60- 7.62 (1H)

### Preparation 91

### 4-(3-Oxobutyl)benzoyl chloride

To a solution of the compound of Preparation 50 (1.2 g, 6.2 mmol) in dichloromethane (15 ml) was added *N*,*N*-dimethylformamide (30 µl). The mixture was cooled to 0°C and the compound of Preparation 147 (0.9 ml) was added. The reaction mixture was allowed to warm to room temperature and stirred for 18 h. The mixture was concentrated *in vacuo* to give the title compound (1.3 g), which was used directly.

### Similarly prepared was:

### Preparation 92

### 3-(3-Oxobutyl)benzoyl chloride from the compound of Preparation 51 and the compound of Preparation 147

### Preparation 93

### N-{2-[2-(2-Methyl-1,3-dioxolan-2-yl)ethyl]phenyl}ethanesulfonamide

To a solution of the compound of Preparation 83 (350 mg, 1.7 mmol) in dichloromethane (5 ml) was added pyridine (0.4 ml, 5.1 mmol) and the mixture was cooled in an ice bath. To the mixture was added dropwise the compound of Preparation 155 (0.2 ml, 1.9 mmol) in dichloromethane (5 ml). After stirring for 10 min, the reaction mixture was allowed to warm to room temperature and stirred for a further 2 h. Excess sodium hydrogen carbonate was added and the mixture was stirred for 18 h, before addition of water (5 ml) and dichloromethane (10 ml). The two layers were separated and the organic phase was concentrated *in vacuo* to give the title compound (400 mg), which was used directly.

### Similarly prepared was: .

### Preparation 94

### N-{3-[2-(2-Methyl-1,3-dioxolan-2-yl)ethyl]phenyl}methanesulfonamide from the compound of Preparation 84 and the compound of Preparation 174

### Preparation 95

### N-Cyclopropyl-4-(3-oxobutyl)benzamide

To a solution of the compound of Preparation 50 (200 mg, 1.0 mmol) in acetonitrile (10 ml) was added thionyl chloride (151 µl, 2.1 mmol) and the mixture was stirred at room temperature for 18 h. To the mixture was added the compound of Preparation 160 (511 µl, 7.3 mmol) and the reaction mixture was stirred at room temperature for 30 min. The mixture was extracted with ethyl acetate and the combined extracts were washed with aqueous sodium hydrogen carbonate solution and water, dried (MgSO₄) and concentrated *in vacuo* to give the title compound (65 mg).
¹H-NMR (CDCl₃): 0.59 - 0.64 (2H), 0.82 - 0.87 (2H), 2.15 - 2.17 (3H), 2.70 - 2.74 (2H), 2.88 - 2.95 (3H), 7.20 - 7.23 (2H), 7.61 - 7.63 (2H)

### Preparation 96

### 3-(3-Oxobutyl)benzamide

A solution of the compound of Preparation 92 (307 mg, 1.5 mmol) in ammonia (0.5M in dioxane, 17.5 ml) was stirred for 15 min. The mixture was extracted with water (2 x 2 ml) and the organic phase was dried (MgSO₄) and concentrated *in vacuo* to give the title compound (130 mg).
¹H-NMR (CDCl₃): 2.14 - 2.16 (3H), 2.78 - 2.82 (2H), 2.95 - 2.99 (2H), 7.38 - 7.40 (1H), 7.41 - 7.43 (1H), 7.90 - 7.92 (2H)

### Similarly prepared was:

### Preparation 97

### 4-(3-Oxobutyl)benzamide from the compound of Preparation 91.

¹H-NMR (CDCl₃): 2.16 - 2.18 (3H), 2.78 - 2.81 (2H), 2.97 - 3.00 (2H), 7.30 - 7.32 (2H), 8.00 - 8.02 (2H)

### Preparation 98

### 4-(2-Chloro-3-hydroxy-phenyl)-but-3-en-2-one

A mixture of the compound of Preparation 149 (157 mg, 1.0 mmol) and the compound of Preparation 150 (637 mg, 2.0 mmol) in tetrahydrofuran (20 ml) was heated under reflux for 12 h. The mixture was concentrated *in vacuo* and the residue was purified by column chromatography (silica), eluting with ethyl acetate : hexane. The appropriate fractions were combined and concentrated to give the title compound (100 mg).
¹H-NMR (CDCl₃): 2.40 - 2.42 (3H), 6.61 - 6.64 (1H), 7.08 - 7.10 (1H), 7.40 - 7.45 (2H), 7.80 - 7.82 (1H)

### Similarly prepared was:

### Preparation 99

### 4-(2,6-Dichloro-3-hydroxy-phenyl)-but-3-en-2-one from Preparation 169

Experimental MH⁺ 233.2; expected 233.0

### Preparation 100

### 4-(2-hydroxyphenyl)but-3-en-2-one

To a solution of the compound of Preparation 109 (150.0 g, 1.2 mol) in acetone (700 ml), at 0°C and under nitrogen, was added carefully aqueous sodium hydroxide solution (1N, 1350 ml), ensuring the temperature of the mixture remained below 10°C. The reaction mixture was allowed to warm to room temperature and stirred for 18. h. To the mixture was added hydrochloric acid (3N, 600 ml) and the resulting solid was collected by filtration under nitrogen. The solid was washed with water (3 x 300 ml and 1 x 500 ml), followed by cyclohexane (250 ml) and dried *in vacuo.* The residue was slurried in cyclohexane (750 ml) for 2 days, filtered, washed with cyclohexane (2 x 150 ml) and concentrated in *vacuo* to give the title compound (155.0 g).
¹H-NMR (CD₃OD): 2.37 - 2.39 (3H), 6.81 - 6.83 (1H), 6.83 - 6.86 (2H), 7.20 - 7.23 (1H), 7.51 - 7.53 (1H), 7.93-7.96 (1H)

### Preparation 101

### N-[4-(3-Oxobutyl)phenyl]benzenesulfonamide

To a mixture of the compound of Preparation 103 (163 mg, 1.0 mmol) and triethylamine (35 µl, 2.5 mmol) in tetrahydrofuran and dichloromethane (8.5 ml) was added the compound of Preparation 161 (194 mg, 1.1 mmol). The reaction mixture was heated under reflux for 12 h and then concentrated *in vacuo.* The residue was extracted with ethyl acetate and the combined extracts were dried (MgSO₄) and concentrated *in vacuo.* The residue was purified by column chromatography (silica), eluting with ethyl acetate : hexane. The appropriate fractions were combined and concentrated to give the title compound (250 mg).
Experimental MH⁺ 304.2; expected 304.1

### Preparation 102

### 1,1,1-Trifluoro-N-[4-(3-oxobutyl)phenyl]methanesulfonamide

To a mixture of the compound of Preparation 103 (163 mg, 1.0 mmol) and triethylamine (35 µl, 2.5 mmol) in dichloromethane (8.5 ml) was added the compound of Preparation 162 (185 mg, 1.1 mmol). The reaction mixture was stirred at room temperature for 12 h and then concentrated *in vacuo.* To the residue was added methanol and aqueous sodium hydroxide solution and the mixture was stirred for a further 12 h. The mixture was concentrated *in vacuo* and the residue was extracted with ethyl acetate. The combined extracts were dried (MgSO₄) and concentrated *in vacuo.* The residue was purified by column chromatography (silica), eluting with ethyl acetate : hexane. The appropriate fractions were combined and concentrated to give the title compound (103 mg).
¹H-NMR (*d*₆-DMSO): 2.08 - 2.10 (3H), 2.72 - 2.75 (4H), 7.10 - 7.13 (2H), 6.40 - 6.42 (2H)

### Preparation 103

### 4-(4-Amino-phenyl)-butan-2-one

A mixture of the compound of Preparation 100 (150 mg, 0.8 mmol) and palladium (10% on carbon, 15 mg) in ethanol (5 ml) was stirred under hydrogen (50 psi) at room temperature for 2 h. The reaction mixture was filtered through Celite® and the filtrate was concentrated *in vacuo.* The residue was purified by column chromatography (silica), eluting with ethyl acetate : hexane. The appropriate fractions were combined and concentrated to give the title compound (114 mg).
¹H-NMR (*d*₆-DMSO): 2.05 - 2.07 (3H), 2.56 - 2.60 (2H), 2.60 - 2.64 (2H), 6.42 - 6.44 (2H), 6.80 - 6.82 (2H)

### Preparation 104

### 4-Isopropoxy-phenylbutanone

To a solution of the compound of Preparation 106 (1.00 g, 6.1 mmol) in acetone (30 ml) was added potassium carbonate (1.70 g, 12.2 mmol), the compound of Preparation 143 (1.15 ml, 12.2 mmol) and potassium iodide (2.02 g, 12.2 mmol). The reaction mixture was heated under reflux for 18 h and then quenched by addition of water. The mixture was extracted with ethyl acetate (3 x 50 ml) and the combined extracts were dried (MgSO4) and concentrated in vacuo to give the title compound (1.25 g), which was used directly.

### Preparation 105

### Phenyl 3-methylbut-2-enoate

To a solution of the compound of Preparation 176 (6.7 g, 71 mmol) and the compound of Preparation 144 (8.5 g, 71 mmol) in diethyl ether (150 ml), at 0°C. was added dropwise triethylamine (10.0 ml, 71 mmol). The reaction mixture was stirred for 30 min and then filtered through Celite®, washing through with diethyl ether. The filtrate was washed with water and brine, dried (MgSO₄) and concentrated in *vacuo* to give the title compound (13.5 g).
Experimental MH⁺ 177.3; expected 177.1

### The following compounds may be obtained commercially

| Preparation | | Source Number |
|---|---|---|
| **106** | 4-(4-Hydroxyphenyl)butan-2-one | 1 |
| **107** | (Bromomethyl)cyclopropane | 1 |
| **108** | 3-Chloro-2-hydroxybenzaldehyde | 2 |
| **109** | Salicylaldehyde | 1 |
| **110** | 5-Fluoro-2-hydroxybenzaldehyde | 1 |
| **111** | 2-Hydroxy-3-methoxybenzaldehyde _ | 3 |
| **112** | 4-Phenylbutan-2-one | 1 |
| **113** | 3,5-Difluoro-2-hydroxybenzaldehyde | 1 |
| **114** | 4-(1,3-Benzodioxol-5-yl)butan-2-one | 1 |
| **115** | 2-Methylbenzaldehyde | 1 |
| **116** | 4-Methyl-4-phenylpentan-2-one | 1 |
| **117** | 2,6-Dimethylphenol | 1 |
| **118** | But-3-en-2-one | 1 |
| **119** | 2-Hydroxy-3-methylbenzaldehyde | 1 |
| **120** | 4-[4-(Trifluoromethyl)phenyl]but-3-en-2-one | 2 |
| **121** | 2-Methoxybenzaldehyde | 1 |
| **122** | 2-Hydroxy-5-methoxybenzaldehyde | 1 |
| **123** | 3-Bromo-4-hydroxybenzonitrile | 1 |
| **124** | 4-Fluorobenzaldehyde | 2 |
| **125** | 4-Fluoro-2-hydroxybenzaldehyde | 4 |
| **126** | 3,5-Dichloro-2-hydroxybenzaldehyde | 1 |
| **127** | 4-Chloro-2-hydroxybenzaldehyde | 2 |
| **128** | 3-Chloro-5-fluoro-2-hydroxybenzaldehyde | 4 |
| **129** | 3-Fluoro-2-hydroxybenzaldehyde | 2 |
| **130** | 4-(2,4-Dichlorophenyl)but-3-en-2-one | 1 |
| **131** | 4-[3-(Trifluoromethyl)phenyl]but-3-en-2-one | 2 |
| **132** | 2-Bromo-4,5-difluorophenol | 1 |
| **133** | 2-Bromo-4-trifluoromethyl-phenol | 1 |
| **134** | 4-(2-Chloro-6-fluorophenyl)but-3-en-2-one | 5 |
| **135** | 2-lodoaniline | 2 |
| **136** | 4-(4-Methoxyphenyl)butan-2-one | 1 |
| **137** | 4-(2-Hydroxyphenyl)but-3-en-2-one | 1 |
| **138** | 2-Fluorobenzaldehyde | 2 |
| **139** | But-3-en-2-ol | 1 |
| **140** | Benzene-1,2-diamine | 1 |
| **141** | Ethyl acetoacetate | 1 |
| **142** | Di-tert-butyl dicarbonate | 2 |
| **143** | 2-Bromopropane | 1 |
| **144** | 3-Methylbut-2-enoyl chloride | 1 |
| **145** | 4-Nitro-benzaldehyde | 1 |
| **146** | 3-Nitro-benzaldehyde | 1 |
| **147** | Oxalyl chloride | 1 |
| **148** | *N*-Bromosuccinimide | 1 |
| **149** | 2-Chloro-3-hydroxy-benzaldehyde | 1 |
| **150** | Acetyl methylene triphenyl phosphorane | 1 |
| **151** | 2-Nitro-benzaldehyde | 1 |
| **152** | 2,2,2-Trifluoroethanamine | 6 |
| **153** | 4-Formyl-benzoic acid | 1 |
| **154** | 3-Formyl-benzoic acid | 1 |
| **155** | Ethanesulfonyl chloride | 3 |
| **156** | 2-Fluoro-phenol | 1 |
| **157** | 4-Hydroxy-3-trifluoromethyl-benzaldehyde | 4 |
| **158** | Propionyl chloride | 1 |
| **159** | Acetyl chloride | 1 |
| **160** | Cyclopropylamine | 1 |
| **161** | Benzene sulfonyl chloride | 1 |
| **162** | Trifluofomethanesulfonyl chloride | 1 |
| **163** | 2,3-Difluoro-phenol | 1 |
| **164** | 3-Fluoro-phenol | 1 |
| **165** | 3-lodo-phenol | 1 |
| **166** | Ethyl-4-bromobutyrate | 1 |
| **167** | 2-lodopropane | 1 |

| | | |
|---|---|---|
| Source 1 Sigma-Aldrich, P O Box 14508, St. Louis, MO, 63178, USA Source 2 Fluorochem Ltd., Wesley Street, Old Glossop, Derbyshire, SK13 7RY, UK Source 3 Pfaltz & Bauer, Inc., 172 E. Aurora Street, Waterbury, CT, 06708, USA Source 4 Apollo Scientific Ltd., Whitefield Rd., Bredbury, Stock port, Cheshire, SK6 2QR, UK Source 5 Lancaster Synthesis Ltd., Newgate, White Lund, Morecambe, Lancashire, LA3 3BN, UK Source 6 ASDI Inc, 601 Interchange Blvd., Newark, DE, 19711, USA | | |

### Preparation 168

### 4-(5-Chloro-2-hydroxyphenyl)but-3-en-2-one

This compound was prepared according to the method described in WO-9946266 A1 Example 4.

### Preparation 169

### 2,6-Dichloro-3-hdroxybenzaldehyde

This compound was prepared according to the method described in Synthesis (2004), (12), 2062-2065.

### Preparation 170

### 4-(2,3-Dihydro-1-benzofuran-5-yl)butan-2-one

This compound was prepared according to the method described in WO-0279143 A1 Preparation 140.

### Preparation 171

### 4-(3-Hydroxyphenyl)but-3-en-2-one

This compound was prepared according to the method described in WO-0208188 A1 Example 1 Step 1.

### Preparation 172

### 3-(Hydroxymethyl)benzaldehyde

This compound was prepared according to the method described in Can. J. Chem, 1973, 51, 3756-3764.

### The following compounds may be obtained commercially

| Preparation | | Source Number |
|---|---|---|
| **173** | Trifluoroacetic anhydride | 1 |
| **174** | Methanesulfonyl chloride | 1 |
| **175** | Methylamine | 1 |
| **176** | Phenol | 1 |

| | | |
|---|---|---|
| Source 1 Sigma-Aldrich, P O Box 14508, St. Louis, MO, 63178, USA | | |

## Claims

1. A compound of formula (I) 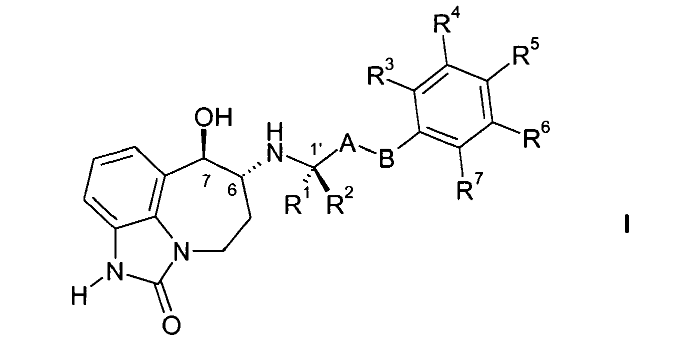 or a pharmaceutically or veterinarily acceptable salt or solvate of said compound, wherein:
A is -CH₂-; and
B is -CH₂-, -C(CH₃)₂-, -O-, -CH₂-CH₂-,CH₂-O-, or -O-CH₂-; or -A-B- is-CH=CH-;
one of R¹ and R² is CH₃ and the other is H;
R³, R⁴, R⁵, R⁶ and R⁷ are each independently selected from H, R⁸ and R⁹; or
R⁴ and R⁵ together are -O-CH₂-CH₂-, -CH₂-CH₂-O- or -O-CH₂-O-, and R³, R⁶ and R⁷ are each independently selected from H, R⁸ and R⁹;
R⁸ is halo, -CN, C₁-C₄ alkyl, C₁-C₄ haloalkyl, -CH₂OH, -O-(C₁-C₄ alkyl), -O-CH₂-(C₃-C₅)cycloalkyl, -CO₂H, -CO₂(C₁-C₄ alkyl), -CONH₂, -CONH(C₁-C₄ alkyl), -CONH(C₁-C₄ haloalkyl), -CONH(C₃-C₆ cycloalkyl) or NH₂; and
R⁹ is -OH, -NHSO₂(C₁-C₃ alkyl), -NHCO(C₁-C₄ alkyl), -NHCO(C₁-C₄ haloalkyl), -NHSO₂(C₁-C₃ haloalkyl) or -NHSO₂(phenyl).

2. A compound according to claim 1, or a pharmaceutically or veterinarily acceptable salt or solvate of said compound, wherein:
-A- is -CH₂- and -B- is -CH₂-, or -C(CH₃)₂-; or
-A-B- is -CH=CH-.

3. A compound according to claim 2, or a pharmaceutically or veterinarily acceptable salt or solvate of said compound, wherein -A- is -CH₂- and -B- is -CH₂-.

4. A compound according to any one of claims 1 to 3, or a pharmaceutically or veterinarily acceptable salt or solvate of said compound, wherein R¹ is H and R² is CH₃.

5. A compound according to claim 4, or a pharmaceutically or veterinarily acceptable salt or solvate of said compound, wherein the absolute stereochemistry at C-1', C-6 and C-7 is *R*, *R*, *R*.

6. A compound according to any one of claims 1 to 5, or a pharmaceutically or veterinarily acceptable salt or solvate of said compound, wherein R³ R⁴, R⁵, R⁶ and R⁷ are each independently selected from H, R⁸ and R⁹, provided that at least two of R³, R⁴, R⁵, R⁶ and R⁷ are H; or
R⁴ and R⁵ together are -O-CH₂-CH₂-, -CH₂-CH₂-O- or -O-CH₂-O-, and R³, R⁶ and R⁷ are H;
R⁸ is halo, -CN, C₁-C₄ alkyl, -CF₃, -CH₂OH, -O-(C₁-C₄ alkyl), or -O-CH₂-(C₃-C₅)cycloalkyl; and
R⁹ is -OH or -NHSO₂(C₁-C₃ alkyl).

7. A compound according to claim 6, or a pharmaceutically or veterinarily acceptable salt or solvate of said compound, wherein one of R³, R⁴, R⁵, R⁶ and R⁷ is R⁸ or R⁹, another two of R³, R⁴, R⁵, R⁶ and R⁷ are H or R⁸, and the other two of R³, R⁴, R⁵, R⁶ and R⁷ are H.

8. A compound according to claim 7, or a pharmaceutically or veterinarily acceptable salt or solvate of said compound, wherein R³ is R⁹.

9. A compound according to claim 8, or a pharmaceutically or veterinarily acceptable salt or solvate of said compound, wherein R⁹ is -OH.

10. A compound according to claim 1 selected from:
(6*R**,7*R**)-7-hydroxy-6-{(1*R**)-[3-(4-hydroxyphenyl)-1-methylpropyl]amino}-4,5,6,7-tetrahydro-imidazo[4,5,1-*jk*][1]benzazepin-2(1*H*)-one
(6*R**,7*R**)-7-hydroxy-6-{(1*S**)-[3-(4-hydroxyphenyl)-1-methylpropyl]amino}-4,5,6,7-tetrahydro-imidazo[4,5,1-*jk*][1]benzazepin-2(1*H*)-one
(6*R*,7*R*)-7-hydroxy-6-{(1R*S*)-[3-(4-hydroxyphenyl)-1-methylpropyl]amino}-4,5,6,7-tetrahydro-imidazo[4,5,1-*jk*][1]benzazepin-2(1*H*)-one
(6*R*,7*R*)-7-hydroxy-6-{(1*S*)-[3-(4-hydroxyphenyl)-1-methylpropyl]amino}-4,5,6,7-tetrahydro-imidazo[4,5,1-*jk*][1]benzazepin-2(1*H*)-one
(6*R*,7*R*)-7-hydroxy-6-{(1*R*)-[3-(4-hydroxyphenyl)-1-methylpropyl]amino}-4,5,6,7-tetrahydro-imidazo[4,5,1-*jk*][1]benzazepin-2(1*H*)-one
(6*R**,7*R**)-7-hydroxy-6-{[(1*R**)-3-(2-hydroxyphenyl)-1-methylpropyl]amino}-4,5,6,7-tetrahydro-imidazo[4,5,1-*jk*][1]benzazepin-2(1*H*)-one
(6*R**,7*R**)-7-hydroxy-6-{[(1*S**)-3-(2-hydroxyphenyl)-1-methylpropyl]amino}-4,5,6,7-tetrahydro-imidazo[4,5,1-*jk*][1]benzazepin-2(1*H*)-one,
(6*R*,7*R*)-7-hydroxy-6-{[(*1RS*)-3-(2-hydroxyphenyl)-1-methylpropyl]amino}-4,5,6,7-tetrahydro-imidazo[4,5,1-*jk*][1]benzazepin-2(1*H*)-one,
(6*R*,7*R*)-7-hydroxy-6-{[(*1R*)-3-(2-hydroxyphenyl)1-methylpropyl]amino}-4,5,6,7-tetrahydro-imidazo[4,5,1-*jk*][1]benzazepin-2(1*H*)-one,
(6*R*,7*R*)-7-hydroxy-6-{[(1*S*)-3-(2-hydroxyphenyl)-1-methylpropyl]amino}-4,5,6,7-tetrahydro-imidazo[4,5,1-*jk*][1]benzazepin-2(1*H*)-one,
(6*R**,7*R**)-6-{[(1*R**)-3-(5-fluoro-2-hydroxyphenyl)-1-methylpropyl]amino}-7-hydroxy-4,5,6,7-tetrahydroimidazo[4,5,1-*jk*][1]benzazepin-2(1*H*)-one,
(6*R**,7*R**)-6-{[(1*S**)-3-(5-fluoro-2-hydroxyphenyl)-1-methylpropyl]amino}-7-hydroxy-4,5,6,7-tetrahydromidazo[4,5,*jk*][1]benzazepin-2(1*H*)-one,
(6*R*,7*R*)-6-{[(1*RS*)-3-(5-fluoro-2-hydroxyphenyl)-1-methylpropyl]amino}-7-hydroxy-4,5,6,7-tetrahydroimidazo[4,5,*jk*][1]benzazepin-2(1*H*)-one,
(6*R*,7*R*)-6-{[(1*R*)-3-(5-fluoro-2-hydroxyphenyl)-1-methylpropyl]amino}-7-hydroxy-4,5,6,7-tetrahydroimidazo[4,5,1-*jk*][1]benzazepin-2(1*H*)-one,
(6*R*,7*R*)-6-{[(1*S*)-3-(5-fluoro-2-hydroxyphenyl)-1-methylpropyl]amino}-7-hydroxy-4,5,6,7-tetrahydroimidazo[4,5,1-*jk*][1]benzazepin-2(1*H*)-one,
(6*R**,7*R**)-6-{[(1*R**)-3-(4,5-difluoro-2-hydroxyphenyl)1-methylpropyl]amino}-7-hydroxy-4,5,6,7-tetrahydroimidazo[4,5,1-*jk*][1]benzazepin-2(1*H*)-one,
(6*R**,7*R**)-6-{[(1*S**)-3-(4,5-difluoro-2-hydroxyphenyl)-1-methylpropyl]amino}-7-hydroxy-4,5,6,7-tetrahydroimidazo[4,5,1-*jk*][1]benzazepin-2(1*H*)-one,
(6*R*,7*R*)-6-{[(*1RS*)-3-(4,5-difluoro-2-hydroxyphenyl)-1-methylpropyl]amino}-7-hydroxy-4,5,6,7-tetrahydroimidazo[4,5,1-*jk*][1]benzazepin-2(1*H*)-one,
(6*R*,7*R*)-6-{[(*1R*)-3-(4,5-difluoro-2-hydroxyphenyl)-1-methylpropyl]amino}-7-hydroxy-4,5,6,7-tetrahydroimidazo[4,5,1-*jk*][1]benzazepin-2(1*H*)-one, and
(6*R*,7*R*)-6-{[(*1S*)-3-(4,5-difluoro-2-hydroxyphenyl)1-methylpropyl]amino}-7-hydroxy-4,5,6,7-tetrahydroimidazo[4,5,1-*jk*][1]benzazepin-2(1*H*)-one,
or a pharmaceutically or veterinarily acceptable salt or solvate of said compound.

11. A feed additive for a livestock animal comprising a compound according to any one of claims 1 to 10, or a pharmaceutically or veterinarily acceptable salt or solvate of said compound.

12. A compound according to any one of claims 1 to 10, or a pharmaceutically or veterinarily acceptable salt or solvate of said compound, for use as a medicament.

13. A pharmaceutical composition comprising a compound according to any one of claims 1 to 10, or a pharmaceutically or veterinarily acceptable salt or solvate of said compound, and a pharmaceutically acceptable carrier.

14. Use of a compound of formula (I) according to any one of claims 1 to 10, or a pharmaceutically or veterinarily acceptable salt or solvate of said compound, for the manufacture of a medicament for the treatment of diseases, disorders, and conditions in which the beta-2 receptor is involved.

## Patentansprüche

1. Verbindung der Formel. (I) 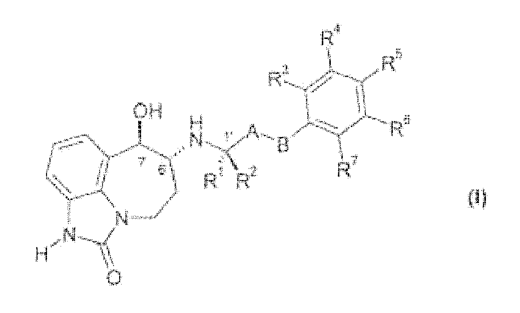 oder ein pharmazeutisch oder veterinär verträgliches Salz oder Solvat der Verbindung, worin:
A -CH₂- ist; und
B -CH₂-, -C(CH₃)₂-, -O-, -CH₂-CH₂-, -CH₂-O oder -O-CH₂ ist oder -A-B- -CH=CH- ist;
eines von R¹ und R² CH₃ ist und das andere H ist;
R³, R⁴, R⁵, R⁶ und R⁷ jeweils unabhängig ausgewählt sind aus H, R⁸ und R⁹ oder
R⁴ und R⁵ zusammen -O-CH₂-CH₂-, -CH₂-CH₂-O- oder -O-CH₂-O- sind und R³, R⁶ und R⁷ jeweils unabhängig ausgewählt sind aus H, R⁸ und R⁹;
R⁸ Halogen, -CN, C₁-C₄-Alkyl, C₁-C₄-Halogenalkyl, -CH₂OH, -O-(C₁-C₄-Alkyl) , -O-CH₂- (C₃-C₅) Cycloalkyl, -CO₂H, -CO₂(-C₁-C₄-Alkyl), -CONH₂, -CONH (C₁-C₄-Alkyl) , -CONH (C₁-C₄-Halogenalkyl), -CONH(C₃-C₆-Cycloalkyl) oder NH₂ ist und
R⁹ -OH, -NHSO₂(C₁-C₃-Alkyl), -NHCO(C₁-C₄-Alkyl), NHCO(C₁-C₄-Halogenalkyl), -NHSO₂ (C₁-C₃-Halogenalkyl) oder -NHSO₂(Phenyl) ist.

2. Verbindung gemäß Anspruch 1 oder pharmazeutisch oder veterinär verträgliches Salz oder Solvat der Verbindung, wobei: -A- -CH₂- ist und -B- -CH₂- oder -C(CH₃)₂- ist oder -A-B- -CH=CH- ist.

3. Verbindung gemäß Anspruch 2 oder ein pharmazeutisch oder veterinär verträgliches Salz oder Solvat der Verbindung, wobei -A- -CH₂- ist und -B- -CH₂- ist.

4. Verbindung gemäß einem der Ansprüche 1 bis 3 oder ein pharmazeutisch oder veterinär verträgliches Salz oder Solvat der Verbindung, wobei R¹ H ist und R² CH₃ ist.

5. Verbindung gemäß Anspruch 4 oder ein pharmazeutisch oder veterinär verträgliches Salz oder Solvat der Verbindung, wobei die absolute Stereochemie an C-1', C-6 und C-7 R,R,R ist.

6. Verbindung gemäß einem der Ansprüche 1 bis 5 oder ein pharmazeutisch oder veterinär verträgliches Salz oder Solvat der Verbindung, wobei R³, R⁴, R⁵, R⁶ und R⁷ jeweils unabhängig ausgewählt sind aus H, R⁸ und R⁹, mit der Maßgabe, dass wenigstens zwei von R³, R⁴, R⁵, R⁶ und R⁷ H sind oder
R⁴ und R⁵ zusammen -O-CH₂-CH₂-, -CH₂-CH₂-O- oder -O-CH₂-O- sind und R³, R⁶ und R⁷ H sind;
R⁸ Halogen, -CN, C₁-C₄-Alkyl, -CF₃, -CH₂OH, -O-(C₁-C₄-Alkyl) oder -O-CH₂-(C₃-C₅)Cycloalkyl ist und
R⁹ -OH oder -NHSO₂(C₁-C₃-Alkyl) ist.

7. Verbindung gemäß Anspruch 6 oder ein pharmazeutisch oder veterinär verträgliches Salz oder Solvat der Verbindung, wobei eines von R³, R⁴, R⁵, R⁶ und R⁷ R⁸ oder R⁹ ist, zwei andere von R³, R⁴ , R⁵, R⁶ und R⁷ H oder R⁸ sind und die anderen zwei von R³, R⁴, R⁵, R⁶ und R⁷ H sind.

8. Verbindung gemäß Anspruch 7 oder ein pharmazeutisch oder veterinär verträgliches Salz oder Solvat der Verbindung, wobei R³ R⁹ ist.

9. Verbindung gemäß Anspruch 8 oder ein pharmazeutisch oder veterinär verträgliches Salz oder Solvat der Verbindung, wobei R⁹ -OH ist.

10. Verbindung gemäß Anspruch 1, ausgewählt aus:
(6R*,7R*)-7-Hydroxy-6-{1R*)-[3-(4-hydroxyphenyl)-1-methylpropyl]amino}-4,5,6,7-tetrahydroimidazo[4,5,1-jk][1]benzazepin-2(1H)-on,
(6R*,7R*)-7-Hydroxy-6-{(1S*)-[3-(4-hydroxyphenyl)-1-methylpropyl]amino}-4,5,6,7-tetrahydroimidazo[4,5,1-jk][1]benzazepin-2(1H)-on,
(6R,7R)-7-Hydroxy-6-{(1RS)-[3-(4-hydroxyphenyl)-1-methylpropyl]amino}-4,5,6,7-tetrahydroimidazo[4,5,1-jk][1]benzazepin-2(1H)-on,
(6R,7R)-7-Hydroxy-6-{(1S)-[3-(4-hydroxyphenyl)-1-methylpropyl]amino}-4,5,6,7-tetrahydroimidazo[4,5,1-jk][1]benzazepin-2(1H)-on,
(6R,7R)-7-Hydroxy-6-{(1R)-[3-(4-hydroxyphenyl)-1-methylpropyl]amino}-4,5,6,7-tetrahydroimidazo[4,5,1-jk][1]benzazepin-2(1H)-on,
(6R*,7R*)-7-Hydroxy-6-{[(1R*)-3-(2-hydroxyphenyl)-1-methylpropyl]amino}-4,5,6,7-tetrahydroimidazo[4,5,1-jk] [1]benzazepin-2(1H)-on,
(6R*,7R*)-7-Hydroxy-6-{[(1S*)-3-(2-hydroxyphenyl)-1-methylpropyl]amino}-4,5,6,7-tetrahydroimidazo[4,5,1-jk] [1]benzazepin-2(1H)-on,
(6R,7R)-7-Hydroxy-6-{[(1RS)-3-(2-hydroxyphenyl)-1-methylpropyl]amino}-4,5,6,7-tetrahydroimidazo[4,5,1-jk] [1] benzazepin-2 (1H)-on,
(6R,7R)-7-Hydroxy-6-{[(1R)-3-(2-hydroxyphenyl)-1-methylpropyl]amino}-4,5,6,7-tetrahydroimidazo[4,5,1-jk] [1]benzazepin-2(1H)-on,
(6R,7R)-7-Hydroxy-6-{[(1S)-3-(2-hydroxyphenyl)-1-methylpropyl]amino}-4,5,6,7-tetrahydroimidazo[4,5,1-jk] [1]benzazepin-2(1H)-on,
(6R*,7R*)-6-{[(1R*)-3-(5-Fluor-2-hydroxyphenyl)-1-methylpropyl]amino}-7-hydroxy-4,5,6,7-tetrahydroimidazo[4,5,1-jk] [1]benzazepin-2 (1H) -on,
(6R*,7R*)-6-{[(1S*)-3-(5-Fluor-2-hydroxyphenyl)-1-methylpropyl]amino}-7-hydroxy-4,5,6,7-tetrahydromidazo[4,5,1-jk] [1]benzazepin-2 (1H) -on,
(6R,7R)-6-{[(1RS)-3-(5-Fluor-2-hydroxyphenyl)-1-methylpropyl]amino}-7-hydroxy-4,5,6,7-tetrahydroimidazo[4,5,1-jk] [1]benzazepin-2(1H)-on,
(6R,7R)-6-{[(1R)-3-(5-Fluor-2-hydroxyphenyl)-1-methylpropyl]amino}-7-hydroxy-4,5,6,7-tetrahydroimidazo[4,5,1-jk][1]benzazepin-2(1H)-on,
(6R,7R)-6-{[(1S)-3-(5-Fluor-2-hydroxyphenyl)-1-methylpropyl]amino}-7-hydroxy-4,5,6,7-tetrahydroimidazo[4,5,1-jk] [1]benzazepin-2 (1H) -on,
(6R*,7R*)-6-{[(1R*)-3-(4,5-Difluor-2-hydroxyphenyl)-1-methylpropyl]amino}-7-hydroxy-4,5,6,7-tetrahydroimidazo[4,5,1-jk] [1]benzazepin-2(1H)-on,
(6R*,7R*)-6-{[(1S*)-3-(4,5-Difluor-2-hydroxyphenyl)-1-methylpropyl]amino}-7-hydroxy-4,5,6,7-tetrahydroimidazo[4,5,1-jk] [1]benzazepin-2(1H)-on,
(6R,7R)-6-{[(1RS)-3-(4,5-Difluor-2-hydroxyphenyl)-1-methylpropyl]amino}-7-hydroxy-4,5,6,7-tetrahydroimidazo[4,5,1-jk] [1]benzazepin-2(1H)-on,
(6R,7R)-6-{[(1R)-3-(4,5-Difluor-2-hydroxyphenyl)-1-methylpropyl]amino}-7-hydroxy-4,5,6,7-tetrahydroimidazo[4,5,1-jk] [1]benzazepin-2(1H)-on, und
(6R,7R)-6-{[(1S)-3-(4,5-Difluor-2-hydroxyphenyl)-1-methylpropyl]amino}-7-hydroxy-4,5,6,7-tetrahydroimidazo[4,5,1-j k] [1]benzazepin-2 (1H) -on,
oder ein pharmazeutisch oder veterinär verträgliches Salz oder Solvat der Verbindung.

11. Futtermittel-Additiv für ein Nutztier, umfassend eine Verbindung gemäß einem der Ansprüche 1 bis 10 oder ein pharmazeutisch oder veterinär verträgliches Salz oder Solvat der Verbindung.

12. Verbindung gemäß einem der Ansprüche 1 bis 10 oder ein pharmazeutisch oder veterinär verträgliches Salz oder Solvat der Verbindung zur Verwendung als Medikament.

13. Pharmazeutische Zusammensetzung, umfassend eine Verbindung gemäß einem der Ansprüche 1 bis 10 oder ein pharmazeutisch oder veterinär verträgliches Salz oder Solvat der Verbindung und einen pharmazeutisch verträglichen Träger.

14. Verwendung einer Verbindung der Formel (I) gemäß einem der Ansprüche 1 bis 10 oder ein pharmazeutisch oder veterinär verträgliches Salz oder Solvat der Verbindung für die Herstellung eines Medikaments zur Behandlung von Erkrankungen, Störungen und Zuständen, bei denen der beta-2-Rezeptor involviert ist.

## Revendications

1. Composé de formule (I) 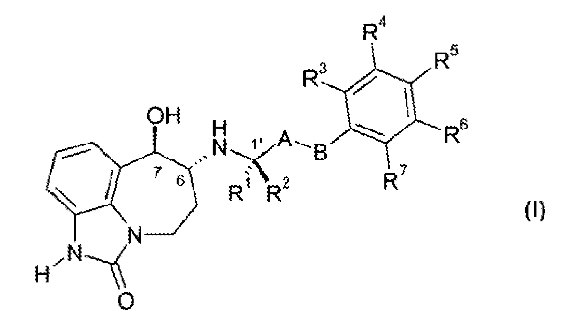 ou sel ou solvate, acceptable sur un plan pharmaceutique ou vétérinaire, dudit composé, dans lequel :
A représente -CH₂- ; et
B représente -CH₂-, -C(CH₃)₂-, -O-, -CH₂-CH₂-, -CH₂-O-, ou -O-CH₂- ; ou
-A-B- représente -CH=CH- ;
l'un de R¹ et R² représente CH₃ et l'autre représente H ;
R³, R⁴, R⁵, R⁶ et R⁷ sont chacun indépendamment sélectionnés parmi H, R⁸ et R⁹ ; ou
R⁴ et R⁵, pris ensemble, représentent -O-CH₂-CH₂-, -CH₂-CH₂-O- ou -O-CH₂-O-, et R³, R⁶ et R⁷ sont chacun indépendamment sélectionnés parmi H, R⁸ et R⁹ ;
R⁸ représente halogéno, -CN, alkyle en C₁-C₄, halogéno(alkyle en C₁-C₄), -CH₂OH, -O-(alkyle en C₁-C₄), -O-CH₂-(cycloalkyle en C₃-C₅), -CO₂H, -CO₂(alkyle en C₁-C₄), -CONH₂, -CONH(alkyle en C₁-C₄), -CONH(halogénoalkyle en C₁-C₄), -CONH(cycloalkyle en C₃-C₆) ou NH₂ ; et
R⁹ représente -OH, -NHSO₂(alkyle en C₁-C₃), -NHCO(alkyle e n C₁-C₄), -NHCO(halogénoalkyle en C₁-C₄), -NHSO₂(halogénoalkyle en C₁-C₃) ou -NHSO₂(phényle).

2. Composé selon la revendication 1, ou sel ou solvate, acceptable sur un plan pharmaceutique ou vétérinaire, dudit composé, dans lequel :
-A- représente -CH₂- et -B- représente -CH₂- ou -C(CH₃)₂- ; ou
-A-B- représente -CH=CH-.

3. Composé selon la revendication 2, ou sel ou solvate, acceptable sur un plan pharmaceutique ou vétérinaire, dudit composé, dans lequel -A- représente -CH₂- et -B- représente -CH₂-.

4. Composé selon l'une quelconque des revendications 1 à 3, ou sel ou solvate, acceptable sur un plan pharmaceutique ou vétérinaire, dudit composé, dans lequel R¹ représente H et R² représente CH₃.

5. Composé selon la revendication 4, ou sel ou solvate, acceptable sur un plan pharmaceutique ou vétérinaire, dudit composé, dans lequel la stéréochimie absolue en C-1', C-6 et C-7 est *R, R, R.*

6. Composé selon l'une quelconque des revendications 1 à 5, ou sel ou solvate, acceptable sur un plan pharmaceutique ou vétérinaire, dudit composé, dans lequel R³, R⁴, R⁵, R⁶ et R⁷ sont chacun indépendamment sélectionnés parmi H, R⁸ et R⁹, pour autant qu'au moins deux parmi R³, R⁴, R⁵, R⁶ et R⁷ représentent H ; ou
R⁴ et R⁵, pris ensemble, représentent -O-CH₂-CH₂-, -CH₂-CH₂-O- ou -O-CH₂-O-, et R³, R⁶ et R⁷ représentent H ; R⁸ représente halogéno, -CN, alkyle en C₁-C₄, -CF₃, -CH₂OH, -O-(alkyle en C₁-C₄), ou -O-CH₂-(cycloalkyle en C₃-C₅) ; et R⁹ représente -OH ou -NHSO₂(alkyle en C₁-C₃).

7. Composé selon la revendication 6, ou sel ou solvate, acceptable sur un plan pharmaceutique ou vétérinaire, dudit composé, dans lequel l'un de R³, R⁴, R⁵, R⁶ et R⁷ représente R⁸ ou R⁹, deux autres parmi R³, R⁴, R⁵, R⁶ et R⁷ représentent H ou R⁸, et les deux autres parmi R³, R⁴, R⁵, R⁶ et R⁷ représentent H.

8. Composé selon la revendication 7, ou sel ou solvate, acceptable sur un plan pharmaceutique ou vétérinaire, dudit composé, dans lequel R³ représente R⁹

9. Composé selon la revendication 8, ou sel ou solvate, acceptable sur un plan pharmaceutique ou vétérinaire, dudit composé, dans lequel R⁹ représente -OH.

10. Composé selon la revendication 1 sélectionné parmi :
■ la (6*R**,7*R**)-7-hydroxy-6-{(1*R**)-[3-(4-hydroxyphényl)-1-méthylpropyl]amino}-4,5,6,7-tétrahydro-imidazo[4,5,1-*jk*] [1]benzazépin-2(1*H*)-one,
■ la (6*R**,7*R**)-7-hydroxy-6-{(1*S**)-[3-(4-hydroxyphényl)-1-méthylpropyl]amino}-4,5,6,7-tétrahydro-imidazo[4,5,1-*jk*] [1]benzazépin-2(1*H*)-one,
■ la (6*R*,7*R*)-7-hydroxy-6-t(1*RS*)-[3-(4-hydroxyphényl)-1-méthylpropyl]amino}-4,5,6,7-tétrahydro-imidazo[4,5,1-*jk*] [1]benzazépin-2(1*H*)-one,
■ la (6*R*,7*R*)-7-hydroxy-6-{(1*S*)-[3-(4-hydroxyphényl)-1-méthylpropyl]amino}-4,5,6,7-tétrahydro-imidazo[4,5,1-*jk*] [1]benzazépin-2(1*H*)-one,
■ la (6*R*,7*R*)-7-hydroxy-6-{(1*R*)-[3-(4-hydroxyphényl)-1-méthylpropyl]amino}-4,5,6,7-tétrahydro-imidazo[4,5,1-*jk*] [1]benzazépin-2(1*H*)-one,
■ la (6*R**,7*R**)-7-hydroxy-6-{[(1*R**)-3-(2-hydroxyphényl)-1-méthylpropyl]amino}-4,5,6,7-tétrahydro-imidazo[4,5,1-*jk*] [1]benzazépin-2(1*H*)-one,
■ la (6*R**,7*R**)-7-hydroxy-6-{[(1*S**)-3-(2-hydroxyphényl)-1-méthylpropyl]amino}-4,5,6,7-tétrahydro-imidazo[4,5,1-*jk*] [1]benzazépin-2(1*H*)-one,
■ la (6*R*,7*R*)-7-hydroxy-6-{[(1*RS*)-3-(2-hydroxyphényl)-1-méthylpropyl]amino}-4,5,6,7-tétrahydro-imidazo[4,5,1-*jk*] [1]benzazépin-2(1*H*)-one,
■ la (6*R*,7*R*)-7-hydroxy-6-{[(1*R*)-3-(2-hydroxyphényl)-1-méthylpropyl]amino}-4,5,6,7-tétrahydro-imidazo[4,5,1-*jk*] [1]benzazépin-2(1*H*)-one,
■ la (6*R*,7*R*)-7-hydroxy-6-{[(1*S*)-3-(2-hydroxyphényl)-1-méthylpropyl]amino}-4,5,6,7-tétrahydro-imidazo[4,5,1-*jk*] [1]benzazépin-2(1*H*)-one,
■ la (6*R**,7*R**)-6-{[(1*R**)-3-(5-fluoro-2-hydroxyphényl)-1-méthylpropyl]amino}-7-hydroxy-4,5,6,7-tétrahydroimidazo [4,5,1-*jk*][1]benzazépin-2(1*H*)-one
■ la (6*R**,7*R**)-6-{[(1*S**)-3-(5-fluoro-2-hydroxyphényl)-1-méthylpropyl]amino}-7-hydroxy-4,5,6,7-tétrahydroimidazo [4,5,1-*jk*][1]benzazépin-2(1*H*)-one,
■ la (6*R*,7*R*)-6-{[(1*RS*)-3-(5-fluoro-2-hydroxyphényl)-1-méthylpropyl]amino}-7-hydroxy-4,5,6,7-tétrahydroimidazo [4,5,1-*jk*][1]benzazépin-2(1*H*)-one,
■ la (6*R*,7*R*)-6-{[(1*R*)-3-(5-fluoro-2-hydroxyphényl)-1-méthylpropyl]amino}-7-hydroxy-4,5,6,7-tétrahydroimidazo [4,5,1-*jk*][1]benzazépin-2(1*H*)-one,
■ la (6*R*,7*R*)-6-{[(1*S*)-3-(5-fluoro-2-hydroxyphényl)-1-méthylpropyl]amino}-7-hydroxy-4,5,6,7-tétrahydroimidazo [4,5,1-*jk*][1]benzazépin-2(1*H*)-one,
■ la (6*R**,7*R**)-6-{[(1*R**)-3-(4,5-difluoro-2-hydroxyphényl)-1-méthylpropyl]amino}-7-hydroxy-4,5,6,7-tétrahydroimidazo [4,5,1-*jk*][1]benzazépin-2(1*H*)-one,
■ la (6*R**,7*R**)-6-{[(1*S**)-3-(4,5-difluoro-2-hydroxyphényl)-1-méthylpropyl]amino}-7-hydroxy-4,5,6,7-tétrahydroimidazo [4,5,1-*jk*][1]benzazépin-2(1*H*)-one,
■ la (6*R*,7*R*)-6-{[(1*RS*)-3-(4,5-difluoro-2-hydroxyphényl)-1-méthylpropyl]amino}-7-hydroxy-4,5,6,7-tétrahydroimidazo [4,5,1-*jk*][1]benzazépin-2(1*H*)-one,
■ la (6*R*,7*R*)-6-{[(1*R*)-3-(4,5-difluoro-2-hydroxyphényl)-1-méthylpropyl]amino}-7-hydroxy-4,5,6,7-tétrahydroimidazo [4,5,1-*jk*][1]benzazépin-2(1*H*)-one,et
■ la (6*R*,7*R*)-6-{[(1*S*)-3-(4,5-difluoro-2-hydroxyphényl)-1-méthylpropyl]amino}-7-hydroxy-4,5,6,7-tétrahydroimidazo [4,5,1-*jk*][1]benzazépin-2(1*H*)-one,
ou sel ou solvate, acceptable sur un plan pharmaceutique ou vétérinaire, dudit composé.

11. Complément alimentaire pour animal de bétail comprenant un composé selon l'une quelconque des revendications 1 à 10, ou un sel ou solvate, acceptable sur un plan pharmaceutique ou vétérinaire, dudit composé.

12. Composé selon l'une quelconque des revendications 1 à 10, ou sel ou solvate, acceptable sur un plan pharmaceutique ou vétérinaire, dudit composé, pour une utilisation comme médicament.

13. Composition pharmaceutique comprenant un composé selon l'une des revendications 1 à 10, ou un sel ou solvate, acceptable sur un plan pharmaceutique ou vétérinaire, dudit composé, et un support pharmaceutiquement acceptable.

14. Utilisation d'un composé de formule (I) selon l'une quelconque des revendications 1 à 10, ou d'un sel ou solvate, acceptable sur un plan pharmaceutique ou vétérinaire, dudit composé, pour la fabrication d'un médicament pour le traitement de maladies, de troubles et d'états dans lesquels le récepteur bêta-2 est impliqué.
